# EUROPEAN PATENT SPECIFICATION

(11) **EP 1 104 412 B1**
(45) Date of publication and mention of the grant of the patent: **15.06.2005**
(21) Application number: 99933079.8
(22) Date of filing: 05.08.1999
(51) Int. Cl.: C07D 279/12, C07D 265/30, A61K 31/535, A61K 31/54, C07D 241/24

(54) **TACE INHIBITORS**
TACE INHIBITOREN
INHIBITEURS DE L'ENZYME DE CONVERSION DU FACTEUR DE NECROSE TUMORALE ALPHA (TACE)

(30) Priority: 12.08.1998 US 96256 P
(43) Date of publication of application: 06.06.2001
(73) Proprietor: Pfizer Products Inc., Groton, Connecticut 06340 (US)
(72) Inventor: McCLURE, Kim, Francis, Mystic, CT 06355 (US); NOE, Mark, Carl, Mystic, CT 06355 (US); LETAVIC, Michael, Anthony, Mystic, CT 06355 (US); CHUPAK, Louis, Stanley, Old Saybrook, CT 06475 (US)
(74) Representative: Ruddock, Keith Stephen
(86) International application number: PCT/IB1999/001392
(87) International publication number: WO 2000/009492

(56) References cited:
- WO-A-97/20824
- WO-A-98/08825
- WO-A-98/27069
- WO-A-98/34918
- US-A- 5 753 653
- BECKETT R.P. ET AL: 'Matrix metalloproteinase inhibitors 1998' EXP.OPIN.THER.PATENTS vol. 8, no. 3, March 1998, pages 259 - 282
- Data provided by the Applicant with fax of 05/08/1998

## Description

### Background of the Invention

The present invention relates to heterocyclic hydroxamide derivatives, and to pharmaceutical compositions comprising such derivatives and to the use of such derivatives for the manufacture of a medicament for the treatment of arthritis, cancer and other diseases.

The compounds of the present invention are inhibitors of zinc metalloendopeptidases, especially those belonging to the matrix metalloproteinase (also called MMP or matrixin) and reprolysin (also known as adamylsin) subfamilies of the metzincins (Rawlings, et al., Methods in Enzymology, 248, 183-228 (1995) and Stocker, et al., Protein Science, 4, 823-840 (1995)).

The MMP subfamily of enzymes, currently contains seventeen members (MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20). The MMP's are most well known for their role in regulating the turn-over of extracellular matrix proteins and as such play important roles in normal physiological processes such as reproduction, development and differentiation. In addition, the MMP's are expressed in many pathological situations in which abnormal connective tissue turnover is occurring. For example, MMP-13 an enzyme with potent activity at degrading type II collagen (the principal collagen in cartilage), has been demonstrated to be overexpressed in osteoarthritic cartilage (Mitchell, et al., J. Clin. Invest., 97, 761 (1996)). Other MMPs (MMP-2, MMP-3, MMP-8, MMP-9, MMP-12) are also overexpressed in osteoarthritic cartilage and inhibition of some or all of these MMP's is expected to slow or block the accelerated loss of cartilage typical of joint diseases such as osteoarthritis or rheumatoid arthritis.

The mammalian reprolysins are known as ADAMs (A Disintegrin And Metalloproteinase) (Wolfberg, et al., J. Cell Biol., 131, 275-278 (1995)) and contain a disintegrin domain in addition to a metalloproteinase-like domain. To date twenty-three distinct ADAM's have been identified.

ADAM-17, also known as tumor necrosis factor-alpha converting enzyme (TACE), is the most well known ADAM. ADAM-17 (TACE) is responsible for cleavage of cell bound tumor necrosis factor-alpha (TNF-α, also known as cachectin). TNF-α is recognized to be involved in many infectious and autoimmune diseases (W. Friers, FEBS Letters, 285, 199 (1991)). Furthermore, it has been shown that TNF-α is the prime mediator of the inflammatory response seen in sepsis and septic shock (Spooner, et al., Clinical Immunology and Immunopathology, 62 S11 (1992)). There are two forms of TNF-α, a type II membrane protein of relative molecular mass 26,000 (26 kD) and a soluble 17 kD form generated from the cell bound protein by specific proteolytic cleavage. The soluble 17 kD form of TNF-α is released by the cell and is associated with the deleterious effects of TNF-α. This form of TNF-α is also capable of acting at sites distant from the site of synthesis. Thus, inhibitors of TACE prevent the formation of soluble TNF-α and prevent the deleterious effects of the soluble factor (see United States Patent 5,830,742 issued November 3, 1998 ).

Select compounds of the invention are potent inhibitors of aggrecanase, an enzyme important in the degradation of cartilage aggrecan. Aggrecanase is also believed to be an ADAM. The loss of aggrecan from the cartilage matrix is an important factor in the progression of joint diseases such as osteoarthritis and rheumatoid arthritis and inhibition of aggrecanase is expected to slow or block the loss of cartilage in these diseases.

Other ADAMs that have shown expression in pathological situations include ADAM TS-1 (Kuno, et al., J. Biol. Chem., 272, 556-562 (1997)), and ADAM's 10, 12 and 15 (Wu, et al., Biochem. Biophys. Res. Comm., 235, 437-442, (1997)). As knowledge of the expression, physiological substrates and disease association of the ADAM's increases the full significance of the role of inhibition of this class of enzymes will be appreciated.

The compounds of the invention are useful in the treatment of arthritis (including osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity, cachexia, allergic reactions, allergic contact hypersensitivity, cancer (such as solid tumor cancer including colon cancer, breast cancer, lung cancer and prostrate cancer and hematopoietic malignancies including leukemias and lymphomas), tissue ulceration, restenosis, periodontal disease, epidermolysis bullosa, osteoporosis, loosening of artificial joint implants, atherosclerosis (including atherosclerotic plaque rupture), aortic aneurysm (including abdominal aortic aneurysm and brain aortic aneurysm), congestive heart failure, myocardial infarction, stroke, cerebral ischemia, head trauma, spinal cord injury, neuro-degenerative disorders (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, diabetes, tumor invasion, tumor growth, tumor metastasis, corneal scarring, scleritis, AIDS, sepsis or septic shock.

The compounds of the present invention are also useful in the treatment of diseases in which inhibition of MMP's and/or ADAM's will provide therapeutic benefit, such as those characterized by matrix metalloproteinase or ADAM expression.

The present inventors have also discovered that it is possible to identify inhibitors with differential metalloprotease and reprolysin activity (preferably TACE inhibitory activity). One group of preferred inhibitors include those which selectively inhibit TACE preferentially over MMP-1. Another group of preferred inhibitors include those molecules which selectively inhibit TACE and matrix metalloprotease-13 (MMP-13) preferentially over MMP-1. Another group of preferred inhibitors include those molecules which selectively inhibit Aggrecanase and matrix metalloprotease-13 (MMP-13) preferentially over MMP-1. Another group of preferred inhibitors include those molecules which selectively inhibit Aggrecanase and TACE preferentially over MMP-1. Another group of preferred inhibitors include those molecules which selectively inhibit Aggrecanase preferentially over MMP-1. Another group of preferred inhibitors include those molecules which selectively inhibit MMP-13 preferentially over MMP-1. Another group of preferred inhibitors include those molecules which selectively inhibit Aggrecanase, TACE and MMP-13 preferentially over MMP-1.

Matrix metalloproteinase and reprolysin inhibitors are well known in the literature. Specifically, European Patent Publication 606,046, published July 13, 1994, refers to certain heterocyclic MMP inhibitors. United States Patent 5,861,510, issued January 19, 1999, refers to cyclic arylsulfonylamino hydroxamic acids that are useful as MMP inhibitors. PCT Publication WO 98/34918, published August 13, 1998, refers to heterocyclic hydroxamic acids including certain dialkyl substituted compounds that are useful as MMP inhibitors. PCT publications WO 96/27583 and WO 98/07697, published March 7, 1996 and February 26, 1998, respectively, refer to arylsulfonyl hydroxamic acids. PCT publication WO 98/03516, published January 29, 1998 refers to phosphinates with MMP activity. PCT publication 98/33768, published August 6, 1998, refers to N-unsubstituted arylsulfonylamino hydroxamic acids. PCT Publication WO 98/08825, published March 5, 1998, refers to certain MMP inhibitors.

### Summary of the Invention

The present invention relates to a compound of the formula 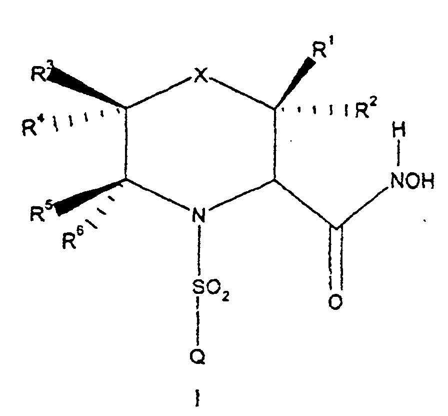 or the pharmaceutically acceptable salt thereof, wherein
X is oxygen, sulfur, SO, SO₂ or NR⁷;
R¹, R², R³, R⁴, R⁵ and R⁶ are selected from the group consisting of hydrogen, hydroxy, (C₁-C₆)alkyl, -CN, (C₂-C₆)alkenyl, (C₆-C₁₀)aryl(C₂-C₆)alkenyl, (C₂-C₉)heteroaryl(C₂-C₆)alkenyl, (C₂-C₆)alkynyl, (C₆-C₁₀)aryl(C₂-C₆)alkynyl, (C₂-C₉)heteroaryl(C₂-C₆)alkynyl, (C₁-C₆)alkylamino, (C₁-C₆)alkylthio, (C₁-C₆)alkoxy, perfluoro(C₁-C₆)alkyl, (C₆-C₁₀)aryl, (C₂-C₉)heteroaryl, (C₆-C₁₀)arylamino, (C₆-C₁₀)arylthio, (C₆-C₁₀)aryloxy, (C₂-C₉)heteroarylamino, (C₂-C₉)heteroarylthio, (C₂-C₉)heteroaryloxy, (C₃-C₆)cycloalkyl, (C₁-C₆)alkyl(hydroxymethylene), piperidyl, (C₁-C₆)alkylpiperidyl, (C₁-C₆)acylamino, (C₁-C₆)acylthio, (C₁-C₆)acyloxy, (C₁-C₆)alkoxy-(C=O)-, -CO₂H, (C₁-C₆)alkyl-NH-(C=O)-, and [(C₁-C₆)alky]₂-N-(C=O)-;
wherein said (C₁-C₆)alkyl moiety is optionally substituted by one or two groups independently selected from (C₁-C₆)alkylthio, (C₁-C₆)alkoxy, trifluoromethyl, halo, -CN, (C₆-C₁₀)aryl, (C₂-C₉)heteroaryl, (C₆-C₁₀)arylamino, (C₆-C₁₀)arylthio, (C₆-C₁₀)aryloxy, (C₂-C₉)heteroarylamino, (C₂-C₉)heteroarylthio, (C₂-C₉)heteroaryloxy, (C₆-C₁₀)aryl(C₆-C₁₀)aryl, (C₃-C₆)cycloalkyl, hydroxy, piperazinyl, (C₆-C₁₀)aryl(C₁-C₆)alkoxy, (C₂-C₉)heteroaryl(C₁-C₆)alkoxy, (C₁-C₆)acylamino, (C₁-C₆)acylthio, (C₁-C₆)acyloxy, (C₁-C₆)alkylsulfinyl, (C₆-C₁₀)arylsulfinyl, (C₁-C₆)alkylsulfonyl, (C₆-C₁₀)arylsulfonyl, amino, (C₁-C₆)alkylamino or ((C₁-C₆)alkyl)₂amino;
R⁷ is hydrogen or (C₁-C₆)alkyl optionally substituted by one or more of, hydroxy, -CN, (C₁-C₆)alkylamino, (C₁-C₆)alkylthio, (C₁-C₆)alkoxy, perfluoro(C₁-C₆)alkyl, (C₆-C₁₀)aryl, (C₈-C₁₀)arylthio, (C₆-C₁₀)aryloxy, (C₂-C₉)heteroarylamino, (C₃-C₆)cycloalkyl, (C₁-C₆)alkyl(hydroxymethylene), piperidyl, (C₁-C₆)alkylpiperidyl, (C₁-C₆)acylamino, (C₁-C₆)acyloxy, (C₁-C₆)alkoxy-(C=O)-, -CO₂H, (C₁-C₆)alkyl-NH-(C=O)-, and [(C₁-C₆)alky]₂-N-(C=O)-; (C₆-C₁₀)arylsulfonyl, (C₁-C₆)alkylsulfonyl, (C₁-C₆)alkyl-NH-(C=O)-, (C₁-C₆)alkoxy-(C=O)-, (C₁-C₆)alkyl-(C=O)-, [(C₁-C₆)alky]₂-N-(C=O)-, (R⁸R⁹N)-(C=O) where R⁸ and R⁹ are taken together with the nitrogen that they are attached to form an azetidinyl, pyrrolidinyl, piperidinyl, morpholinyl and thiomorphonyl;
Q is (C₆-C₁₀)aryl(C₁-C₆)alkoxy(C₆-C₁₀)aryl, (C₆-C₁₀)aryl(C₁-C₆)alkoxy(C₂-C₉)heteroaryl, (C₂-C₉)heteroaryl(C₁-C₆)alkoxy(C₆-C₁₀)aryl, or (C₂-C₉)heteroaryl(C₁-C₆)alkoxyC₂-C₉)heteroaryl wherein each of said (C₆-C₁₀)aryl or (C₂-C₉)heteroaryl groups may optionally be substituted by one or more substituents, preferably one to three substituents per ring, most preferably one to three substituents on the terminal ring (i.e. the ring furthest from the point of attachment) independently selected from the group consisting of halo, -CN, (C₁-C₆)alkyl optionally substituted with one or more fluorine atoms (preferably one to three fluorine atoms), hydroxy, hydroxy-(C₁-C₆)alkyl, (C₁-C₆)alkoxy optionally substituted with one or more fluorine atoms (preferably one to three fluorine atoms), (C₁-C₆)alkoxy(C₁-C₆)alkyl, HO-(C=O)-, (C₁-C₆)alkyl-O-(C=O)-, HO-(C=O)-(C₁-C₆)alkyl, (C₁-C₆)alkyl-O-(C=O)-(C₁-C₆)alkyl, (C₁-C₆)alkyl-(C=O)-O-, (C₁-C₆)alkyl-(C=O)-O-(C₁-C₆)alkyl, H(O=C)-, H(O=C)-(C₁-C₆)alkyl, (C₁-C₆)alkyl(O=C)-, (C₁-C₆)alkyl(O=C)-(C₁-C₆)alkyl, NO₂, amino, (C₁-C₆)alkylamino, [(C₁-C₆)alkyl]₂amino, amino(C₁-C₆)alkyl, (C₁-C₆)alkylamino(C₁-C₆)alkyl, [(C₁-C₆)alkyl]₂amino(C₁-C₆)alkyl, H₂N-(C=O)-, (C₁-C₆)alkyl-NH-(C=O)-, [(C₁-C₆)alkyl]₂N-(C=O)-, H₂N(C=O)-(C₁-C₆)alkyl, (C₁-C₆)alkyl-HN(C=O)-(C₁-C₆)alkyl, [(C₁-C₆)alkyl]₂N-(C=O)-(C₁-C₆)alkyl, H(O=C)-NH-, (C₁-C₆)alkyl(C=O)-NH, (C₁-C₆)alkyl(C=O)-[NH](C₁-C₆)alkyl, (C₁-C₆)alkyl(C=O)-[N(C₁-C₆)alkyl](C₁-C₆)alkyl, (C₁-C₆)alkyl-S-, (C₁-C₆)alkyl-(S=O)-, (C₁-C₆)alkyl-SO₂-, (C₁-C₆)alkyl-SO₂-NH-, (C₁-C₆)alkyl-SO₂-[N-(C₁-C₆)alkyl]-, H₂N-SO₂-, H₂N-SO₂-(C₁-C₆)alkyl, (C₁-C₆)alkylHN-SO₂-(C₁-C₆)alkyl, [(C₁-C₆)alkyl]₂N-SO₂-(C₁-C₆)alkyl, CF₃SO₃-, (C₁-C₆)alkyl-SO₃-, phenyl, phenyl(C₁-C₆)alkyl, (C₃-C₁₀)cycloalkyl, (C₂-C₉)heterocycloalkyl, and (C₂-C₉)heteroaryl;
with the provisio that when X is SO or SO₂, and R³ and R⁴ are a substituent comprising a heteroatom, the heteroatom cannot be bonded to the ring
and with the proviso that at least one of R¹-R⁶ must be (C₁-C₆)alkyl;
and with the proviso that when X is oxygen or sulfur and R³-R⁶ are each hydrogen then R¹ and R² cannot both be methyl.

Preferred compounds of the present invention are those wherein X is NR⁷, sulfur or oxygen.

Other preferred compounds of the present invention are those wherein Q is optionally substituted (C₆-C₁₀)aryl(C₁-C₆)alkoxy(C₆-C₁₀)aryl, (C₆-C₁₀)aryl(C₁-C₆)alkoxy(C₂-C₉)heteroaryl, (C₂-C₉)heteroaryl(C₁-C₆)alkoxy(C₆-C₁₀)aryl or (C₂-C₉)heteroaryl(C₁-C₆)alkoxy(C₂-C₉)heteroaryl, wherein each of the above aryl or hetero aryl groups is optionally substituted by one or more substituents, preferably one to three substituents per ring, most preferably one to three substituents on the terminal ring, wherein said substituents are selected from halo, (C,-C₆)alkyl, (C₁-C₆)alkoxy or perfluoro(C₁-C₃)alkyl.

Other preferred compounds of the present invention are those wherein Q is (C₆-C₁₀)arylmethoxy(C₆-C₁₀)aryl, (C₆-C₁₀)arylmethoxy(C₂-C₉)heteroaryl, (C₂-C₉)heteroarylmethoxy(C₆-C₁₀)aryl or (C₂-C₉)heteroarylmethoxyC₂-C₉)heteroaryl optionally substituted by one or more substituents, preferably one to three substituents per ring, most preferably one to three substituents on the terminal ring, wherein said substituents are selected from halo, (C₁-C₆)alkyl, (C₁-C₆)alkoxy or perfluoro(C₁-C₃)alkyl.

More preferred compounds of the invention are those wherein Q is optionally substituted (C₆-C₁₀)arylmethoxyoxyphenyl, (C₆-C₁₀)arylmethoxypyridyl, (C₆-C₁₀)arylmethoxyfuryl, (C₆-C₁₀)arylmethoxypyroyl, (C₆-C₁₀)arylmethoxythienyl, (C₆-C₁₀)arylmethoxyisothiazolyl, (C₆-C₁₀)arylmethoxyimidazolyl, (C₆-C₁₀)arylmethoxybenzimidazolyl, (C₆-C₁₀)arylmethoxytetrazolyl, (C₆-C₁₀)arylmethoxypyrazinyl, (C₆-C₁₀)arylmethoxypyrimidyl, (C₆-C₁₀)arylmethoxyquinolyl, (C₆-C₁₀)arylmethoxyisoquinolyl, (C₆-C₁₀)arylmethoxybenzofuryl, (C₆-C₁₀)arylmethoxyisobenzofuryl, (C₆-C₁₀)arylmethoxybenzothienyl, (C₆-C₁₀)arylmethoxypyrazolyl, (C₆-C₁₀)arylmethoxyindolyl, (C₆-C₁₀)arylmethoxyisoindolyl, (C₆-C₁₀)arylmethoxypurinyl, (C₆-C₁₀)arylmethoxycarbazolyl, (C₆-C₁₀)arylmethoxyisoxazolyl, (C₆-C₁₀)arylmethoxythiazolyl, (C₆-C₁₀)arylmethoxyoxazolyl, (C₆-C₁₀)arylmethoxybenzthiazolyl, (C₆-C₁₀)arylmethoxybenzoxazolyl, pyridylmethoxyphenyl, furylmethoxyphenyl, pyrroylmethoxyphenyl, thienylmethoxyphenyl, isothiazolylmethoxyphenyl, imidazolylmethoxyphenyl, benzimidazolylmethoxyphenyl, tetrazolylmethoxyphenyl, pyrazinylmethoxyphenyl, pyrimidylmethoxyphenyl, quinolylmethoxyphenyl, isoquinolylmethoxyphenyl, benzofurylmethoxyphenyl, isobenzofurylmethoxyphenyl, benzothienylmethoxyphenyl, pyrazolylmethoxyphenyl, indolylmethoxyphenyl, isoindolylmethoxyphenyl, purinylmethoxyphenyl, carbazolylmethoxyphenyl, isoxazolylmethoxyphenyl, thiazolylmethoxyphenyl, oxazolylmethoxyphenyl, benzthiazolylmethoxyphenyl, benzoxazolylmethoxyphenyl, pyridylmethoxypyridyl, pyridylmethoxyfuryl, pyridylmethoxypyroyl, pyridylmethoxythienyl, pyridylmethoxyisothiazolyl, pyridylmethoxyimidazolyl, pyridylmethoxybenzimidazolyl, pyridylmethoxytetrazolyl, pyridylmethoxypyrazinyl, pyridylmethoxypyrimidyl, pyridylmethoxyquinolyl, pyridylmethoxyisoquinolyl, pyridylmethoxybenzofuryl, pyridylmethoxyisobenzofuryl, pyridylmethoxybenzothienyl, pyridylmethoxypyrazolyl, pyridylmethoxyindolyl, pyridylmethoxyisoindolyl, pyridylmethoxypurinyl, pyridylmethoxycarbazolyl, pyridylmethoxyisoxazolyl, pyridylmethoxythiazolyl, pyridylmethoxyoxazolyl, pyridylmethoxybenzthiazolyl, pyridylmethoxybenzoxazolyl, furylmethoxypyridyl, furylmethoxyfuryl, furylmethoxypyroyl, furylmethoxythienyl, furylmethoxyisothiazolyl, furylmethoxyimidazolyl, furylmethoxybenzimidazolyl, furylmethoxytetrazolyl, furylmethoxypyrazinyl, furylmethoxypyrimidyl, furylmethoxyquinolyl, furylmethoxyisoquinolyl, furylmethoxybenzofuryl, furylmethoxyisobenzofuryl, furylmethoxybenzothienyl, furylmethoxypyrazolyl, furylmethoxyindolyl, furylmethoxyisoindolyl, furylmethoxypurinyl, furylmethoxycarbazolyl, furylmethoxyisoxazolyl, furylmethoxythiazolyl, furylmethoxyoxazolyl, furylmethoxybenzthiazolyl, furylmethoxybenzoxazolyl, pyrroylmethoxypyridyl, pyrroylmethoxyfuryl, pyrroylmethoxypyroyl, pyrroylmethoxythienyl, pyrroylmethoxyisothiazolyl, pyrroylmethoxyimidazolyl, pyrroylmethoxybenzimidazolyl, pyrroylmethoxytetrazolyl, pyrroylmethoxypyrazinyl, pyrroylmethoxypyrimidyl, pyrroylmethoxyquinolyl, pyrroylmethoxyisoquinolyl, pyrroylmethoxybenzofuryl, pyrroylmethoxyisobenzofuryl, pyrroylmethoxybenzothienyl, pyrroylmethoxypyrazolyl, pyrroylmethoxyindolyl, pyrroylmethoxyisoindolyl, pyrroylmethoxypurinyl, pyrroylmethoxycarbazolyl, pyrroylmethoxyisoxazolyl, pyrroylmethoxythiazolyl, pyrroylmethoxyoxazolyl, pyrroylmethoxybenzthiazolyl, pyrroylmethoxybenzoxazolyl, thienylmethoxypyridyl, thienylmethoxyfuryl, thienylmethoxypyroyl, thienylmethoxythienyl, thienylmethoxyisothiazolyl, thienylmethoxyimidazolyl, thienylmethoxybenzimidazolyl, thienylmethoxytetrazolyl, thienylmethoxypyrazinyl, thienylmethoxypyrimidyl, thienylmethoxyquinolyl, thienylmethoxyisoquinolyl, thienylmethoxybenzofuryl, thienylmethoxyisobenzofuryl, thienylmethoxybenzothienyl, thienylmethoxypyrazolyl, thienylmethoxyindolyl, thienylmethoxyisoindolyl, thienylmethoxypurinyl, thienylmethoxycarbazolyl, thienylmethoxyisoxazolyl, thienylmethoxythiazolyl, thienylmethoxyoxazolyl, thienylmethoxybenzthiazolyl, thienylmethoxybenzoxazolyl, pyrazinylmethoxypyridyl, pyrazinylmethoxyfuryl, pyrazinylmethoxypyroyl, pyrazinylmethoxythienyl, pyrazinylmethoxyisothiazolyl, pyrazinylmethoxyimidazolyl, pyrazinylmethoxybenzimidazolyl, pyrazinylmethoxytetrazolyl, pyrazinylmethoxypyrazinyl, pyrazinylmethoxypyrimidyl, pyrazinylmethoxyquinolyl, pyrazinylmethoxyisoquinolyl, pyrazinylmethoxybenzofuryl, pyrazinylmethoxyisobenzofuryl, pyrazinylmethoxybenzothienyl, pyrazinylmethoxypyrazolyl, pyrazinylmethoxyindolyl, pyrazinylmethoxyisoindolyl, pyrazinylmethoxypurinyl, pyrazinylmethoxycarbazolyl, pyrazinylmethoxyisoxazolyl, pyrazinylmethoxythiazolyl, pyrazinylmethoxyoxazolyl, pyrazinylmethoxybenzthiazolyl, pyrazinylmethoxybenzoxazolyl, pyrimidylmethoxypyridyl, pyrimidylmethoxyfuryl, pyrimidylmethoxypyroyl, pyrimidylmethoxythienyl, pyrimidylmethoxyisothiazolyl, pyrimidylmethoxyimidazolyl, pyrimidylmethoxybenzimidazolyl, pyrimidylmethoxytetrazolyl, pyrimidylmethoxypyrazinyl, pyrimidylmethoxypyrimidyl, pyrimidylmethoxyquinolyl, pyrimidylmethoxyisoquinolyl, pyrimidylmethoxybenzofuryl, pyrimidylmethoxyisobenzofuryl, pyrimidylmethoxybenzothienyl, pyrimidylmethoxypyrazolyl, pyrimidylmethoxyindolyl, pyrimidylmethoxyisoindolyl, pyrimidylmethoxypurinyl, pyrimidylmethoxycarbazolyl, pyrimidylmethoxyisoxazolyl, pyrimidylmethoxythiazolyl, pyrimidylmethoxyoxazolyl, pyrimidylmethoxybenzthiazolyl, pyrimidylmethoxybenzoxazolyl, thiazolylmethoxypyridyl, thiazolylmethoxyfuryl, thiazolylmethoxypyroyl, thiazolylmethoxythienyl, thiazolylmethoxyisothiazolyl, thiazolylmethoxyimidazolyl, thiazolylmethoxybenzimidazolyl, thiazolylmethoxytetrazolyl, thiazolylmethoxypyrazinyl, thiazolylmethoxypyrimidyl, thiazolylmethoxyquinolyl, thiazolylmethoxyisoquinolyl, thiazolylmethoxybenzofuryl, thiazolylmethoxyisobenzofuryl, thiazolylmethoxybenzothienyl, thiazolylmethoxypyrazolyl, thiazolylmethoxyindolyl, thiazolylmethoxyisoindolyl, thiazolylmethoxypurinyl, thiazolylmethoxycarbazolyl, thiazolylmethoxyisoxazolyl, thiazolylmethoxythiazolyl, thiazolylmethoxyoxazolyl, thiazolylmethoxybenzthiazolyl, thiazolylmethoxybenzoxazolyl, and oxazolylmethoxypyridyl, oxazolylmethoxyfuryl, oxazolylmethoxypyroyl, oxazolylmethoxythienyl, oxazolylmethoxyisothiazolyl, oxazolylmethoxyimidazolyl, oxazolylmethoxybenzimidazolyl, oxazolylmethoxytetrazolyl, oxazolylmethoxypyrazinyl, oxazolylmethoxypyrimidyl, oxazolylmethoxyquinolyl, oxazolylmethoxyisoquinolyl, oxazolylmethoxybenzofuryl, oxazolylmethoxyisobenzofuryl, oxazolylmethoxybenzothienyl, oxazolylmethoxypyrazolyl, oxazolylmethoxyindolyl, oxazolylmethoxyisoindolyl, oxazolylmethoxypurinyl, oxazolylmethoxycarbazolyl, oxazolylmethoxyisoxazolyl, oxazolylmethoxythiazolyl, oxazolylmethoxyoxazolyl, oxazolylmethoxybenzthiazolyl, oxazolylmethoxybenzoxazolyl.

More preferred compounds of the invention are those wherein Q is optionally substituted (C₆-C₁₀)arylmethoxyoxyphenyl, (C₆-C₁₀)arylmethoxypyridyl, (C₆-C₁₀)arylmethoxyfuryl, (C₆-C₁₀)arylmethoxypyroyl, (C₆-C₁₀)arylmethoxythienyl, (C₆-C₁₀)arylmethoxyisothiazolyl, (C₆-C₁₀)arylmethoxyimidazolyl, (C₆-C₁₀)arylmethoxybenzimidazolyl, (C₆-C₁₀)arylmethoxytetrazolyl, (C₆-C₁₀)arylmethoxypyrazinyl, (C₆-C₁₀)arylmethoxypyrimidyl, (C₆-C₁₀)arylmethoxyquinolyl, (C₆-C₁₀)arylmethoxyisoquinolyl, (C₆-C₁₀)arylmethoxybenzofuryl, (C₆-C₁₀)arylmethoxyisobenzofuryl, (C₆-C₁₀)arylmethoxybenzothienyl, (C₆-C₁₀)arylmethoxypyrazolyl, (C₆-C₁₀)arylmethoxyindolyl, (C₆-C₁₀)arylmethoxyisoindolyl, (C₆-C₁₀)arylmethoxypurinyl, (C₆-C₁₀)arylmethoxycarbazolyl, (C₆-C₁₀)arylmethoxyisoxazolyl, (C₆-C₁₀)arylmethoxythiazolyl, (C₆-C₁₀)arylmethoxyoxazolyl, (C₆-C₁₀)arylmethoxybenzthiazolyl, (C₆-C₁₀)arylmethoxybenzoxazolyl, pyridylmethoxyphenyl, furylmethoxyphenyl, pyroylmethoxyphenyl, thienylmethoxyphenyl, isothiazolylmethoxyphenyl, imidazolylmethoxyphenyl, benzimidazolylmethoxyphenyl, tetrazolylmethoxyphenyl, pyrazinylmethoxyphenyl, pyrimidylmethoxyphenyl, quinolylmethoxyphenyl, isoquinolylmethoxyphenyl, benzofurylmethoxyphenyl, isobenzofurylmethoxyphenyl, benzothienylmethoxyphenyl, pyrazolylmethoxyphenyl, indolylmethoxyphenyl, isoindolylmethoxyphenyl, purinylmethoxyphenyl, carbazolylmethoxyphenyl, isoxazolylmethoxyphenyl, thiazolylmethoxyphenyl, oxazolylmethoxyphenyl, benzthiazolylmethoxyphenyl, and benzoxazolylmethoxyphenyl.

Most preferred compounds of the invention are those wherein Q is optionally substituted (C₆-C₁₀)arylmethoxyphenyl, pyridylmethoxyphenyl, thienylmethoxyphenyl, pyrazinylmethoxyphenyl, pyrimidylmethoxyphenyl, pyridazinylmethoxyphenyl, thiazolylmethoxyphenyl, oxazolylmethoxyphenyl.

Other more preferred compounds of the present invention are those wherein Q is (C₆-C₁₀)arylmethoxy(C₆)aryl optionally substituted by one or more, preferably one to three substituents per ring, most preferably one to three substituents on the terminal ring, wherein said substituents are independently selected from halo, (C₁-C₆)alkyl, (C₁-C₆)alkoxy or perfluoro(C₁-C₃)alkyl.

Other more preferred compounds of the present invention are those wherein Q is (C₆-C₁₀)arylmethoxy(C₂-C₉)heteroaryl optionally substituted by one or more, preferably one to three substituents per ring, most preferably one to three substituents on the terminal ring, wherein said substituents are independently selected from halo, (C₁-C₆)alkyl, (C₁-C₆)alkoxy or perfluoro(C₁-C₃)alkyl.

Other more preferred compounds of the present invention are those wherein Q is (C₂-C₉)heteroarylmethoxy(C₆)aryl optionally substituted by one or more, preferably one to three substituents per ring, most preferably one to three substituents on the terminal ring, wherein said substituents are independently selected from halo, (C₁-C₆)alkyl, (C₁-C₆)alkoxy or perfluoro(C₁-C₃)alkyl.

Other more preferred compounds of the present invention are those wherein Q is (C₂-C₉)heteroarylmethoxy(C₂-C₉)heteroaryl optionally substituted by one or more, preferably one to three substituents per ring, most preferably one to three substituents on the terminal ring, wherein said substituents are independently selected from halo, (C₁-C₆)alkyl, (C,-C₆)alkoxy or perfluoro(C₁-C₃)alkyl.

Other more preferred compounds of the present invention are those wherein R⁴ is hydrogen.

Other more preferred compounds of the present invention are those wherein R² or R³ are hydrogen.

Other more preferred compounds of the present invention are those wherein at least one of R² or R³ is other than hydrogen.

Other more preferred compounds of the present invention are those wherein at least one of R¹-R³ is (C₁-C₆)alkyl, preferably wherein at least one of R¹-R³ is methyl, more preferably wherein R¹ and R⁴ are each methyl.

Other more preferred compounds of the present invention are those wherein R¹ and R² are (C₁-C₆)alkyl, and R³ or R⁶ is (C₁-C₆)alkyl.

Other more preferred compounds of the present invention are those wherein R¹ and R² are each methyl, and R³ or R⁶ is (C₁-C₆)alkyl.

Other more preferred compounds of the present invention are those wherein at least one of R¹-R³ is methyl.

Other more preferred compounds of the present invention are those wherein R¹ methyl and R² is hydrogen.

Other more preferred compounds of the present invention are those wherein R¹ is hydrogen and R³ is methyl.

Other more preferred compounds of the present invention are those wherein X is nitrogen and R⁷ is (C₁-C₆)alkyl.

Other more preferred compounds of the present invention are those wherein X is nitrogen and R⁷ is (C₁-C₆)alkylsulfonyl.

Other more preferred compounds of the present invention are those wherein X is nitrogen and R⁷ is (C₆-C₁₀)arylsulfonyl.

Other more preferred compounds of the present invention are those wherein X is nitrogen and R⁷ is [(C₁-C₆)alkyl]₂N-(C=O) or (C₁-C₆)alkylNH-(C=O).

Other more preferred compounds of the present invention are those wherein X is nitrogen and R⁷ is (C₁-C₆)alkyl(C=O)-.

Most preferred compounds of the present invention are selected from the group consisting of:
(2*S*,3*R*)-4-[4-(3,5-difluro-benzyloxy)-benzenesulfonyl]-2-methyl-thiomorpholine-3-carboxylic acid hydroxyamide;
(2S,3R)-4-[4-(4-fluoro-benzyloxy)-benzensulfonyl]-2-methyl-thiomorpholine-3-carboxylic acid hydroxyamide;
(2*S*,3*R*,6*S*)-2,6-dimethyl-4-[4-(2-methyl-benzyloxy)-benzenesulfonyl]-morpholine-3-carboxylic acid hydroxyamide;
4-(4-benzyloxy-benzenesulfonyl)-2-methyl-morpholine-3-carboxylic acid hydroxyamide;
(2*S*,3*R*,6*S*)-4-[4-(4-fluoro-benzyloxy)-benzenesulfonyl]-2,6-dimethyl-morpholine-3-carboxylic acid hydroxyamide;
(3*R*,6*S*)-4-[4-(4-fluoro-benzyloxy)-benzenesulfonyl]-2,2,6-trimethyl-morpholine-3-carboxylic acid hydroxyamide;
(2*S*,3*R*,6*S*)-6-ethyl-4-[4-(4-fluoro-benzyloxy)-benzenesulfonyl]-2-methyl-morpholine-3-carboxylic acid hydroxyamide;
(2*R*,3*R*,6*S*)-4-[4-(4-fluoro-benzyloxy)-benzenesulfonyl]-2,6-dimethyl-morpholine-3-carboxylic acid hydroxyamide;
(2*R*,3*R*,6*R*)-4-[4-(4-fluoro-benzyloxy)-benzenesulfonyl]-2,6-dimethyl-morpholine-3-carboxylic acid hydroxyamide;
(2*S*,3*R*,6*S*)-2,6-dimethyl-4-[4-(pyridin-4-ylmethoxy)-benzenesulfonyl]-morpholine-3-carboxylic acid hydroxyamide;
(2*S*,3*R*,6*S*)-2,6-dimethyl-4-[4-(pyridin-2-ylmethoxy)-benzenesulfonyl]-morpholine-3-carboxylic acid hydroxyamide;
(2*S*,3*R*,6*S*)-2,6-dimethyl-4-[4-(pyridin-3-ylmethoxy)-benzenesulfonyl]-morpholine-3-carboxylic acid hydroxyamide;
(2*S*,3*R*,6*S*)-2,6-dimethyl-4-[4-(2-methyl-pyridin-3-ylmethoxy)-benzenesulfonyl]-morpholine-3-carboxylic acid hydroxyamide;
(3*R*,6*S*)-2,2,6-trimethyl-4-[4-(2-trifluoromethyl-benzyloxy)-benzenesulfonyl]-morpholine-3-carboxylic acid hydroxyamide;
(2*S*,3*R*)-2,6,6-trimethyl-4-[4-(pyridin-4-ylmethoxy)-benzenesulfonyl]-morpholine-3-carboxylic acid hydroxyamide;
(3*R*,6*S*)-2,2,6-trimethyl-4-[4-(2-methyl-pyridin-3-ylmethoxy)-benzenesulfonyl]-morpholine-3-carboxylic acid hydroxyamide;
(2*S*,3*R*,6*S*)-[4-(2,5-dimethyl-benzyloxy)-benzenesulfonyl]-2,6-dimethyl-morpholine-3-carboxylic acid hydroxyamide;
(2*S*,3*R*,6*S*)-4-[4-(3,5-difluoro-benzyloxy)-benzenesulfonyl]-2,6-dimethyl-morpholine-3-carboxylic acid hydroxyamide;
(2*S*,3*R*,6*S*)-4-[4-(3-methoxy-benzyloxy)-benzenesulfonyl]-2,6-dimethyl-morpholine-3-carboxylic acid hydroxyamide;
(2*S*,3*R*,6*S*)-4-[4-(5-fluoro-2-methyl-benzyloxy)-benzenesulfonyl]-2,6-dimethyl-morpholine-3-carboxylic acid hydroxyamide;
(2*S*,3*R*,6*S*)-4-[4-(furan-3-ylmethoxy)-benzenesulfonyl]-2,6-dimethyl-morpholine-3-carboxylic acid hydroxyamide;
(2*S*,3*R*,6*S*)-4-[4-(2-fluoro-3-methyl-benzyloxy)-benzenesulfonyl]-2,6-dimethyl-morpholine-3-carboxylic acid hydroxyamide;
(2*S*,3*R*)-4-[4-(4-fluoro-benzyloxy)-benzenesulfonyl]-2,6,6-trimethyl-morpholine-3-carboxylic acid hydroxyamide;
(3*R*)-4-[4-(4-fluoro-benzyloxy)-benzenesulfonyl]-6,6-dimethyl-morpholine-3-carboxylic acid hydroxyamide;
(3*R*)-6,6-dimethyl-4-[4-(pyridin-4-ylmethoxy)-benzenesulfonyl]-morpholine-3-carboxylic acid hydroxyamide;
(3*R*)-6,6-dimethyl-4-[4-(2-methyl-benzyloxy)-benzenesulfonyl]-morpholine-3-carboxylic acid hydroxyamide;
(2*S*,3*R*,6*S*)-4-(4-cyclohexylmethoxy-benzenesulfonyl)-2,6-dimethyl-morpholine-3-carboxylic acid hydroxyamide;
(3*R*,6*S*)-*4*-[4-(2,5-dimethyl-benzyloxy)-benzenesulfonyl]-2,2,6-trimethyl-morpholine-3-carboxylic acid hydroxyamide;
(2*S*,3*R*)-4-[4-(4-fluoro-benzyloxy)-benzenesulfonyl]-6-methoxymethyl-2-methylmorpholine-3-carboxylic acid hydroxyamide;
(2*S*,3*R*,6*S*)-4-[4-(3-chloro-benzyloxy)-benzenesulfonyl]-6-[(ethyl-methyl-amino)-methyl]-2-methyl-morpholine-3-carboxylic acid hydroxyamide;
(2*S*,3*R*)-4-[4-(3-chloro-benzyloxy)-benzenesulfonyl]-6-methoxy-2-methyl-morpholine-3-carboxylic acid hydroxyamide;
(2*S*,3*R*,6*R*)-4-[4-(4-fluoro-benzyloxy)-benzenesulfonyl]-6-hydroxymethyl-2-methylmorpholine-3-carboxylic acid hydroxyamide.

Other compounds of the invention include:
4-[4-(3-Fluoro-benzyloxy)-benzenesulfonyl]-2-methyl-thiomorpholine-3-carboxylic acid hydroxyamide,
4-[4-(3-Chloro-benzyloxy)-benzenesulfonyl]-2-methyl-thiomorpholine-3-carboxylic acid hydroxyamide,
4-[4-(4-Fluoro-benzyloxy)-benzenesulfonyl]-2-methyl-1-oxo-thiomorpholine-3-carboxylic acid hydroxyamide,
4-[4-(4-Fluoro-benzyloxy)-benzenesulfonyl]-2-methyl-1,1-dioxide-thiomorpholine-3-carboxylic acid hydroxyamide,
4-[4-(3,5-Difluoro-benzyloxy)-benzenesulfonyl]-2-methyl-1-oxo-thiomorpholine-3-carboxylic acid hydroxyamide,
4-[4-(3,5-Difluoro-benzyloxy)-benzenesulfonyl]-2-methyl-1,1-dioxide-thiomorpholine-3-carboxylic acid hydroxyamide,
4-[4-(3-Fluoro-benzyloxy)-benzenesulfonyl]-2-methyl-1-oxo-thiomorpholine-3-carboxylic acid hydroxyamide,
4-[4-(3-Fluoro-benzyloxy)-benzenesulfonyl]-2-methyl-1,1-dioxide-thiomorpholine-3-carboxylic acid hydroxyamide,
4-[4-(3-Chloro-benzyloxy)-benzenesulfonyl]-2-methyl-1,1-dioxide-thiomorpholine-3-carboxylic acid hydroxyamide,
4-[4-(3-Chloro-benzyloxy)-benzenesulfonyl]-2-methyl-1-oxo-thiomorpholine-3-carboxylic acid hydroxyamide,
3-Methyl-1-[4-(2-methyl-benzyloxy)-benzenesulfonyl]-piperazine-2-carboxylic acid hydroxyamide,
1-(4-Benzyloxy-benzenesulfonyl)-3-methyl-piperazine-2-carboxylic acid hydroxyamide,
3-Methyl-1-[4-(2-methyl-pyridin-3-ylmethoxy)-benzenesulfonyl]-piperazine-2-carboxylic acid hydroxyamide,
3-Methyl-1-[4-(2-trifluoromethyl-benzyloxy)-benzenesulfonyl]-piperazine-2-carboxylic acid hydroxyamide,
1-[4-(5-Fluoro-2-trifluoromethyl-benzyloxy)-benzenesulfonyl]-3-methyl-piperazine-2-carboxylic acid hydroxyamide,
1-[4-(5-Fluoro-2-methyl-benzyloxy)-benzenesulfonyl]-3-methyl-piperazine-2-carboxylic acid hydroxyamide,
1-[4-(2,5-Dimethyl-benzyloxy)-benzenesulfonyl]-3-methyl-piperazine-2-carboxylic acid hydroxyamide,
3-Methyl-1-[4-(2-methyl-benzyloxy)-benzenesulfonyl]-piperazine-2-carboxylic acid hydroxyamide,
1-[4-(2-Methyl-benzyloxy)-benzenesulfonyl]-piperazine-2-carboxylic acid hydroxyamide.
4-Methyl-1-[4-(2-methyl-benzyloxy)-benzenesulfonyl]-piperazine-2-carboxylic acid hydroxyamide,
3,4-Dimethyl-1-[4-(2-methyl-benzyloxy)-benzenesulfonyl]-piperazine-2-carboxylic acid hydroxyamide,
1-[4-(2,5-Dimethyl-benzyloxy)-benzenesulfonyl]-3,4-dimethyl-piperazine-2-carboxylic acid hydroxyamide,
1-[4-(5-Fluoro-2-methyl-benzyloxy)-benzenesulfonyl]-3,4-dimethyl-piperazine-2-carboxylic acid hydroxyamide,
1-[4-(5-Fluoro-2-trifluoromethyl-benzyloxy)-benzenesulfonyl]-3,4-dimethyl-piperazine-2-carboxylic acid hydroxyamide,
3,4-Dimethyl-1-[4-(2-trifluoromethyl-benzyloxy)-benzenesulfonyl]-piperazine-2-carboxylic acid hydroxyamide,
3,4-Dimethyl-1-[4-(2-methyl-pyridin-3-ylmethoxy)-benzenesulfonyl]-piperazine-2-carboxylic acid hydroxyamide,
4-Methanesulfonyl-1-[4-(2-methyl-benzyloxy)-benzenesulfonyl]-piperazine-2-carboxylic acid hydroxyamide,
4-Methanesulfonyl-1-[4-(2-trifluoromethyl-benzyloxy)-benzenesulfonyl]-piperazine-2-carboxylic acid hydroxyamide,
1-[4-(5-Fluoro-2-trifluoromethyl-benzyloxy)-benzenesulfonyl]-4-methanesulfonyl-piperazine-2-carboxylic acid hydroxyamide,
1-[4-(2,5-Dimethyl-benzyloxy)-benzenesulfonyl]-4-methanesulfonyl-piperazine-2-carboxylic acid hydroxyamide,
1-[4-(5-Fluoro-2-methyl-benzyloxy)-benzenesulfonyl]-4-methanesulfonyl-piperazine-2-carboxylic acid hydroxyamide,
4-Methanesulfonyl-1-[4-(2-methyl-pyridin-3-ylmethoxy)-benzenesulfonyl]-piperazine-2-carboxylic acid hydroxyamide,
4-Acetyl-1-[4-(2-trifluoromethyl-benzyloxy)-benzenesulfonyl]-piperazine-2-carboxylic acid hydroxyamide,
4-Acetyl-1-[4-(5-fluoro-2-trifluoromethyl-benzyloxy)-benzenesulfonyl]-piperazine-2-carboxylic acid hydroxyamide,
4-Acetyl-1-[4-(5-fluoro-2-methyl-benzyloxy)-benzenesulfonyl]-piperazine-2-carboxylic acid hydroxyamide,
4-Acetyl-1-[4-(2-methyl-benzyloxy)-benzenesulfonyl]-piperazine-2-carboxylic acid hydroxyamide,
4-Acetyl-1-[4-(2,5-dimethyl-benzyloxy)-benzenesulfonyl]-piperazine-2-carboxylic acid hydroxyamide,
4-Acetyl-1-[4-(2-methyl-pyridin-3-ylmethoxy)-benzenesulfonyl]-piperazine-2-carboxylic acid hydroxyamide,
4-Methyl-1-[4-(2-methyl-pyridin-3-ylmethoxy)-benzenesulfonyl]-piperazine-2-carboxylic acid hydroxyamide,
1-[4-(2,5-Dimethyl-benzyloxy)-benzenesulfonyl]-4-methyl-piperazine-2-carboxylic acid hydroxyamide,
4-Acetyl-3-methyl-1-[4-(2-methyl-benzyloxy)-benzenesulfonyl]-piperazine-2-carboxylic acid hydroxyamide,
4-Acetyl-1-[4-(2,5-dimethyl-benzyloxy)-benzenesulfonyl]-3-methyl-piperazine-2-carboxylic acid hydroxyamide,
4-Acetyl-1-[4-(5-fluoro-2-methyl-benzyloxy)-benzenesulfonyl]-3-methyl-piperazine-2-carboxylic acid hydroxyamide,
4-Acetyl-1-[4-(5-fluoro-2-trifluoromethyl-benzyloxy)-benzenesulfonyl]-3-methylpiperazine-2-carboxylic acid hydroxyamide,
4-Acetyl-3-methyl-1-[4-(2-trifluoromethyl-benzyloxy)-benzenesulfonyl]-piperazine-2-carboxylic acid hydroxyamide,
4-Acetyl-3-methyl-1-[4-(2-methyl-pyridin-3-ylmethoxy)-benzenesulfonyl]-piperazine-2-carboxylic acid hydroxyamide,
4-Methanesulfonyl-3-methyl-1-[4-(2-methyl-pyridin-3-ylmethoxy)-benzenesulfonyl]-piperazine-2-carboxylic acid hydroxyamide,
4-Methanesulfonyl-3-methyl-1-[4-(2-trifluoromethyl-benzyloxy)-benzenesulfonyl]-piperazine-2-carboxylic acid hydroxyamide,
1-[4-(5-Fluoro-2-trifluoromethyl-benzyloxy)-benzenesulfonyl]-4-methanesulfonyl-3-methyl-piperazine-2-carboxylic acid hydroxyamide,
1-[4-(5-Fluoro-2-methyl-benzyloxy)-benzenesulfonyl]-4-methanesulfonyl-3-methyl-piperazine-2-carboxylic acid hydroxyamide,
1-[4-(2,5-Dimethyl-benzyloxy)-benzenesulfonyl]-4-methanesulfonyl-3-methylpiperazine-2-carboxylic acid hydroxyamide,
4-Methanesulfonyl-3-methyl-1-[4-(2-methyl-benzyloxy)-benzenesulfonyl]-piperazine-2-carboxylic acid hydroxyamide,
1-[4-(4-Fluoro-benzyloxy)-benzenesulfonyl]-3-methyl-piperazine-2-carboxylic acid hydroxyamide,
1-[4-(4-Fluoro-benzyloxy)-benzenesulfonyl]-3,4-dimethyl-piperazine-2-carboxylic acid hydroxyamide,
4-Acetyl-1-[4-(4-fluoro-benzyloxy)-benzenesulfonyl]-3-methyl-piperazine-2-carboxylic acid hydroxyamide,
1-[4-(4-Fluoro-benzyloxy)-benzenesulfonyl]-4-methanesulfonyl-3-methyl-piperazine-2-carboxylic acid hydroxyamide,
3,5-Dimethyl-1-[4-(2-methyl-benzyloxy)-benzenesulfonyl]-piperazine-2-carboxylic acid hydroxyamide,
3,3-Dimethyl-1-[4-(2-methyl-benzyloxy)-benzenesulfonyl]-piperazine-2-carboxylic acid hydroxyamide,
4-Methanesulfonyl-3,3-dimethyl-1-[4-(2-methyl-benzyloxy)-benzenesulfonyl]-piperazine-2-carboxylic acid hydroxyamide,
1-(4-Benzyloxy-benzenesulfonyl)-4-(4-methoxy-benzenesulfonyl)-3-methylpiperazine-2-carboxylic acid hydroxyamide,
4-[4-(4-Fluoro-benzyloxy)-benzenesulfonyl]-3-hydroxycarbamoyl-2-methyl-piperazine-1-carboxylic acid methyl ester,
4-[4-(4-Fluoro-benzyloxy)-benzenesulfonyl]-2-methyl-piperazine-1,3-dicarboxylic acid 1-dimethylamide 3-hydroxyamide,
4-[4-(2-Ethyl-benzyloxy)-benzenesulfonyl]-2,2,6-trimethyl-morpholine-3-carboxylic acid hydroxyamide,
4-[4-(2-Ethyl-benzyloxy)-benzenesulfonyl]-6-(2-hydroxy-2-methyl-propyl)-2,2-dimethyl-morpholine-3-carboxylic acid hydroxyamide,
4-[4-(2-Ethyl-benzyloxy)-benzenesulfonyl]-6-(2-hydroxy-2-methyl-propyl)-2-methylmorpholine-3-carboxylic acid hydroxyamide,
4-[4-(2-Ethyl-benzyloxy)-benzenesulfonyl]-2,5-dimethyl-morpholine-3-carboxylic acid hydroxyamide,
4-(4-Benzyloxy-benzenesulfonyl)-2,5,6-trimethyl-morpholine-3-carboxylic acid hydroxyamide,
4-(4-Benzyloxy-benzenesulfonyl)-2,2,5,6-tetramethyl-morpholine-3-carboxylic acid hydroxyamide,
4-(4-Benzyloxy-benzenesulfonyl)-5-hydroxymethyl-2,6-dimethyl-morpholine-3-carboxylic acid hydroxyamide,
4-(4-Benzyloxy-benzenesulfonyl)-2-methyl-6-methylcarbamoylmethyl-morpholine-3-carboxylic acid hydroxyamide,
4-(4-Benzyloxy-benzenesulfonyl)-2-isopropyl-6-methyl-morpholine-3-carboxylic acid hydroxyamide, and
4-(5-Benzyloxy-pyridine-2-sulfonyl)-2,6-dimethyl-morpholine-3-carboxylic acid hydroxyamide.

The term "alkyl", as used herein, unless otherwise indicated, includes saturated monovalent hydrocarbon radicals having straight, branched or cyclic moieties or combinations thereof.

The term "alkoxy", as used herein, includes O-alkyl groups wherein "alkyl" is defined above.

The term "aryl", as used herein, unless otherwise indicated, includes an organic radical derived from an aromatic hydrocarbon by removal of one hydrogen, such as phenyl or naphthyl, optionally substituted by 1 to 3 suitable substituents such as fluoro, chloro, cyano, nitro, trifluoromethyl, (C₁-C₆)alkoxy, (C₆-C₁₀)aryloxy, trifluoromethoxy, difluoromethoxy and (C₁-C₆)alkyl.

The term "heteroaryl", as used herein, unless otherwise indicated, includes an organic radical derived from an aromatic heterocyclic compound by removal of one hydrogen, such as pyridyl, furyl, pyroyl, thienyl, isothiazolyl, imidazolyl, benzimidazolyl, tetrazolyl, pyrazinyl, pyrimidyl, quinolyl, isoquinolyl, benzofuryl, isobenzofuryl, benzothienyl, pyrazolyl, indolyl, isoindolyl, purinyl, carbazolyl, isoxazolyl, thiazolyl, oxazolyl, benzthiazolyl or benzoxazolyl, optionally substituted by 1 to 3 suitable substituents such as fluoro, chloro, trifluoromethyl, (C₁-C₆)alkoxy, (C₆-C₁₀)aryloxy, trifluoromethoxy, difluoromethoxy and (C₁-C₆)alkyl.

The term "acyl", as used herein, unless otherwise indicated, includes a radical of the general formula RCO wherein R is alkyl, alkoxy (such as methyloxy carbonyl), aryl, arylalkyl or arylalkyloxy and the terms "alkyl" or "aryl" are as defined above.

The term "acyloxy", as used herein, includes O-acyl groups wherein "acyl" is defined above.

"A suitable substituent" is intended to mean a chemically and pharmaceutically acceptable functional group i.e., a moiety that does not negate the inhibitory activity of the inventive compounds. Such suitable substituents may be routinely selected by those skilled in the art. Illustrative examples of suitable substituents include, but are not limited to, alkyl groups, hydroxy groups, oxo groups, mercapto groups, alkylthio groups, alkoxy groups, aryl or heteroaryl groups, aralkyl or heteroaralkyl groups, aralkoxy or heteroaralkoxy groups, carboxy groups, amino groups, alkyl- and dialkylamino groups, carbamoyl groups, alkylcarbonyl groups, alkoxycarbonyl groups, alkylaminocarbonyl groups dialkyamino carbonyl groups, arylcarbonyl groups, aryloxycarbonyl groups, alkylsulfonyl groups, an arylsulfonyl groups and the like.

The term "D- or L-amino acid", as used herein, unless otherwise indicated, includes glycine, alanine, valine, leucine, isoleucine, phenylalanine, asparagine, glutamine, tryptophan, proline, serine, threonine, tyrosine, hydroxyproline, cysteine, cystine, methionine, aspartic acid, glutamic acid, lysine, arginine or histidine.

*The positions on the ring of formula I, as used herein, are defined as follows:

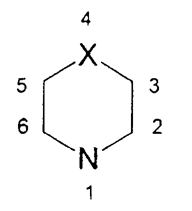

The compound of formula I may have chiral centers and therefore exist in different enantiomeric forms. This invention relates to all optical isomers, diasteriomers, atropisomers, stereoisomers and tautomers of the compounds of formula I and mixtures thereof.

A small molecule as used herein refers to non-DNA, non-RNA, non-polypeptide and non-monoclonal antibody molecules with a molecular weight of under 1000 AMV. Preferred small molecules possess a hydroxamic acid group (-(C=O)(NH)OH), a heterocyclic group, a sulfonamide group, and/or an aryl group.

An "agent" as used herein (e.g. agent that selectively inhibits TNF-α) refers to any chemical or pharmaceutical molecule that possesses the inhibitory activity claimed, such as DNA, RNA, antisense or sense oligonucleotide, monoclonal or polyclonal antibody or small moleule.

The present invention also relates to the pharmaceutically acceptable acid addition salts of compounds of the formula I. The acids which are used to prepare the pharmaceutically acceptable acid addition salts of the aforementioned base compounds of this invention are those which form non-toxic acid addition salts, i.e., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [i.e., 1,1'-methylene-bis-(2-hydroxy-3-naphthoate)]salts.

The invention also relates to base addition salts of formula I. The chemical bases that may be used as reagents to prepare pharmaceutically acceptable base salts of those compounds of formula I that are acidic in nature are those that form non-toxic base salts with such compounds. Such non-toxic base salts include, but are not limited to those derived from such pharmacologically acceptable cations such as alkali metal cations (e.g., potassium and sodium) and alkaline earth metal cations (e.g., calcium and magnesium), ammonium or watersoluble amine addition salts such as N-methylglucamine-(meglumine), and the lower alkanolammonium and other base salts of pharmaceutically acceptable organic amines.

The subject invention also includes isotopically-labelled compounds, which are identical to those recited in Formula I, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine and chlorine, such as ²H, ³H, ¹³C, ¹⁴C, ¹⁵N, ¹⁸O, ¹⁷O, ³¹P, ³²P, ³⁵S, ¹⁸F, and ³⁶Cl, respectively. Compounds of the present invention, prodrugs thereof, and pharmaceutically acceptable salts of said compounds or of said prodrugs which contain the aforementioned isotopes and/or other isotopes of other atoms are within the scope of this invention. Certain isotopically-labelled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically labelled compounds of Formula I of this invention and prodrugs thereof can generally be prepared by carrying out the procedures disclosed in the Schemes and/or in the Examples and Preparations below, by substituting a readily available isotopically labelled reagent for a non-isotopically labelled reagent.

The present invention also relates to a pharmaceutical composition for the treatment of a condition selected from the group consisting of arthritis (including osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity, cachexia, allergic reactions, allergic contact hypersensitivity, cancer (such as solid tumor cancer including colon cancer breast cancer, lung cancer and prostrate cancer and hematopoietic malignancies including leukemias and lymphomas), tissue ulceration, restenosis, periodontal disease, epidermolysis bullosa, osteoporosis, loosening of artificial joint implants, atherosclerosis (including atherosclerotic plaque rupture), aortic aneurysm (including abdominal aortic aneurysm and brain aortic aneurysm), congestive heart failure, myocardial infarction, stroke, cerebral ischemia, head trauma, spinal cord injury, neuro-degenerative disorders (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, bums, diabetes, tumor invasion, tumor growth, tumor metastasis, corneal scarring, scleritis, AIDS, sepsis and septic shock in a mammal, including a human, comprising an amount of a compound of formula I or a pharmaceutically acceptable salt thereof effective in such treatments and a pharmaceutically acceptable carrier.

The present invention also relates to the use of a compound of formula I or a pharmaceutically acceptable salt thereof, for the manufacture of a medicament for treating a condition selected from the group consisting of arthritis (including osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity, cachexia, allergic reactions, allergic contact hypersensitivity, cancer (such as solid tumor cancer including colon cancer breast cancer, lung cancer and prostrate cancer and hematopoietic malignancies including leukemias and lymphomas), tissue ulceration, restenosis, periodontal disease, epidermolysis bullosa, osteoporosis, loosening of artificial joint implants, atherosclerosis (including atherosclerotic plaque rupture), aortic aneurysm (including abdominal aortic aneurysm and brain aortic aneurysm), congestive heart failure, myocardial infarction, stroke, cerebral ischemia, head trauma, spinal cord injury, neuro-degenerative disorders (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, bums, diabetes, tumor invasion, tumor growth, tumor metastasis, corneal scarring, scleritis, AIDS, sepsis and septic shock.

The term "treating", as used herein, refers to reversing, alleviating, inhibiting the progress of, or preventing the disorder or condition to which such term applies, or one or more symptoms of such disorder or condition. The term "treatment", as used herein, refers to the act of treating, as "treating" is defined immediately above.

Compounds of formula I having free amino, amido, hydroxy or carboxylic groups can be converted into prodrugs. Prodrugs include compounds wherein an amino acid residue, or a polypeptide chain of two or more (e.g., two, three or four) amino acid residues which are covalently joined through peptide bonds to free amino, hydroxy or carboxylic acid groups of compounds of formula I. The amino acid residues include the 20 naturally occurring amino acids commonly designated by three letter symbols and also include, 4-hydroxyproline, hydroxylysine, citrulline, homocysteine, homoserine, ornithine and methionine sulfone. Prodrugs also include compounds wherein carbonates, carbamates, amides and alkyl esters which are covalently bonded to the above substituents of formula I through the carbonyl carbon prodrug sidechain.

One of ordinary skill in the art will appreciate that the compounds of the invention are useful in treating a diverse array of diseases. One of ordinary skill in the art will also appreciate that when using the compounds of the invention in the treatment of a specific disease that the compounds of the invention may be combined with various existing therapeutic agents used for that disease.

For the treatment of rheumatoid arthritis, the compounds of the invention may be combined with agents such as TNF-α inhibitors such as anti-TNF monoclonal antibodies and TNF receptor immunoglobulin molecules (such as Enbrel®), COX-2 inhibitors low dose methotrexate, lefunimide, hydroxychloroquine, d-penicilamine, auranofin or parenteral or oral gold.

The compounds of the invention can also be used in combination with existing therapeutic agents for the treatment of osteoarthritis. Suitable agents to be used in combination include standard non-steroidal anti-inflammatory agents (hereinafter NSAID's) such as piroxicam, diclofenac, propionic acids such as naproxen, flubiprofen, fenoprofen, ketoprofen and ibuprofen, fenamates such as mefenamic acid, indomethacin, sulindac, apazone, pyrazolones such as phenylbutazone, salicylates such as aspirin, COX-2 inhibitors such as celecoxib and rofecoxib, analgesics and intraarticular therapies such as corticosteroids and hyaluronic acids such as hyalgan and synvisc.

The compounds of the present invention may also be used in combination with anticancer agents such as endostatin and angiostatin or cytotoxic drugs such as adriamycin, daunomycin, cis-platinum, etoposide, taxol, taxotere and alkaloids, such as vincristine, and antimetabolites such as methotrexate.

The compounds of the present invention may also be used in combination with cardiovascular agents such as calcium channel blockers, lipid lowering agents such as statins, fibrates, beta-blockers, Ace inhibitors, Angiotensin-2 receptor antagonists and platelet aggregation inhibitors.

The compounds of the present invention may also be used in combination with CNS agents such as antidepressants (such as sertraline), anti-Parkinsonian drugs (such as deprenyl, L-dopa, requip, miratex, MAOB inhibitors such as selegine and rasagiline, comP inhibitors such as Tasmar, A-2 inhibitors, dopamine reuptake inhibitors, NMDA antagonists, Nicotine agonists, Dopamine agonists and inhibitors of neuronal nitric oxide synthase), and anti-Alzheimer's drugs such as donepezil, tacrine, COX-2 inhibitors, propentofylline or metryfonate.

The compounds of the present invention may also be used in combination with osteoporosis agents such as roloxifene, droloxilene or fosomax and immunosuppressant agents such as FK-506 and rapamycin.

### Detailed Description of the Invention

The following reaction Schemes illustrate the preparation of the compounds of the present invention. Unless otherwise indicated Q, X, and R¹-R⁹ in the reaction Schemes and the discussion that follow are defined as above.

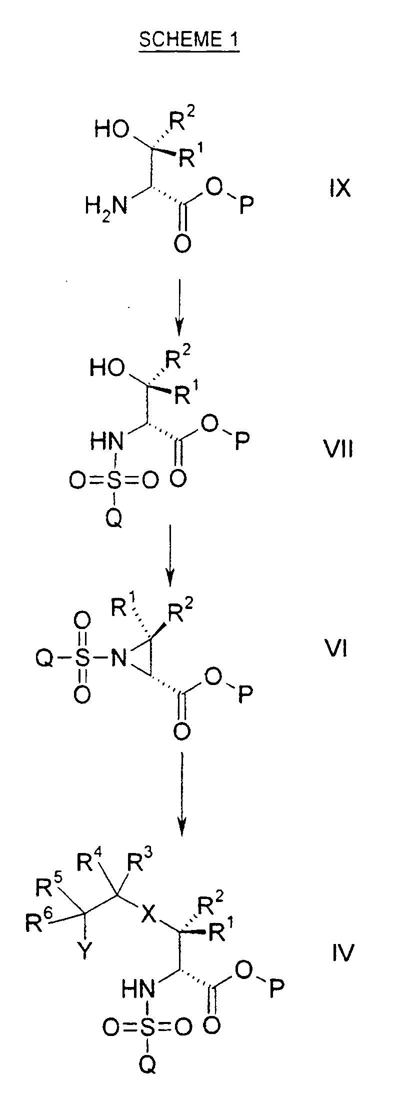

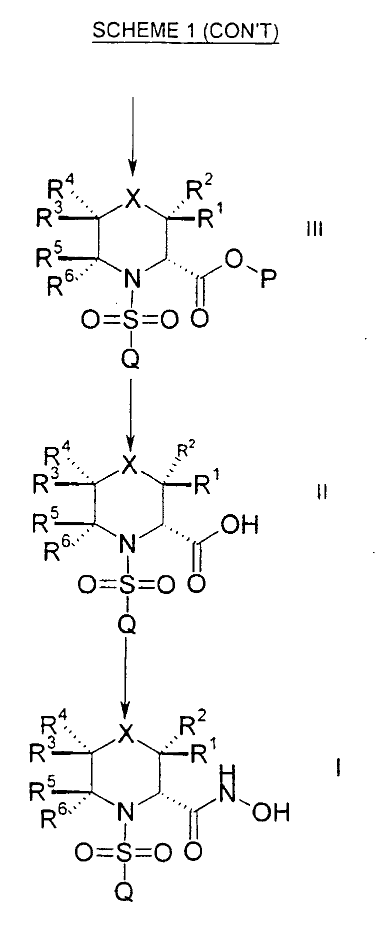

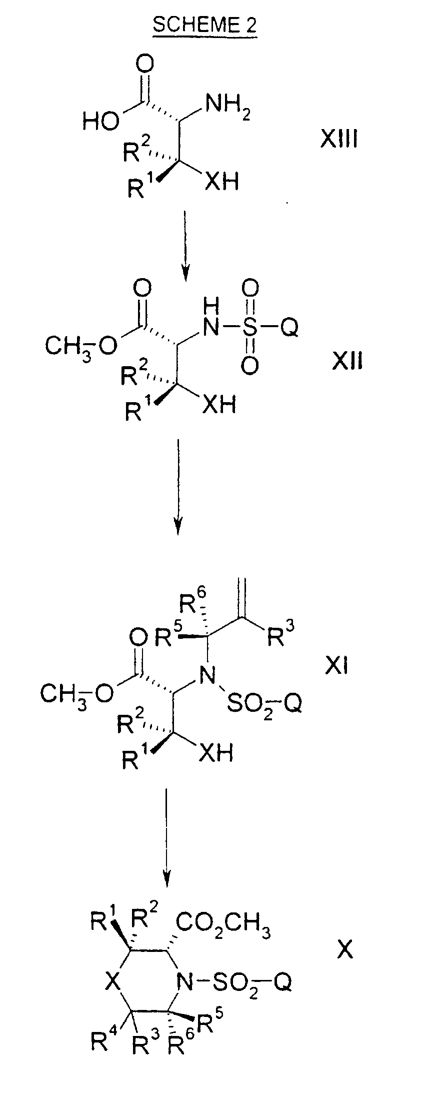

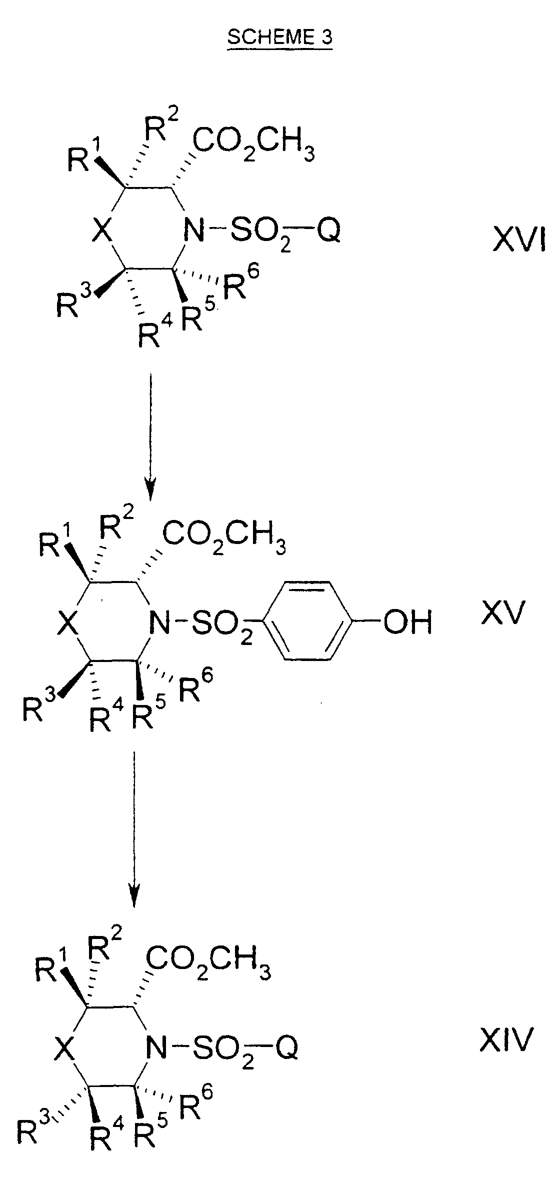

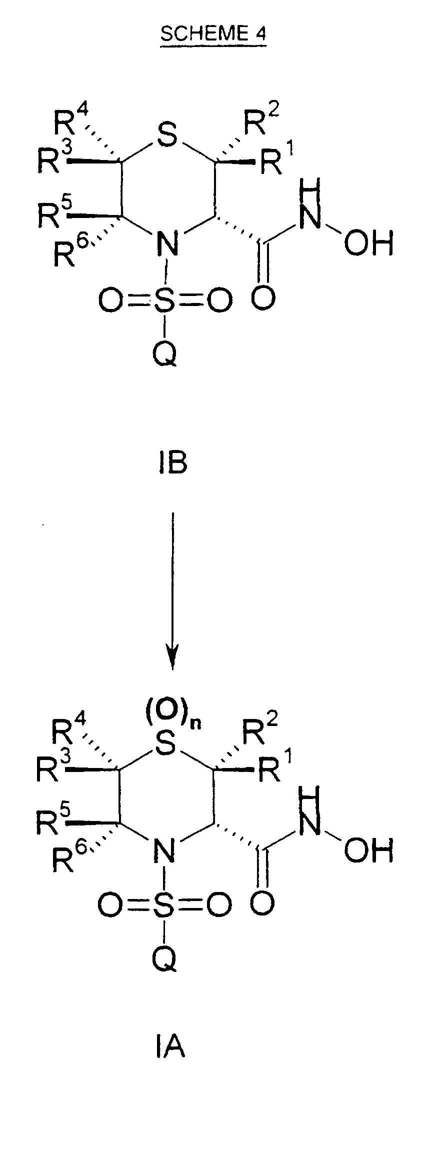

Scheme 1 refers to the preparation of compounds of the formula I. Referring to Scheme 1, compounds of formula I are prepared from compounds of formula II by activation of the carboxylic acid moiety in compounds of formula II followed by treatment of the activated acid with a hydroxylamine or a protected hydroxylamine equivalent that is then deprotected to form the hydroxamic acid. Activation of the carboxyl group of formula II is achieved through the action of a suitable activating agent such as dialkyl carbodiimides, benzotriazol-1-yloxyl)tris(dialkylamino)-phosphonium salts, or oxalyl chloride in the presence of a catalytic amount of *N*,*N*-dimethylformamide. Preferably the activating agent is benzotriazol-1-yloxy-tris(dimethylamino)phosphonium hexafluorophosphate. Generally, the hydroxylamine or protected hydroxylamine equivalent is generated *in situ* from a salt form, such as hydroxylamine hydrochloride, in the presence of an amine base such as triethylamine, or diisopropylethylamine. Suitable protected hydroxylamines include *O*-*tert*-butylhydroxylamine, *O*-allylhydroxylamine, *O*-tert-butyldimethylsilylhydroxylamine, *O*-trimethylsilylethylhydroxylamine, *O*-benzylhydroxylamine, or *N,O-bis* trimethylsilylhydroxylamine. Removal of the protecting group is carried out by hydrogenolysis in the instance where *O*-benzylhydroxylamine is used (5% palladium on barium sulfate is the preferred catalyst) or by treatment with a strong acid such as trifluoroacetic acid in the situation where *O-tert*-butylhydroxylamine or *O*-trimethylsilylethylhydroxylamine is used. When *O*-allylhydroxylamine is employed, the allyl group is removed either by treatment with ammonium formate in the presence of a catalytic amount of tetrakis(triphenylphosphine)palladium(0) in aqueous acetonitrile at 60 °C or by treatment with piperidine in the presence of a catalytic amount of allylpalladium chloride dimer and diphenylphosphinoethane in tetrahydrofuran at 23 °C. In the case where *N,O-bis*-trimethylsilylhydroxylamine is used (preferably generated *in situ* from trimethylsilylchloride and hydroxylamine hydrochloride in pyridine at 0 °C), the silyl protective groups are removed by treatment with dilute aqueous acid such as 1 N hydrochloric acid. Suitable solvents for the aforesaid activation and hydroxylamine reaction include methylene chloride, *N,N*-dimethylformamide, or tetrahydrofuran, preferably methylene chloride. The aforesaid activation and hydroxylamine reactions are run at temperatures between about 0 °C to about 60 °C (23 °C is preferred) for periods of time between about 1 hour and about 20 hours (4 hours is preferred).

Compounds of formula II are prepared from compounds of formula III by removal of the protective group P to form a carboxylic acid. In the case where the protecting group P is *tert*-butyl, this conversion is accomplished through the action of a suitably strong acid such as hydrochloric acid or trifluoroacetic acid (trifluoroacetic acid is preferred). Preferably this reaction is conducted in a solvent such as ethyl acetate, 1,4-dioxane, or methylene chloride (methylene chloride is preferred). In cases where the protecting group P is methyl or ethyl, this conversion is achieved by saponification with a suitable source of hydroxide such as sodium or lithium hydroxide (lithium hydroxide is preferred). Preferably the saponification is conducted with stirring, in an aqueous solvent mixture such as tetrahydrofuran-methanol-water or 1,4-dioxane-methanol-water at a temperature between about 0°C to near the boiling point of the solvent system (60 °C is preferred). In cases where the protecting group P is trialkylsilyl, the silyl group can be removed by treatment with dilute aqueous acid such as dilute hydrochloric acid, in aqueous methanol or by heating in methanol at reflux. In cases where the protecting group P is benzyl, the conversion is achieved by hydrogenolysis of the benzyl group. The hydrogenolysis is carried out in a suitable solvent such as ethanol, methanol, or ethyl acetate under an atmosphere of hydrogen, in the presence of a catalyst such a 10% palladium on carbon. Generally, reactions involving the removal of protecting group P are run for periods of time between about 30 minutes to about 8 hours, preferably about 4 hours. Unless otherwise mentioned, the aforesaid reactions are performed at a temperature from about 0°C to about 25°C, preferably about 23°C.

Alternatively compounds of formula III can be converted directly to compounds of formula I through the action of hydroxylamine. Preferably, the protecting group P is methyl. Suitable solvents include methanol, ethanol, or 2-propanol, preferably methanol. For this reaction the preferred method for generating the hydroxylamine is by treatment of hydroxylamine hydrochloride with potassium hydroxide. The reaction is performed at a temperature between about 0 °C to about 23 °C (0 °C is preferred) for a period of time from about 10 minutes to about 4 hours (2 hours is preferred).

Compounds of the formula III, i.e. thiomorpholines, morpholines, or piperazines, are prepared from compounds of formula IV, wherein Y is halo or hydroxy by intramolecular ring cyclization methods specific to the nature of the Y group. In cases where Y is chloro or bromo the ring can form spontaneously or by treatment with a suitable base such as diisopropylethylamine, or an alkaline earth carbonate. Preferably this closure is conducted in a solvent such as tetrahydrofuran, benzene, chloroform, or *N,N*-dimethylformamide (tetrahydrofuran is preferred). The reaction is preferably stirred at a temperature from about ambient temperature (22 °C) to the boiling point of the solvent used for a period of time from about 30 minutes to about 24 hours (12 hours is preferred). In cases where Y is hydroxy, the hydroxyl group is preferably activated through the action of a complex generated by the mixture of a trialkylphosphine and a dialkyl azodicarboxylate (triphenylphosphine and dimethyl azodicarboxylate are preferred) in a suitable solvent such as tetrahydrofuran. The preferred sequence involves the addition of compounds of formula III to a the pre-formed complex of the trialkylphosphine and a dialkyl azodicarboxylate. The reaction is stirred at a temperature between about 0 °C to about 25 °C, preferably at about 0°C for a period of time between about 10 minutes to about 4 hours, followed by a period of about 16 hours at 23 °C.

Compounds of the formula IV are prepared by reaction of a compound of the formula VI with a compound of the formula 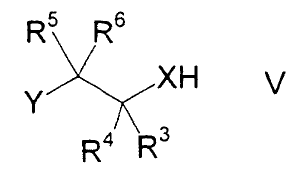 wherein R³, R⁴, R⁵, R⁶, and Y are as defined above, and X is oxygen, sulfur, or NR⁷, wherein R⁷ is described above. Preferably the reaction is run in a suitable solvent such as chloroform, methylene chloride, tetrahydrofuran, or benzene (methylene chloride is preferred), in the presence of a catalyst such as a Lewis acid such as zinc chloride, magnesium chloride, or borontrifluoride etherate (borontrifluoride etherate is preferred). The reaction is stirred at a temperature between about 0 °C to the boiling point of the solvent used, preferably at ambient temperature (22-23°C), for a time period between about 1 hour to about 4 days, preferably about 2 days.

Compounds of the formula VI containing an aziridine ring are prepared from compounds of formula VII by activation of the hydroxyl group, to form a leaving group, followed by intramolecular cyclization. Preferably this activation is achieved by the conversion of the alcohol to the corresponding sulfonate ester (mesyl is preferred), or through the action of a complex generated by the mixture of a trialkylphosphine and a dialkyl azodicarboxylate (triphenylphosphine and dimethyl azodicarboxylate are preferred) in a suitable solvent such as tetrahydrofuran. In the former case of the sulfonate, the aziridine ring is preferably formed by subsequent treatment with a base such as diisopropylethylamine or potassium *tert*-butoxide. In the latter case, the preferred sequence involves the addition of compounds of the formula VII to the pre-formed complex of the trialkylphosphine and a dialkyl azodicarboxylate. The reaction is stirred at a temperature between about 0 °C to about 25 °C, preferably at 0 °C for a period of time between about 10 minutes to about 4 hours, followed by a period of about 16 hours at 23 °C.

Compounds of the formula VII, wherein P is methyl, ethyl, *tert*-butyl, trialkylsilyl, or benzyl (methyl and *tert*-butyl are preferred), are prepared by reacting a protected amino acid of formula IX, wherein P is methyl, ethyl, *tert*-butyl, trialkylsilyl, or benzyl (methyl and *tert*-butyl are preferred), with a sulfonyl chloride of the formula 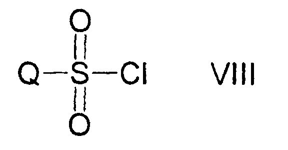 in the presence of a base in a reaction inert solvent. Suitable solvents include methylene chloride, tetrahydrofuran, *N,N*-dimethylformamide, or a mixture of 1,4-dioxane and water, or a mixture of ethyl acetate and water. Suitable bases include triethylamine, diisopropylethylamine, or an alkaline earth carbonate or hydroxide. The use of methylene chloride as solvent and diisopropylethylamine as the base is preferred. The reaction is stirred at a temperature between about 0 °C to about 25 °C, preferably at 0 °C, for a time period between about 10 minutes to about 1 day, preferably about 12 hours.

Compounds of formula IX are protected amino acids and are commercially available or can be prepared in ways well known to one of ordinary skill in the art by methods including, but not limited to those described in Preparation 2, by Williams in "Synthesis of Optically Active α-Amino Acids" Baldwin, J. E., Ed., Pergamon Press, Oxford, 1989, or by Coppola and Schuster in "Asymmetric Synthesis. Construction of Chiral Molecules Using Amino Acids", John Wiley & Son; New York, 1987, and references cited therein.

Compounds of formula VIII are commercially available or can be made according to the methods of Preparation 2. Compounds of the formula V are commercially available or can be made by methods well known to those of ordinary skill in the art. Methods for the preparation of compounds of formula V include the procedures described by Einhorn in "An Easy and Efficient Epoxide Opening to give Halohydrins using Tin(II) Halides" J. Chem. Soc. Chem. Comm. 1368-1369 (1986), by Cambie in "Reactions of Alkenes with Electrophilic Iodine in Tetramethylene Sulphone-Chloroform" J. Chem. Soc. Perkin Trans I 226-230 (1986), and by Ciacco in "Dilithium Tetrachlorocuprate. A Reagent for Regioselective Cleavage of Epoxides to Chlorohydrins" Tetrahedron Lett. 27 3697-3700 (1986). It is understood to one skilled in the art that additional chemical modifications of the products generated my methods described in the above references can lead to further analogs of formula V.

Scheme 2 refers to the preparation of compounds of the formula X. Compounds of formula X are compounds of the formula III, in Scheme 1, wherein P is methyl. Compounds of the formula X can be converted to compounds of the formula I according to the methods of Scheme 1. Referring to Scheme 2, compounds of the formula X are prepared from compounds of the formula XI by a cyclization reaction. One of ordinary skill in the art will appreciate that R³ in formula XI can be either R³ or R⁴, as result of which, R³ and R⁴ in formula X are depicted without stereochemistry. Preferably, this cyclization reaction is conducted by activation or oxidative cleavage of the olefin. It is understood by one of ordinary skill in the art that the nature of the olefin activation or olefin cleavage, will determine the nature of one of either R³ or R⁴. One of ordinary skill in the art will also appreciate that in some instances the X group of formula XI will need temporary protection by a protective group prior to olefin activation or oxidative cleavage of the olefin. Methods of olefin activation include treatment of compounds of formula XI with electrophilic agents such as strong acids, mercuric salts, palladium (II), iodine, peracids. One such method involves the treatment of compounds of formula XI with a mercury (II) salt. The resulting intermediate organomercurial complex can be treated with a variety of reagents to give compounds of formula X. Suitable mercury salts include mercury (II) acetate and mercury(II) trifluoroacetate (mercury(II) trifluoroacetate is preferred). Preferably the aforesaid reaction is run in the presence of a base such as potassium carbonate, in a solvent such as tetrahydrofuran, at a temperature of about -10°C to about 30°C, preferably 0°C to about 25°C for a period from about 30 minutes to about 2 hours, preferably about 1 hour. Suitable reagents for the transformation of the intermediate organomercurial complex to compounds of formula X include, but are not limited to, sodium-amalgam, sodium borohydride, sodium borohydride in the presence of triethyl borane, sodium borohydride in the presence of oxygen, and iodine. The use of sodium borohydride with triethyl borane is the preferred method to replace the mercury with a hydrogen atom. The use of sodium borohydride in the presence of oxygen is the preferred method for replacing the mercury with a hydroxyl group. The use of iodine is the preferred method of replacing the mercury with iodide. One of ordinary skill in the art will appreciate that the compounds of formula X derived by replacement of the mercury atom can be further functionalized to give additional compounds of formula X. Reactions of the organomercurial complex are performed in a solvent such as tetrahydrofuran or methylene chloride (tetrahydrofuran is preferred) at a temperature of about -10°C to about 20°C, for a period from about 30 minutes to about 2 hours, preferably about 1 hour. Alternatively, the olefin can be oxidatively cleaved by methods that include the treatment of compounds of the formula XI with ozone, ruthenium tetroxide, or osmium tetroxide and sodium metaperiodate to give compounds of formula X. The preferred method of olefin cleavage involves the use of osmium tetroxide and sodium metaperiodate. Preferably the olefin cleavage is performed in a solvent mixture such as carbon tetrachloride and water, a mixture of dioxane and water, and tetrahydrofuran and water, for a period of time from about 1 hour to about 24 hours, 12 hours is preferred.

The compounds of formula XI are prepared from a compounds of formula XII by reaction with a suitable allylic species such as an allylic halide, an allylic sulfonate ester, or an allylic acetate. One of ordinary skill in the art will understand that such allylic species are commercially available or readily prepared from commercial sources. Preferably the allylation reaction is run in the presence of a suitable base or catalyst. Suitable bases include trialkylamines such as diisopropylethylamine, alkaline earth carbonates, and sodium hydride. Suitable catalysts include palladium(0), nickel(0), tungsten(0), or molybdenum salts in the presence of ligands such as trialkylphosphines, cyclooctadiene, dibenzylidene acetone, carbon monoxide, and acetylacetonate. Preferably, the allylation reactions are run in a solvent such as *N,N*-dimethyl formamide, tetrahydrofuran, or methylene chloride. One of ordinary skill in the art will also appreciate that in some instances the X group of formula XII will need temporary protection by a protective group prior to allylation. The reaction of compounds of formula XII with allyl iodide in the presence of cesium carbonate, in *N,N*-dimethyl formamide for a period of time from about 1 hour to 24 hours (2.5 hours is preferred) at a temperature from about 0°C to about 60°C (23 °C is preferred) is the preferred method for the allylation reaction.

The compound of formula XII is prepared from a compound of formula XIII by the formation of the corresponding methyl ester followed by reaction with an arylsulfonyl chloride of the formula QSO₂Cl (wherein Q is as defined above). The methyl ester can be formed by treatment of compounds of formula XIII with a strong acid such as toluene sulfonic acid, hydrochloric acid, or sulfuric acid in methanol. The use of anhydrous hydrochloric acid gas or hydrochloric acid generated by addition of thionyl chloride to methanol is preferred. The aforesaid reaction is performed at a temperature of about 0°C to about the boiling point of the methanol for a period from about 1 hour to about 24hours (14 hours is preferred). Preferably the reaction of the arylsulfonyl chloride of the formula QSO₂Cl with the methyl ester is conducted in a solvent such as methylene chloride, dioxane and water, *N,N*-dimethyl formamide, or tetrahydrofuran (methylene chloride is preferred) , in the presence of a base such as diisopropylethylamine or potassium carbonate (diisopropylethylamine is preferred), for a time from about 2 hours to about 2 days (14 hours is preferred), at temperature ranging from about 0 °C to about 60 °C (23 °C is preferred).

Compounds of the formula XIII are commercially available or can be made by methods well known to those of ordinary skill in the art such as the methods described in Preparation 1 and by Goodman in "An Enantiomeric Synthesis of allo-Threonine and β-Hydroxyvalines." J. Org. Chem. **61** 2582-2583 (1996) and references cited therein. Compounds of the formula QSO₂Cl can be made according to the methods described in PCT Publication WO 98/07697, published February 26, 1998, and PCT publication WO 98/33768 published August 6, 1998.

Scheme 3 refers to a process of introducing different Q groups into compounds of the formula XIV. Compounds of formula XIV and XVI are compounds of formula III in Scheme 1, wherein P is methyl. Compounds of the formula XIV can be converted to compounds of the formula I according to the methods of Scheme 1. Referring to Scheme 3, Compounds of the formula XIV can be prepared by treatment of a compound of the formula XV with an optionally substituted (C₆-C₁₀)aryl(C₁-C₆)alkyl halide or (C₂-C₉)heteroaryl(C₁-C₆)alkyl halide (preferably a bromide or chloride) in the presence of a base such as cesium carbonate in a polar solvent such as *N,N*-dimethylformamide. The reaction mixture is stirred at a temperature from about 0 °C to about 60 °C, preferably at about 40 °C for a period from about 30 minutes to about 4 hours, preferably about 1 hour.

The compound of formula XV can be prepared from a compound of formula XVI by cleaving agent. Suitable cleaving agents include trimethylsilyl iodide, boron trichloride, and boron tribromide, (boron tribromide is preferred). Preferably the cleavage reaction is run in a suitable solvent such as methylene chloride at a temperature from about -78 °C to about 23 °C for a period of time from about 1 hour to about 8 hours. Alternatively, the cleavage can be performed by hydrogenolysis when X in formula XV is not sulfur,. Preferably the hydrogenolysis is carried out in the presence of a suitable catalyst such as 10% palladium on carbon, in a suitable solvent such as methanol, ethanol, or ethyl acetate (methanol is preferred), under an atmosphere of hydrogen gas (35 psi is preferred), at ambient temperature, for a period of time from about 4 hour to about 24 (12 hours is preferred).

The compound of formula XVI is a compound of formula III from Scheme 1, wherein P is methyl and Q terminates in a benzylic group. Optionally substituted (C₆-C₁₀)aryl(C₁-C₆)alkyl halide or (C₂-C₉)heteroaryl(C₁-C₆)alkyl halide (preferably a bromide or chloride) are commercially available or can be made by methods well known to those of ordinary skill in the art.

Scheme 4 refers to the preparation of compounds of formula I, wherein X is SO or SO₂, from other compounds of formula I, wherein X is S. Referring to Scheme 4, compounds of formula IB, where X is sulfur are converted to sulfoxides (n=1) or sulfones (n=2) of formula IA by selective oxidation of the sulfur atom. Preferably this oxidation is conducted with a mild oxidizing agent such as "Oxone"™ (potassium peroxymonosulfate, Aldrich Chemical Co.), sodium periodate, or sodium perborate tetrahydrate (Oxone is preferred). Preferably, the oxidation reaction is run in a suitable solvent or solvent mixture, such as methanol-water, acetone-water, tetrahydrofuran-methanol-water, or 1,4-dioxane-methanol-water (methanol-water is preferred). The reaction is stirred at a temperature between about 0 °C to about 25 °C, preferably at 22 °C for a period of time between about 5 minutes to about 4 hours, preferably for about 30 minutes.

Compounds of formula IB are prepared according to the methods of Scheme 1.

The ability of the compounds of formula I or their pharmaceutically acceptable salts (hereinafter also referred to as the compounds of the present invention) to inhibit metalloproteinases or mammalian reprolysin and, consequently, demonstrate their effectiveness for treating diseases characterized by metalloproteinase or the production of tumor necrosis factor is shown by the following in vitro assay tests.

### Biological Assay

### Inhibition of soluble TNF Production

The ability of the compounds or the pharmaceutically acceptable salts thereof to inhibit the cellular production/release of TNF and, consequently, demonstrate their effectiveness for treating diseases involving the dysregulated of TNF is shown by the following in vitro assay:

### Method for the evaluation of recombinant TNFα Converting Enzyme Activity.

### Preparation of recombinant TACE:

A DNA fragment coding for the signal sequence, prodomain and catalytic domain of TACE (amino acids 1-473), was amplified by polymerase chain reaction using a human lung cDNA library as a template. The amplified fragment was cloned into pFastBac vector. The DNA sequence of the insert was confirmed for both the strands. A bacmid prepared using pFastBac in E. coli DH10Bac was transfected into SF9 insect cells. The virus particles were amplified to P1, P2, P3 stages. The P3 virus was infected into both Sf9 and High Five insect cells and grown at 27°C for 48 hours. The medium was collected and used for assays and further purification.

### Preparation of fluorescent quenched substrate:

A model peptidic TNF-α substrate (LY-LeucineAlanineGlutamineAlanineValine-ArginineSerine-SerineLysine(CMTR)-Arginine (LY=Lucifer Yellow; CMTR= 5-carboxytetramethyl Rhodamine)) was prepared and the concentration estimated by absorbance at 560 nm (E₅₆₀, 60,000 M-1CM-1) according to the method of Geoghegan, KF, "Improved method for converting an unmodified peptide to an energy-transfer substrate for a proteinase." Bioconjugate Chem. **7,** 385-391 (1995). This peptide encompasses the cleavage cite on pro-TNF which is cleaved in vivo by TACE.

### Enzyme reaction.

The reaction, carried out in a 96 well plate (Dynatech), was comprised of 70 µl of buffer solution (25 mM Hepes-HCl, pH7.5, plus 20 uM ZnCl₂), 10 µl of 100 µM fluorescent quenched substrate, 10 µl of a DMSO (5%) solution of test compound, and an amount of r-TACE enzyme which will cause 50% cleavage in 60 minutes - in a total volume of 100 µl. The specificity of the enzyme cleavage at the amide bond between alanine and valine was verified by HPLC and mass spectrometry. Initial rates of cleavage were monitored by measuring the rate of increase in fluorescence at 530 nm (excitation at 409 nm) over 30 minutes. The experiment was controlled as follows: 1) for background fluorescence of substrate; 2) for fluorescence of fully cleaved substrate; 3) for fluorescence quenching or augmentation from solutions containing test compound.

Data was analyzed as follows. The rates from the non-test compound containing "control" reactions were averaged to establish the 100% value. The rate of reaction in the presence of test compound was compared to that in the absence of compound, and tabulated as "percent of non-test compound containing control. The results were plotted as "% of control" vs. the log of compound concentration and a half-maximal point or IC₅₀ value determined. The IC₅₀ for the above assay is a measure of the inhibition of the TNF-α proteolytic activity of TACE. Blockage of binding of TNF-α to TACE as used herein is as described in United States Patents 5,830,742, issued November 3, 1998.

Preferred compounds of the invention are at least 100 fold less potent against r-MMP-1 than in the above TACE assay. Table A reports the activity of representative compounds of the invention for TACE, MMP-1 and MMP-13 inhibition.

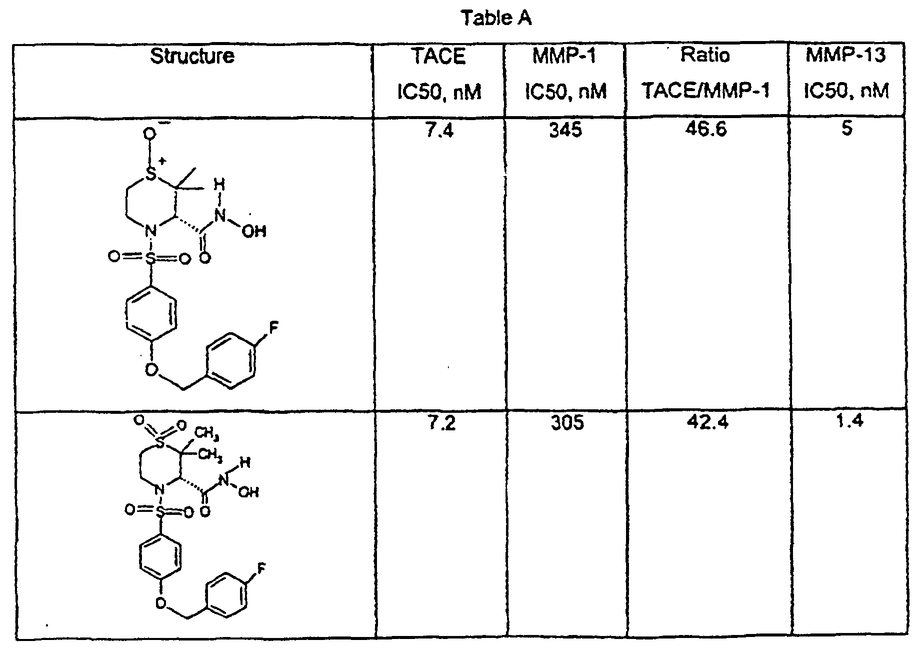

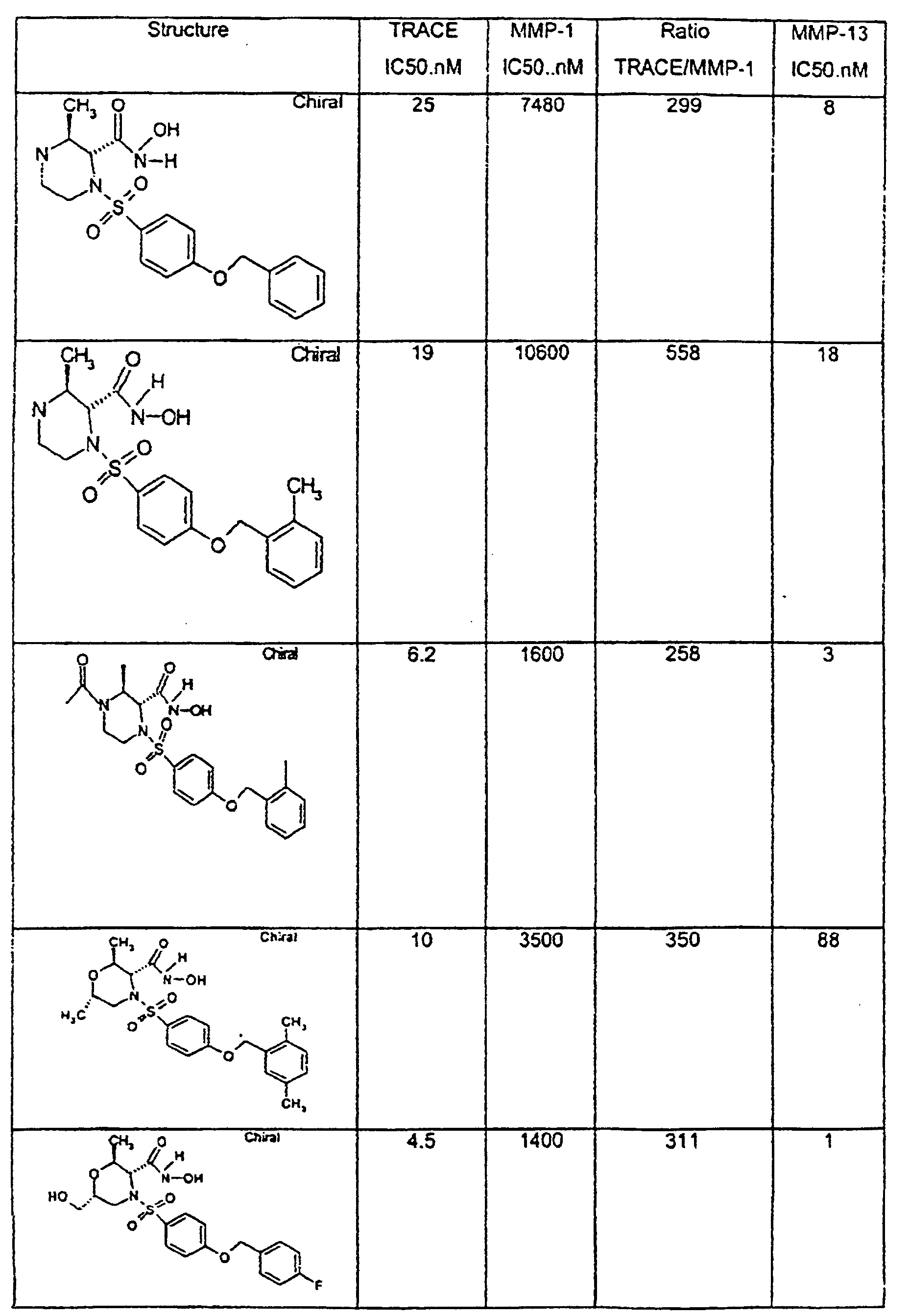

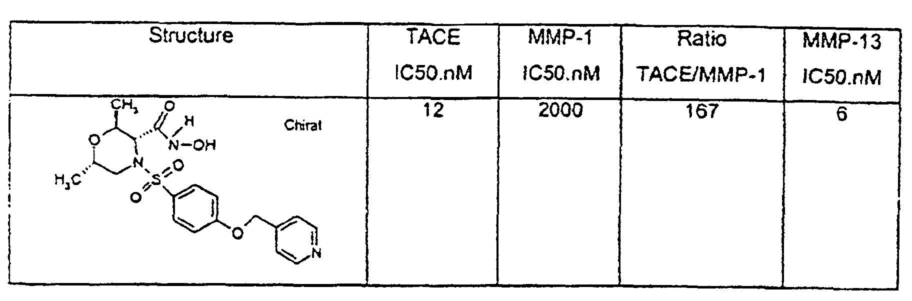

### Monocyte Assay

Human mononuclear cells were isolated from anti-coagulated human blood using a one-step Ficoll-hypaque separation technique. (2) The mononuclear cells were washed three times in Hanks balanced salt solution (HBSS) with divalent cations and resuspended to a density of 2 x 10⁶/ml in HBSS containing 1% BSA. Differential counts determined using the Abbott Cell Dyn 3500 analyzer indicated that monocytes ranged from 17 to 24% of the total cells in these preparations.

180m of the cell suspension was aliquoted into flat bottom 96 well plates (Costar). Additions of compounds and LPS (100 ng/ml final concentration) gave a final volume of 200 µl. All conditions were performed in triplicate. After a four hour incubation at 37°C in an humidified CO₂ incubator, plates were removed and centrifuged (10 minutes at approximately 250 x g) and the supernatants removed and assayed for TNF-α using the R&D ELISA Kit.

### MMP Assays

Collagenase-3 (matrix metalloproteinase-13) selective inhibitors as used herein refer to agents which exhibit at least a 100 fold selectivity for the inhibition of collagenase-3 enzyme activity over collagenase-1 enzyme activity and a potency of less than 100 nM as defined by the IC₅₀ results from the MMP-13/MMP-1 fluorescence assays described below. Collagenase-3 selective inhibitors can be identified by screening the inhibitors of the present invention through the MMP-13/MMP-1 fluorescence assays described below and selecting those agents with MMP-13/MMP-1 inhibition IC₅₀ ratios of 100 or greater and potency of less than 100 nM.

Non-selective collagenase inhibitors as used herein refer to agents which exhibit less than a 100 fold selectivity for the inhibition of collagenase-3 enzyme activity over collagenase-1 enzyme activity or a potency of more than 100nM as defined by the IC₅₀ results from the MMP-13/MMP-1 fluorescence assays described below.

The ability of collagenase inhibitors to inhibit collagenase activity is well known in the art. The following assays may be used to identify matrix metalloproteinase inhibitors.

### Inhibition of Human Collagenase (MMP-1)

Human recombinant collagenase is activated with trypsin using the following ratio: 10 µg trypsin per 100 µg of collagenase. The trypsin and collagenase are incubated at room temperature for 10 minutes then a five fold excess (50 µg/10 µg trypsin) of soybean trypsin inhibitor is added.

10 mM stock solutions of inhibitors are made up in dimethyl sulfoxide and then diluted using the following Scheme:

10 mM → 120 µM → 12 µM → 1.2 µM → 0.12 µM

Twenty-five microliters of each concentration is then added in triplicate to appropriate wells of a 96 well microfluor plate. The final concentration of inhibitor will be a 1:4 dilution after addition of enzyme and substrate. Positive controls (enzyme, no inhibitor) are set up in wells D1-D6 and blanks (no enzyme, no inhibitors) are set in wells D7-D12.

Collagenase is diluted to 400 ng/ml and 25 µl is then added to appropriate wells of the microfluor plate. Final concentration of collagenase in the assay is 100 ng/ml.

Substrate (DNP-Pro-Cha-Gly-Cys(Me)-His-Ala-Lys(NMA)-NH₂) is made as a 5 mM stock in dimethyl sulfoxide and then diluted to 20 mM in assay buffer. The assay is initiated by the addition of 50 µl substrate per well of the microfluor plate to give a final concentration of 10 µM.

Fluorescence readings (360 nM excitation, 460 nm emission) were taken at time 0 and then at 20 minute intervals. The assay is conducted at room temperature with a typical assay time of 3 hours.

Fluorescence vs time is then plotted for both the blank and collagenase containing samples (data from triplicate determinations is averaged). A time point that provides a good signal (the blank) and that is on a linear part of the curve (usually around 120 minutes) is chosen to determine IC₅₀ values. The zero time is used as a blank for each compound at each concentration and these values are subtracted from the 120 minute data. Data is plotted as inhibitor concentration vs % control (inhibitor fluorescence divided by fluorescence of collagenase alone x 100). IC₅₀'s are determined from the concentration of inhibitor that gives a signal that is 50% of the control.

If IC₅₀'s are reported to be <0.03 µM then the inhibitors are assayed at concentrations of 0.3 µM, 0.03 µM, 0.03 µM and 0.003 µM.

### Inhibition of Gelatinase (MMP-2)

Inhibition of gelatinase activity is assayed using the Dnp-Pro-Cha-Gly-Cys(Me)-His-Ala-Lys(NMA)-NH₂ substrate (10 µM) under the same conditions as inhibition of human collagenase (MMP-1).

72kD gelatinase is activated with 1 mM APMA (p-aminophenyl mercuric acetate) for 15 hours at 4°C and is diluted to give a final concentration in the assay of 100 mg/ml. Inhibitors are diluted as for inhibition of human collagenase (MMP-1) to give final concentrations in the assay of 30 µM, 3 µM, 0.3 µM and 0.03 µM. Each concentration is done in triplicate.

Fluorescence readings (360 nm excitation, 460 emission) are taken at time zero and then at 20 minutes intervals for 4 hours.

IC₅₀'s are determined as per inhibition of human collagenase (MMP-1). If IC₅₀'s are reported to be less than 0.03 µM, then the inhibitors are assayed at final concentrations of 0.3 µM. 0.03 µM, 0.003 µM and 0.003 µM.

### Inhibition of Stromelysin Activity (MMP-3)

Inhibition of stromelysin activity is based on a modified spectrophotometric assay described by Weingarten and Feder (Weingarten, H. and Feder, J., Spectrophotometric Assay for Vertebrate Collagenase, Anal. Biochem. 147, 437-440 (1985)). Hydrolysis of the thio peptolide substrate [Ac-Pro-Leu-Gly-SCH[CH₂CH(CH₃)₂]CO-Leu-Gly-OC₂H₅] yields a mercaptan fragment that can be monitored in the presence of Ellman's reagent.

Human recombinant prostromelysin is activated with trypsin using a ratio of 1 µl of a 10 mg/ml trypsin stock per 26 mg of stromelysin. The trypsin and stromelysin are incubated at 37°C for 15 minutes followed by 10 µl of 10 µg/ml soybean trypsin inhibitor for 10 minutes at 37°C for 10 minutes at 37°C to quench trypsin activity.

Assays are conducted in a total volume of 250 ml of assay buffer (200 mM sodium chloride, 50 mM MES, and 10 mM calcium chloride, pH 6.0) in 96-well microliter plates. Activated stromelysin is diluted in assay buffer to 25 µg/ml. Ellman's reagent (3-Carboxy-4-nitrophenyl disulfide) is made as a 1M stock in dimethyl formamide and diluted to 5 mM in assay buffer with 50 ml per well yielding at 1 mM final concentration.

10 mM stock solutions of inhibitors are made in dimethyl sulfoxide and diluted serially in assay buffer such that addition of 50 µL to the appropriate wells yields final concentrations of 3 µM, 0.3 µM, 0.003 µM, and 0.0003 µM. All conditions are completed in triplicate.

A 300 mM dimethyl sulfoxide stock solution of the peptide substrate is diluted to 15 mM in assay buffer and the assay is initiated by addition of 50 µl to each well to give a final concentration of 3 mM substrate. Blanks consist of the peptide substrate and Ellman's reagent without the enzyme. Product formation was monitored at 405 nm with a Molecular Devices UVmax plate reader.

IC₅₀ values were determined in the same manner as for collagenase.

### Inhibition of MMP-13

Human recombinant MMP-13 is activated with 2 mM APMA (p-aminophenyl mercuric acetate) for 1.5 hours, at 37°C and is diluted to 400 mg/ml in assay buffer (50 mM Tris, pH 7.5, 200 mM sodium chloride, 5 mM calcium chloride, 20 µM zinc chloride, 0.02% brij). Twenty-five microliters of diluted enzyme is added per well of a 96 well microfluor plate. The enzyme is then diluted in a 1:4 ratio in the assay by the addition of inhibitor and substrate to give a final concentration in the assay of 100 mg/ml.

10 mM stock solutions of inhibitors are made up in dimethyl sulfoxide and then diluted in assay buffer as per the inhibitor dilution scheme for inhibition of human collagenase (MMP-1): Twenty-five microliters of each concentration is added in triplicate to the microfluor plate. The final concentrations in the assay are 30 µM, 3 µM, 0.3 µM, and 0.03 µM.

Substrate (Dnp-Pro-Cha-Gly-Cys(Me)-His-Ala-Lys(NMA)-NH₂) is prepared as for inhibition of human collagenase (MMP-1) and 50 ml is added to each well to give a final assay concentration of 10 µM. Fluorescence readings (360 nM excitation; 450 emission) are taken at time 0 and every 5 minutes for 1 hour.

Positive controls consist of enzyme and substrate with no inhibitor and blanks consist of substrate only.

IC₅₀'s are determined as per inhibition of human collagenase (MMP-1). If IC₅₀'s are reported to be less than 0.03 µM, inhibitors are then assayed at final concentrations of 0.3 µM, 0.03 µM, 0.003 µM and 0.0003 µM.

### Collagen film MMP-13 Assay

Rat type I collagen is radiolabeled with ¹⁴C acetic anhydride (T.E. Cawston and A.J. Barrett, Anal. Biochem., 99, 340-345 (1979)) and used to prepare 96 well plates containing radiolabeled collagen films (Barbara Johnson-Wint, Anal. Biochem., 104, 175-181 (1980)). When a solution containing collagenase is added to the well, the enzyme cleaves the insoluble collagen which unwinds and is thus solubilized. Collagenase activity is directly proportional to the amount of collagen solubilized, determined by the proportion of radioactivity released into the supernatant as measured in a standard scintillation counter. Collagenase inhibitors are, therefore, compounds which reduce the radioactive counts released with respect to the controls with no inhibitor present. One specific embodiment of this assay is described in detail below.

For determining the selectivity of compounds for MMP-13 versus MMP-1 using collagen as a substrate, the following procedure is used. Recombinant human proMMP-13 or proMMP-1 is activated according to the procedures outlined above. The activated MMP-13 or MMP-1 is diluted to 0.6 ug/ml with buffer ( 50 mM Tris pH 7.5, 150 mM NaCl, 10 mM CaCl₂ , 1 uM ZnCl₂, 0.05% Brij-35, 0.02% sodium azide).

Stock solutions of test compound (10mM) in dimethylsulfoxide are prepared. Dilutions of the test compounds in the Tris buffer, above, are made to 0.2, 2.0, 20, 200, 2000 and 20000 nM.

100 µl of appropriate drug dilution and 100 µl of diluted enzyme are pipetted into wells of a 96 well plate containing collagen films labeled with ¹⁴C-collagen. The final enzyme concentration is 0.3 µg/ml while the final drug concentration is 0.1, 1.0, 10, 100, 1000 nM. Each drug concentration and control is analyzed in triplicate. Triplicate controls are also run for the conditions in which no enzyme is present and for enzyme in the absence of any compound.

The plates are incubated at 37°C for a time period such that around 30 - 50% of the available collagen is solubilized - determined by counting additional control wells at various time points. In most cases around 9 hours of incubation are required. When the assay has progressed sufficiently, the supernatant from each well is removed and counted in a scintillation counter. The background counts (determined by the counts in the wells with no enzyme) are subtracted from each sample and the % release calculated in relation to the wells with enzyme only and no inhibitor. The triplicate values for each point are averaged and the data graphed as percent release versus drug concentration. IC₅₀'s are determined from the point at which 50% inhibition of release of radiolabeled collagen is obtained.

To determine the identity of the active collagenases in cartilage conditioned medium, assays were carried out using collagen as a substrate, cartilage conditioned medium containing collagenase activity and inhibitors of varying selectivity. The cartilage conditioned medium was collected during the time at which collagen degradation was occurring and thus is representative of the collagenases responsible for the collagen breakdown. Assays were carried out as outlined above except that instead of using recombinant MMP-13 or recombinant MMP-1, cartilage conditioned medium was the enzyme source.

### IL-1 Induced Cartilage Collagen Degradation From Bovine Nasal Cartilage

This assay uses bovine nasal cartilage explants which are commonly used to test the efficacy of various compounds to inhibit either IL-1 induced proteoglycan degradation or IL-1 induced collagen degradation. Bovine nasal cartilage is a tissue that is very similar to articular cartilage, i.e. chondrocytes surrounded by a matrix that is primarily type II collagen and aggrecan. The tissue is used because it: (1) is very similar to articular cartilage, (2) is readily available, (3) is relatively homogeneous, and (4) degrades with predictable kinetics after IL-1 stimulation.

Two variations of this assay have been used to assay compounds. Both variations give similar data. The two variations are described below:

### Variation 1

Three plugs of bovine nasal cartilage (approximately 2 mm diameter x 1.5 mm long) are placed into each well of a 24 well tissue culture plate. One ml of serumless medium is then added to each well. Compounds are prepared as 10 mM stock solutions in DMSO and then diluted appropriately in serumless medium to final concentrations, e.g., 50, 500 and 5000 nM. Each concentration is assayed in triplicate.

Human recombinant IL-1α (5ng/mL) (IL-1) is added to triplicate control wells and to each well containing drug. Triplicate control wells are also set up in which neither drug nor IL-1 are added. The medium is removed and fresh medium containing IL-1 and the appropriate drug concentrations is added on days 6, 12, 18 and 24 or every 3 - 4 days if necessary. The media removed at each time point is stored at -20°C for later analysis. When the cartilage in the IL-1 alone wells has almost completely resorbed (about day 21), the experiment is terminated. The medium, is removed and stored. Aliquots (100 ul) from each well at each time point are pooled, digested with papain and then analyzed for hydroxyproline content. Background hydroxyproline (average of wells with no IL-1 and no drug) is subtracted from each data point and the average calculated for each triplicate. The data is then expressed as a percent of the IL-1 alone average value and plotted. The IC₅₀ is determined from this plot.

### Variation 2

The experimental set-up is the same as outlined above in Variation 1, until day 12. On day 12, the conditioned medium from each well is removed and frozen. Then one ml of phosphate buffered saline (PBS) containing 0.5 µg/ml trypsin is added to each well and incubation continued for a further 48 hours at 37°C. After 48 hours incubation in trypsin, the PBS solution is removed. Aliquots (50 µl) of the PBS/trypsin solution and the previous two time points (days 6 and 12) are pooled, hydrolyzed and hydroxyproline content determined. Background hydroxyproline (average of wells with no IL-1 and no drug) is subtracted from each data point and the average calculated for each triplicate. The data is then expressed as a percent of the IL-1 alone average value and plotted. The IC₅₀ is determined from this plot. In this variation, the time course of the experiment is shortened considerably. The addition of trypsin for 48 hours after 12 days of IL-1 stimulation likely releases any type II collagen that has been damaged by collagenase activity but not yet released from the cartilage matrix. In the absence of IL-1 stimulation, trypsin treatment produces only low background levels of collagen degradation in the cartilage explants.

### Inhibition of Human 92 kD Gelatinase (MMP-9)

Inhibition of 92 kD gelatinase (MMP-9) activity is assayed using the Mca-Pro-Leu-Gly-Leu-Dpa-Ala-Arg-NH₂ substrate (10 µM) under similar conditions as described above for the inhibition of human collagenase (MMP-1).

Human recombinant 92 kD gelatinase (MMP-9, gelatinase B) is activated for 2 hours with 1mM p-aminophenyl-mercuric acetate (from a freshly prepared 100 mM stock in 0.2 N NaOH) at 37 C.

10 mM dimethylsulfoxide stock solutions of inhibitors are diluted serially in assay buffer (50 mM TRIS, pH 7.5, 200 mM NaCl, 5 mM CaCl₂, 20 µM ZnCl₂, 0.02% BRIJ-35 (vol./vol.)) using the following scheme:

10 mM→ 120 µM→ 12 µM→ 1.2 µM→ 0.12 µM

Further dilutions are made as necessary following this same scheme. A minimum of four inhibitor concentrations for each compound are performed in each assay. 25 µL of each concentration is then added to triplicate wells of a black 96 well U-bottomed microfluor plate. As the final assay volume is 100 µL, final concentrations of inhibitor are the result of a further 1:4 dilution (i.e. 30 µM → 3 µM → 0.3 µM → 0.03 µM, etc.). A blank (no enzyme, no inhibitor) and a positive enzyme control (with enzyme, no inhibitor) are also prepared in triplicate.

Activated enzyme is diluted to 100 ng/mL in assay buffer, 25 µL per well is added to appropriate wells of the microplate. Final enzyme concentration in the assay is 25 ng/mL (0.27 nM).

A five mM dimethylsulfoxide stock solution of substrate (Mca-Pro-Leu-Gly-Leu-Dpa-Ala-Arg-NH₂) is diluted in assay buffer to 20 µM. The assay is initiated by addition of 50 µL of diluted substrate yielding a final assay concentration of 10 µM substrate. A 0 time fluorescence reading (320 excitation; 390 emission) is immediately taken and subsequent readings are taken every fifteen minutes at room temperature with a PerSeptive Biosystems CytoFluor Multi-Well Plate Reader with the gain at 90 units.

The average value of fluorescence of the enzyme and blank are plotted versus time. An early time point on the linear part of this curve is chosen for IC₅₀ determinations. The 0 time point for each compound at each dilution is subtracted from the latter time point and the data then expressed as percent of enzyme control (inhibitor fluorescence divided by fluorescence of positive enzyme control x 100). Data is plotted as inhibitor concentration versus percent of enzyme control. IC₅₀'s are defined as the concentration of inhibitor that gives a signal that is 50% of the positive enzyme control.

### Aggrecanase Assay

Primary porcine chondrocytes from articular joint cartilage are isolated by sequential trypsin and collagenase digestion followed by collagenase digestion overnight and are plated at 2 X 10⁵ cells per well into 48 well plates with 5 µCi / ml ³⁵S (1000 Ci/mmol) sulphur in type I collagen coated plates. Cells are allowed to incorporate label into their proteoglycan matrix (approximately 1 week) at 37°C, under an atmosphere of 5% CO₂.

The night before initiating the assay, chondrocyte monolayers are washed two times in DMEM/ 1% PSF/G and then allowed to incubate in fresh DMEM /1% FBS overnight.

The following morning chondrocytes are washed once in DMEM/1%PSF/G. The final wash is allowed to sit on the plates in the incubator while making dilutions.

Media and dilutions can be made as described in the Table below.

| | |
|---|---|
| Control Media | DMEM alone (control media) |
| IL-1 Media | DMEM + IL-1 (5 ng/ml) |
| Drug Dilutions | Make all compounds stocks at 10 mM in DMSO. Make a 100 uM stock of each compound in DMEM in 96 well plate. Store in freezer overnight. The next day perform serial dilutions in DMEM with IL-1 to 5 uM, 500 nM, and 50 nM. Aspirate final wash from wells and add 50 ul of compound from above dilutions to 450 ul of IL-1 media in appropriate wells of the 48 well plates. Final compound concentrations equal 500 nM, 50 nM, and 5 nM. All samples completed in triplicate with Control and IL-1 alone samples on each plate. |

| | |
|---|---|
| + | |

Plates are labeled and only the interior 24 wells of the plate are used. On one of the plates, several columns are designated as IL-1 (no drug) and Control (no IL-1, no drug). These control columns are periodically counted to monitor 35S-proteoglycan release. Control and IL-1 media are added to wells (450 ul) followed by compound (50 ul) so as to initiate the assay. Plates are incubated at 37°C, with a 5% CO₂ atmosphere.

At 40-50 % release (when CPM from IL-1 media is 4-5 times control media) as assessed by liquid scintillation counting (LSC) of media samples, the assay is terminated (9-12 hours). Media is removed from all wells and placed in scintillation tubes. Scintillate is added and radioactive counts are acquired (LSC). To solubilize cell layers, 500 ul of papain digestion buffer (0.2 M Tris, pH 7.0, 5 mM EDTA, 5 mM DTT, and 1 mg/ml papain) is added to each well. Plates with digestion solution are incubated at 60°C overnight. The cell layer is removed from the plates the next day and placed in scintillation tubes. Scintillate is then added, and samples counted (LSC).

The percent of released counts from the total present in each well is determined. Averages of the triplicates are made with control background subtracted from each well. The percent of compound inhibition is based on IL-1 samples as 0% inhibition (100% of total counts).

The compounds of the present invention that were tested had IC₅₀ of less than 1 µM, preferably less than 50nM in at least one of the assays described above. The compounds of the present invention also possess differential activity (i.e. are selective for) for one or more reprolysin or MMP. Selectivity as used herein refers to the ratio of the IC₅₀ inhibitory results from two or more of the above protocols. Compounds of the invention which are selective possess a ratio of at least 10. The compounds of the invention possessing the potency or selectivity desired can be identified by assaying a compound compound of formula I according to the protocols described above and determining the IC₅₀ and selectivity ratios.

One group of preferred compounds of the formula I that can be identified by the methods of the present invention include those that possess selective activity against TACE over MMP-1, preferably at least 40 fold, more preferably at least 100 fold, more selective for TACE over MMP-1, more preferably with a TACE IC₅₀ of less than 50nM, most preferably less than 10nM. Another group of preferred compounds of the formula I that can be identified by the methods of the present invention include those inhibitors that possess potent activity for TACE and MMP-13 (preferably an IC₅₀ of less than 100nM, more preferably 50nM, most preferably 10nM), preferably wherein said MMP-13 and TACE inhibitory activity is selective activity for MMP-13 and TACE preferentially over MMP-1, preferably such compounds are equipotent for inhibition for TACE and MMP-13 (IC₅₀) as described in at least one of the TACE or MMP-13 assays described above, more preferably wherein said compounds possess at least 100 fold selectivity for TACE and MMP-13 over MMP-1. Another group of preferred compounds of the formula I that can be identified by the methods of the present invention include those inhibitors that possess potent activity (preferably an IC₅₀ of less than 500nM, more preferably 100nM, most preferably 50nM) for TACE and Aggrecanase, preferably wherein said TACE and Aggrecanase inhibitory activity is selective activity for TACE and Aggrecanase preferentially over MMP-1, more preferably wherein said selectivity is 10 fold, most preferably 40 fold, higher for TACE and Aggrecanase over MMP-1.

Another group of preferred compounds the formula I that can be identified by the methods of the present invention include those inhibitors that possess selective activity against MMP-13 over MMP-1, (preferably an IC₅₀ of less than 500nM, more preferably 100nM, most preferably 50nM) for MMP-13 with a selectivity of at least 10 fold, preferably 40 fold, higher for MMP-13 over MMP-1.

Another group of preferred compounds of the formula I that can be identified by the methods of the present invention include those inhibitors that possess potent inhibitory activity (preferably an IC₅₀ of less than 500nM, more preferably 100nM, most preferably 50nM) for MMP-13 and Aggrecanase, preferably wherein said MMP-13 and Aggrecanase inhibitory activity is selective activity for MMP-13 and Aggrecanase over MMP-1, preferably wherein said selectivity is at least 10 fold, preferably 40 fold, higher for MMP-13 and Aggrecanase over MMP-1.

Another group of preferred compounds of the formula I that can be identified by the methods of the present invention include those that possess potent activity for Aggrecanase (preferably an IC₅₀ of less than 500nM, more preferably 100nM, most preferably 50nM), preferably wherein said Aggrecanase inhibitory activity is selective activity for Aggrecanase over MMP-1, preferably 10 fold, more preferably 40 fold, more selective for Aggrecanase over MMP-1.

Another group of preferred compounds of the formula I that can be identified by the methods of the present invention include those inhibitors that possess potent activity against Aggrecanase, MMP-13 and TACE (preferably an IC₅₀ of less than 500nM, more preferably 100nM, most preferably 50nM) preferably wherein said Aggrecanase, MMP-13 and TACE inhibitory activity is selective activity for Aggrecanase, MMP-13 and TACE over MMP-1, preferably 10 fold more selective for Aggrecanase, MMP-13 and TACE over MMP-1.

For administration to mammals, including humans, for the inhibition of matrix metalloproteinases or mammalian reprolysin (preferably inhibition of TACE), a variety of conventional routes may be used including orally, parenterally and topically. In general, the active compound will be administered orally or parenterally at dosages between about 0.1 and 25 mg/kg body weight of the subject to be treated per day, preferably from about 0.3 to 5 mg/kg. However, some variation in dosage will necessarily occur depending on the condition of the subject being treated. The person responsible for administration will, in any event, determine the appropriate dose for the individual subject.

The compounds of the present invention can be administered in a wide variety of different dosage forms, in general, the therapeutically effective compounds of this invention are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelation and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration (intramuscular, intraperitoneal, subcutaneous and intravenous use) a sterile injectable solution of the active ingredient is usually prepared. Solutions of a therapeutic compound of the present invention in either sesame or peanut oil or in aqueous propylene glycol may be employed. The aqueous solutions should be suitably adjusted and buffered, preferably at a pH of greater than 8, if necessary and the liquid diluent first rendered isotonic. These aqueous solutions are suitable intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

The following Examples illustrate the preparation of the compounds of the present invention. Melting points are uncorrected. NMR data are reported in parts per million (δ) and are referenced to the deuterium lock signal from the sample solvent (deuteriodimethylsulfoxide unless otherwise specified). Commercial reagents were utilized without further purification. THF refers to tetrahydrofuran. DMF refers to N,N-dimethylformamide. Chromatography refers to column chromatography performed using 32-63 mm silica gel and executed under nitrogen pressure (flash chromatography) conditions. Room or ambient temperature refers to 20-25°C. All non-aqueous reactions were run under a nitrogen atmosphere for convenience and to maximize yields. Concentration at reduced pressure means that a rotary evaporator was used.

### EXAMPLE 1

### (2S,3S)-4-[4-(3,5-DIFLURO-BENZYLOXY)-BENZENESULFONYL]-2-METHYL-THIOMORPHOLINE-3-CARBOXYLIC ACID HYDROXYAMIDE

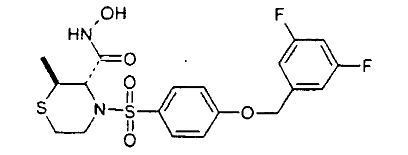

(a) Hydrochloric acid gas was bubbled through a stirred, cool (20 °C) suspension of D-threonine (25 g, 21 mmol) in 150 mL of methanol for 15 minutes. The resulting mixture was heated at reflux for 24 hours while under a nitrogen atmosphere. The mixture was brought to ambient temperature (23 °C) and concentrated to a clear oil to provide (2*R*, 3*S*)-2-amino-3-hydroxy-butyric acid methyl ester hydrochloride, which was used in the subsequent step without purification.
(b) To a stirred, cold (0°C) solution of (2*R*, 3*S*)-2-amino-3-hydroxy-butyric acid methyl ester hydrochloride and diisopropylethylamine (16.5 mL, 94.3 mmol) in 95 mL of methylene chloride was added 4-(3,5-difluro-benzyloxy)-benzenesulfonyl chloride (15 g, 47.2 mmol) in 5 mL of methlyene chloride under a nitrogen atmosphere. The resulting mixture was stirred for 7.5 hours at 0 °C, and then placed in a refrigerator (4 °C) for an additional 17 hours. The mixture was added to water and extracted with ethyl acetate. The combined ethyl acetate extracts were washed with aqueous sodium carbonate, dilute aqueous hydrochloric acid, water, brine, and dried over sodium sulfate. After filtration and concentration of the filtrate, the crude solid was suspended in diethyl ether, and stirred for 16 hours. Filtration and drying provided (2*R*, 3*S*)-2-[4-(3,5-difluro-benzyloxy)-benzenesulfonylamino]-3-hydroxy-butyric acid methyl ester as a pale pink solid.
(c) To a stirred, cold (0 °C) solution of triphenylphosphine (2.1g, 7.9 mmol) in 18 mL of tetrahydrofuran was added 1.2 mL (7.6 mmol) of dimethyl azodicarboxylate dropwise. After 20 minutes a solution of (2*R*, 3*S*)-2-[4-(3,5-difluro-benzyloxy)-benzenesulfonylamino]-3-hydroxy-butyric acid methyl ester (3 g, 7.2 mmol) in 18 mL of tetrahydrofuran was added dropwise. The resulting mixture was stirred for 48 hours at ambient temperature (23 °C) under a nitrogen atmosphere. The reaction mixture was concentrated and purified by silica gel chromatography (elution with 65:35 hexanes/ethyl acetate) to provide (2*R*, 3*R*)-1-[4-(3,5-difluro-benzyloxy)-benzenesulfonyl]-3-methyl-aziridine-2-carboxylic acid methyl ester as a pale pink oil.
(d) To a stirred solution of (2*R*, 3*R*)-1-[4-(3,5-difluro-benzyloxy)-benzenesulfonyl]-3-methyl-aziridine-2-carboxylic acid methyl ester (421 mg, 1.1 mmol) and 2-mercaptoethanol (3.7 mL, 52.9 mmol) in 2.6 mL of methylene chloride was added boron trifluoride etherate (catalytic) at ambient temperature (23 °C) under a nitrogen atmosphere. After 42.5 hours the reaction material was added to phosphate buffer (pH of 7) and the mixture was extracted with ethyl acetate. The combined organic extracts were dried over sodium sulfate. After filtration and concentration of the filtrate, the resulting crude product was purified by silica gel chromatography (elution with 5:3 ethyl acetate/hexanes) to provide (2*S*, 3*S*)-2-[4-(3,5-difluro-benzyloxy)-benzenesulfonylamino]-3-(2-hydroxy-ethylsulfonyl)-butyric acid methyl ester as a white solid.
(e) To a stirred solution of triphenylphosphine (116 mg, 0.44 mmol) in 1.0 mL of tetrahydrofuran was added dimethyl azodicarboxylate (56 µL, 420 µmol) dropwise. After 30 minutes a solution of (2*S*, 3*S*)-2-[4-(3,5-difluro-benzyloxy)-benzenesulfonylamino]-3-(2-hydroxy-ethylsulfonyl)-butyric acid methyl ester (192 mg, 0.40 mmol) in 1.0 mL of tetrahydrofuran was added dropwise. The resulting mixture was stirred for 48 hours at ambient temperature (23 °C) under a nitrogen atmosphere. The reaction material was concentrated and purified by silica gel chromatography (elution with 75:25 hexanes/ethyl acetate) to provide (2*S*, 3*S*)-4-[4-(3,5-difluro-benzyloxy)-benzenesulfonyl]-2-methyl-thiomorpholine-3-carboxylic acid methyl ester as a white foam.
(f) A stirred solution of (2*S*, 3*S*)-4-[4-(3,5-difluro-benzyloxy)-benzenesulfonyl]-2-methyl-thiomorpholine-3-carboxylic acid methyl ester (113 mg, 0.25 mmol), lithium hydroxide monohydrate (41 mg, 0.99 mmol), 1,4-dioxane (6 mL), methanol (3 mL) and water (7 mL) was heated to 60 °C under a nitrogen atmosphere for 5 hours. The reaction mixture was added to dilute aqueous hydrochloric acid. The aqueous layer was separated and further acidified to a pH of 2.5 using 1 N aqueous hydrogen chloride, and extracted with ethyl acetate. The combined organic extracts were dried over sodium sulfate, filtered and the filtrate was concentrated to afford (2*S*, 3*S*)-4-[4-(3,5-difluro-benzyloxy)-benzenesulfonyl]-2-methyl-thiomorpholine-3-carboxylic acid, which was used in the subsequent step without purification.
(g) To a stirred suspension of (2*S*, 3*S*)-4-[4-(3,5-difluro-benzyloxy)-benzenesulfonyl]-2-methyl-thiomorpholine-3-carboxylic acid (96 mg, 0.22 mmol) and dimethylformamide (0.3 µL, 4 µmol) in 900 µL of methylene chloride, at 0 °C, was added oxalylchloride (21 µL, 240 µmol) dropwise while stirring under a nitrogen atmosphere. Meanwhile, chlorotrimethylsilane (83 µL, 650 µmol) was added dropwise, to a stirred cold (0 °C) solution of hydroxylamine hydrochloride (19.6 mg, 0.28mmol) in 130 µL of pyridine. Both reaction mixtures were warmed to ambient temperature (23 °C). After 20 hours both reaction mixtures were cooled to 0 °C and the solution of the acid chloride was added to the stirred suspension of the *bis*-(trimethylsilyl)hyroxylamine via cannula. The resulting mixture was stirred for 2 hours at 0 °C and for 2 hours at 23 °C before 10 µL of 1 N aqueous hydrogen chloride was added and the reaction stirred for an additional 4 hours. The reaction mixture was added to water and extracted with ethyl acetate. The combined organic extracts were washed with water, dried with sodium sulfate, filtered, and the filtrate was concentrated. The resulting crude material was purified by silica gel chromatography (elution with 1:1 ethyl acetate/hexanes) to afford (2*S*, 3*S*)-4-[4-(3,5-difluro-benzyloxy)-benzenesulfonyl]-2-methyl-thiomorpholine-3-carboxylic acid hydroxyamide as a pale yellow solid. Mass spectrum: *m*/*z* 459 (M+H).

### EXAMPLE 2

### (2S,3S)-4-[4-(4-FLUORO-BENZYLOXY)-BENZENSULFONYL]-2-METHYL-THIOMORPHOLINE-3-CARBOXYLIC ACID HYDROXYAMIDE

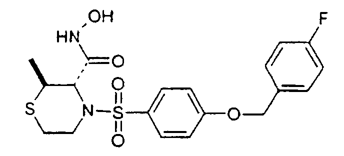

(2S, 3S)-4-[4-(4-Fluoro-benzyloxy)-benzensulfonyl]-2-methyl-thiomorpholine-3-carboxylic acid hydroxyamide was prepared in a similar manner starting from (2R, 3S)-2-[4-(4-fluoro-benzyloxy)-benzensulfonyl]-3-hydroxy-butyric acid methyl ester with the exception that diisopropyl azodicarboxylate was used in steps c and e. Mass spectrum: *m*/*z* 441 (M+H).

### EXAMPLE 3

### (2S,3R,6S)-2,6-DIMETHYL-4-[4-(2-METHYL-BENZYLOXY)-BENZENESULFONYL]-MORPHOLINE-3-CARBOXYLIC ACID HYDROXYAMIDE

### Step 1: Preparation of the acyclic sulfonamide.

### (2R, 3S)-2-[4-(4-Fluoro-benzyloxy)-benzenesulfonylamino]-3-hydroxy-butyric acid methyl ester

Thionylchloride (26 mL) was added dropwise to room temperature methanol. The solution was cooled to 0° C and D-Threonine (14.0 g, 118 mmol) was added in three portions over 30 minutes. After stirring for 1 hour at 0° C, the reaction was stirred at room temperature overnight. The solvent was removed at high vacuum over 48 hours. The resulting glassy white solid was dissolved in ethyl acetate (170 mL) and water (80 mL) at 0°C. Potassium carbonate (22.8 g, 165 mmol) and 4-(4-fluoro-benzyloxy)-benzenesulfonyl chloride (46.9 g, 118 mmol) were added sequentially. The resulting mixture was stirred at 0 °C for 30 minutes then at room temperature for 20 hours. Ethyl acetate (200 mL) was added. The organic phase was separated, extracted with water (3 x 20 mL) and brine ( 20 mL) and dried over magnesium sulfate. The solvent was removed *in vacuo* to give the title compound (42.8 g, 91.3 %).

### Step 2: Allylation of the acyclic sulfonamide.

### (2R, 3S)-2-{Allyl-[4-(4-fluoro-benzyloxy)-benzenesulfonyl]-amino}-3-hydroxy-butyric acid methyl ester

(2*R*, 3*S*)-2-[4-(4-Fluoro-benzyloxy)-benzenesulfonylamino]-3-hydroxy-butyric acid methyl ester (20 g, 50.3 mmol) was dissolved in dimethyl formamide (100 mL). Allyl iodide (16.8 g, 100 mmol) and cesium carbonate (17 g, 52.3 mmol) were added sequentially. The solution was stirred for 2.5 hours at room temperature. Diethyl ether (800 mL) was added and a precipitate formed. The liquid phase was removed, extracted with water (5 x 20 mL) and brine (2 x 20 mL), and dried over magnesium sulfate. The solvent was removed *in vacuo.* The resulting oil was chromatographed on silica gel (40 µM) with ethyl acetate in hexanes to give the title compound (17 g, 77 %).

### Step 3: Cyclization.

### (2S,3R,6S)-4-[4-(4-Fluoro-benzyloxy)-phenylsulfanyl]-2,6-dimethyl-morpholine-3-carboxylic acid methyl ester

(2*R*, 3*S*)-2-{Allyl-[4-(4-fluoro-benzyloxy)-benzenesulfonyl]-amino}-3-hydroxy-butyric acid methyl ester (12.5 g, 28.6 mmol) was dissolved in tetrahydrofuran (286 mL). Potassium carbonate (4.7 g, 34.3 mmol) and mercury(II) trifluoroacetate (14.6 g, 34.3 mmol) were added sequentially at room temperature. The mixture was stirred for 75 minutes at room temperature, then cooled on a dry ice diethyl ether bath. Triethyl borane (1 M, 30 mL, 30 mmoL) in hexanes was added. After 5 minutes, sodium borohydride (1.1 g, 33.4 mmol) was added. The reaction was stirred on the cooling bath for 1 hour, then warmed to room temperature. Water (100 mL) was added and the mixture was extracted with ethyl acetate (4 x 200 mL). The organic portions were combined, extracted with brine (25 mL), and dried over magnesium sulfate. The solvent was removed *in vacuo* to afford the title compound (12.0 g, 96%).

### Step 4: Debenzylation.

### (2S,3R,6S)-4-(4-Hydroxy-phenylsulfanyl)-2,6-dimethyl-morpholine-3-carboxylic acid methyl ester

(2*S*,3*R*,6*S*)-4-[4-(4-Fluoro-benzyloxy)-phenylsulfanyl]-2,6-dimethyl-morpholine-3-carboxylic acid methyl ester (2.0 g, 27.4 mmol) was dissolved in methanol (75 mL) and palladium on carbon (1.5 g) was addeded. This mixture was shaken under hydrogen (35 psi) overnight. The catalyst was removed by filtration and the solvent removed *in vacuo* to provide the title compound (9.0 g, 99%).

### Step 5: Benzylation.

### (2S,3R,6S)-2,6-Dimethyl-4-[4-(2-methyl-benzyloxy)-phenylsulfanyl]-morpholine-3-carboxylic acid methyl ester

(2*S*,3*R*,6*S*)-4-(4-Hydroxy-phenylsulfanyl)-2,6-dimethyl-morpholine-3-carboxylic acid methyl ester (0.66 g, 2.0 mmol) was dissolved in dimethyl formamide (4 mL). 2-Methyl benzylbromide (0..407 g, 2.2 mmol) and cesium carbonate (1.3 g, 4 mmol) were added to the reaction mixture. The reaction mixture was stirred at room temperature for 5 hours and heated at 40 C for 1 hour. Diethyl ether (75 mL) and ethyl acetate (25 mL) were added and a precipitate formed. The liquid phase was removed, extracted with aqueous hydrochloric acid (1 N, 4 x 10 mL) and brine (1 x 10 mL), and dried over magnesium sulfate. The solvent was removed *in vacuo.* The resulting oil was chromatographed on silica gel with ethyl acetate in hexanes to give the title compound (0.42 g, 48 %).

### Step 6: Hydroxamate formation.

### (2S,3R,6S)-2,6-Dimethyl-4-[4-(2-methyl-benzyloxy)-phenylsulfanyl]-morpholine-3-carboxylic acid allyloxy-amide

(2*S*,3*R*,6*S*)-2,6-Dimethyl-4-[4-(2-methyl-benzyloxy)-phenylsulfanyl]-morpholine-3-carboxylic acid methyl ester (0.42 g, 0.97 mmol) was combined with lithium hydroxide monohydrate (420 mg, 10 mmol) in water (5 mL), tetrahydrofuran (10 mL) and methanol (5 mL). The solution was stirred at room temperature for 3 hours. Ethyl acetate (100 mL), aqueous hydrochloric acid (1 N, 25 mL) and excess sodium chloride were added. The aqueous phase was extracted with ethyl acetate (3 x 25 mL). The combined organic portions were extracted with brine (1 x 10 mL), dried over magnesium sulfate, and the solvent removed *in vacuo*. The crude acid was combined with allyl hydroxyl amine hydrochloride (0.44 g, 4 mmol) and hydroxybenzotriazole (0.03 g, 0.2 mmol) in dichloromethane (10 mL). Diisopropyl ethyl amine (1.23 mL, 7 mmol) was added. 1-(3-Dimethylaminopropyl)-3-ethylcarbodiimide (0.575 g, 3.0 mmol) was added and the reaction stirred at room temperature overnight. Ethyl acetate (100 mL) was added and the solution extracted with aqueous hydrochloric acid (1 N, 4 X 10 mL) and brine (10 mL). The organic portion was dried over magnesium sulfate and the solvent was removed *in vacuo*. The crude material was chromatographed on silica gel with ethyl acetate in hexanes to give the title compound (0.23 g, 50 %).

### Step 7: Hydroxamic acid deprotection.

### (2S,3R,6S)-2,6-Dimethyl-4-[4-(2-methyl-benzyloxy)-benzenesulfonyl]-morpholine-3-carboxylic acid hydroxyamide

(2*S*,3*R*,6*S*)-2,6-Dimethyl-4-[4-(2-methyl-benzyloxy)-phenylsulfanyl]-morpholine-3-carboxylic acid allyloxy-amide (0.23 g, 0.48 mmol) was dissolved in tetrahydrofuran (10 mL). Allyl palladium chloride dimer (0.012 g, 0.033 mmol), *bis*1,2-(diphenylphosphino)ethane (0.041 g, 0.1 mmol) and piperidine (50 mL, 0.5 mmol) were added sequentially at room temperature. The color changed from pale to bright yellow upon addition of the amine. The reaction was stirred for 2 hours, during which time it slowly turned orange. The tetrahydrofuran was remove *in vacuo.* The solids were dissolved in aqueous hydrochloric acid (1 N, 10 mL) and ethyl acetate (100 mL). The mixture was stirred open to air for 30 minutes and the phases separated. The organic portion was extracted with aqueous hydrochloric acid (1 N, 2 x 10 mL) and brine (10 mL), then dried over magnesium sulfate. The solvent was removed *in vacuo* and the crude material chromatographed with a mixture of ethyl acetate, hexane, and acetic acid. The title compound (0.108 g, 50 %) was isolated as a solid when dissolved in diethylether and the solvent removed by rotary evaporation.

### EXAMPLE 4

### 4-(4-BENZYLOXY-BENZENESULFONYL)-2-METHYL-MORPHOLINE-3-CARBOXYLIC ACID HYDROXYAMIDE

### 2-(4-Benzyloxy-benzenesulfonylamino)-3-hydroxy-butyric acid methyl ester

The title compound was prepared in a manner similar to (2*R*, 3*S*)-2-[4-(4-Fluoro-benzyloxy)-benzenesulfonylamino]-3-hydroxy-butyric acid methyl ester in Step 1 of Example 3, except that 4-benzyloxy-benzenesulfonyl chloride was substituted for 4-(4-fluoro-benzyloxy)-benzenesulfonyl chloride.

### 1-(4-Benzyloxy-benzenesulfonyl)-3-methyl-aziridine-2-carboxylic acid methyl ester

2-(4-Benzyloxy-benzenesulfonylamino)-3-hydroxy-butyric acid methyl ester (7 g,18.5 mmol) was dissolved in tetrahydrofuran (300 mL) and triphenylphosphine was added (7.27 g, 27.2 mmol). Diethylazodicarboxylate (4.29 mL, 27.7 mmol) was added dropwise over 5 minutes. The reaction was stirred for 12 hours and the partitioned between ethyl acetate and water. The organic portion was extracted with brine and dried over sodium sulfate. The solvent was removed *in* vacuo and the crude material chromatographed on silica gel with 20% ethyl acetate in hexanes to give the title compound (4.2 g, 63%).

### 2-(4-Benzyloxy-benzenesulfonylamino)-3-(2-bromo-ethoxy)-butyric acid methyl ester

1-(4-Benzyloxy-benzenesulfonyl)-3-methyl-aziridine-2-carboxylic acid methyl ester (4 g, 11.08 mmol) was combined with bromoethanol (10 mL) and boron trifluoride etherate (1.85 mL) at room temperature. The reaction was stirred for 12 hours and the volatile portion removed *in vacuo*. The residue was partitioned between ethyl acetate and water. The organic portion was dried over sodium sufate and the solvent removed *in vacuo*. The residue was chromatographed on silica gel with 20% ethyl acetate in hexane to provide the title compound (4 g, 84 %).

### 4-(4-Benzyloxy-benzenesulfonyl)-2-methyl-morpholine-3-carboxylic acid hydroxyamide

2-(4-Benzyloxy-benzenesulfonylamino)-3-(2-bromo-ethoxy)-butyric acid methyl ester (4.0 g, 11.1 mmol) was dissolved in dimethylformamide (40 mL) and stirred for 2 hours at room temperature with potassium carbonate (4.0 g, 28.9 mmol). The reaction was partitioned betweeen ethyl acetate and water. The organic portion was washed with brine and dried over sodium sufate. The solvent was removed *in vacuo* . This material was converted to the hydroxamic acid in a method analogous to that of Example 3.

### EXAMPLE 5

### (2S,3R,6S)-4-[4-(4-FLUORO-BENZYLOXY)-BENZENESULFONYL]-2,6-DIMETHYL-MORPHOLINE-3-CARBOXYLIC ACID HYDROXYAMIDE

The title compound was prepared in manner similar to Example 3 except steps 4 and 5 were omitted.

### EXAMPLE 6

### (3R,6S)-4-[4-(4-FLUORO-BENZYLOXY)-BENZENESULFONYL]-2,2,6-TRIMETHYL-MORPHOLINE-3-CARBOXYLIC ACID HYDROXYAMID

The title compound was prepared in similar manner to Example 3 except (2R)-2-amino-3-hydroxy-3-methyl-butyric acid was substituted for D-threonine in step 1 and steps 4 and 5 were omitted.

### EXAMPLE 7

### (2S,3R,6S)-6-ETHYL-4-[4-(4-FLUORO-BENZYLOXY)-BENZENESULFONYL]-2-METHYL-MORPHOLINE-3-CARBOXYLIC ACID HYDROXYAMIDE

The title compound was prepared in similar manner to Example 3 except an appropriate amount of crotyl bromide was substituted for allyl iodide in step 2 and steps 4 and 5 were omitted..

### EXAMPLE 8

### (2R,3R,6S)4-[4-(4-FLUORO-BENZYLOXY)-BENZENESULFONYL]-2,6-DIMETHYL-MORPHOLINE-3-CARBOXYLIC ACID HYDROXYAMIDE AND (2R,3R,6R)4-[4-(4-FLUORO-BENZYLOXY)-BENZENESULFONYL]-2,6-DIMETHYL-MORPHOLINE-3-CARBOXYLIC ACID HYDROXYAMIDE

The title compounds were prepared in similar manner to Example 3 except D-*allo*-Threonine was substituted for D- Threonine in step 1 and steps 4 and 5 were omitted. A mixture of isomers was obtained and separated by silica gel chromatography in step 3 and carried separately through the synthesis.

### EXAMPLE 9-12

### (2S,3R,6S)-2,6-DIMETHYL-4-[4-(PYRIDIN-4-YLMETHOXY)-BENZENESULFONYL]-MORPHOLINE-3-CARBOXYLIC ACID HYDROXYAMIDE;

### (2S,3R,6S)-2,6-DIMETHYL-4-[4-(PYRIDIN-2-YLMETHOXY)-BENZENESULFONYL]-MORPHOLINE-3-CARBOXYLIC ACID HYDROXYAMIDE;

### (2S,3R,6S)-2,6-DIMETHYL-4-[4-(PYRIDIN-3-YLMETHOXY)-BENZENESULFONYL]-MORPHOLINE-3-CARBOXYLIC ACID HYDROXYAMIDE; and

### (2S,3R,6S)-2,6-DIMETHYL-4-[4-(2-METHYL-PYRIDIN-3-YLMETHOXY)-BENZENESULFONYL]-MORPHOLINE-3-CARBOXYLIC ACID HYDROXYAMIDE

The title compounds were prepared in similar manner to Example 3 except that an appropriate amount of the required pyridyl chloride was used in step 5. Also , the hydroxamic acid was formed directly from the acid as follows:

(2*S*,3*R*,6*S*)2,6-Dimethyl-4-[4-(pyridin-4-ylmethoxy)-benzenesulfonyl]-morpholine-3-carboxylic acid (0.487 g, 1.2 mmol) was dissolved in dichloromethane (10 mL) and dimethylformamide ( 0.1 mL) and cooled to 0°C. Oxalyl chloride (0.27 mL) was added slowly and the reaction stirred at 0°C for 1 hour. The solvent was removed at the rotary evaporator and the crude solid suspended in a minimum of tetrahydrofuran. This suspension was slowly added to a solution of aqueous hydroxyl amine (50%, 0.08 mL) in tetrahydrofuran (2 mL) at 0°C. After stirring at 0°C for 1 hour, the reaction was partitioned between ethyl acetate and water. The pH of the aqueous phase was adjusted to 8 by addition of sodium bicarbonate. The organic portions were dried over sodium sulfate and the solvent removed *in vacuo*. The residue was chromatographed on silica gel with methanol (5%) and acetic acid (2%) in dichloromethane to provide the title compound as a white solid.

### EXAMPLE 13

### (3R,6S)-2,2,6-TRIMETHYL-4-[4-(2-TRIFLUOROMETHYL-BENZYLOXY)-BENZENESULFONYL]-MORPHOLINE-3-CARBOXYLIC ACID HYDROXYAMIDE

The title compound was prepared in manner similar to Example 3 except (2R)-2-amino-3-hydroxy-3-methyl-butyric acid was used in step 1 and an appropriate amount of 4-(2-trifluoromethyl-benzyloxy)-benzenesulfonyl chloride was used in step 5.

### EXAMPLE 14

### (2S,3R)-2,6,6-TRIMETHYL-4-[4-(PYRIDIN-4-YLMETHOXY)-BENZENESULFONYL]-MORPHOLINE-3-CARBOXYLIC ACID HYDROXYAMIDE

The hydroxamic acid was prepared from an ester in manner similar to Example 9. The ester was obtained in a manner similar to Example 3 except an appropriate amount of 1-bromo-2-methylpropene was used instead of allyl iodide in step 2 and 4-chloromethyl-pyridine was used in step 5. The title compound was isolated by precipitation from diethyl ether as its hydrochloric acid salt.

### EXAMPLE 15

### 2,2,6-TRIMETHYL-4-[4-(2-METHYL-PYRIDIN-3-YLMETHOXY)-BENZENESULFONYL]-MORPHOLINE-3-CARBOXYLIC ACID HYDROXYAMIDE

The title compound was prepared in similar manner to Example 12 except from an intermediate derived by substituting an appropriate amount of (2*R*)-2-amino-3-hydroxy-3-methyl-butyric acid for D-threonine in step 1 of Example 3.

### EXAMPLES 16-21

### (2S,3R,6S)-[4-(2,5-DIMETHYL-BENZYLOXY)-BENZENESULFONYL]-2,6-DIMETHYL-MORPHOLINE-3-CARBOXYLIC ACID HYDROXYAMIDE;

### (2S,3R,6S)-4-[4-(3,5-DIFLUORO-BENZYLOXY)-BENZENESULFONYL]-2,6-DIMETHYL-MORPHOLINE-3-CARBOXYLIC ACID HYDROXYAMIDE;

### (2S,3R,6S)-4-[4-(3-METHOXY-BENZYLOXY)-BENZENESULFONYL]-2,6-DIMETHYL-MORPHOLINE-3-CARBOXYLIC ACID HYDROXYAMIDE;

### (2S,3R,6S)-4-[4-(5-FLUORO-2-METHYL-BENZYLOXY)-BENZENESULFONYL]-2,6-DIMETHYL-MORPHOLINE-3-CARBOXYLIC ACID HYDROXYAMIDE;

### (2S,3R,6S)-4-[4-(FURAN-3-YLMETHOXY)-BENZENESULFONYL]-2,6-DIMETHYL-MORPHOLINE-3-CARBOXYLIC ACID HYDROXYAMIDE; and

### (2S,3R,6S)-4-[4-(2-FLUORO-3-METHYL-BENZYLOXY)-BENZENESULFONYL]-2,6-DIMETHYL-MORPHOLINE-3-CARBOXYLIC ACID HYDROXYAMIDE

The title compounds were prepared in similar manner to Example 3 except the required amount of an appropriately substituted aryl bromide or chloride was used in step 5.

### EXAMPLE 22

### (2S,3R)-4-[4-(4-FLUORO-BENZYLOXY)-BENZENESULFONYL]-2,6,6-TRIMETHYL-MORPHOLINE-3-CARBOXYLIC ACID HYDROXYAMIDE

The title compound was prepared in similar manner to Example 3 except an appropriate amount 1-bromo-2-methylpropene was substituted in step 2 and steps 4 and 5 were omitted.

### EXAMPLE 23

### (3R)-4-[4-(4-FLUORO-BENZYLOXY)-BENZENESULFONYL]-6,6-DIMETHYL-MORPHOLINE-3-CARBOXYLIC ACID HYDROXYAMIDE

The title compound was prepared in similar manner to Example 3 except an appropriate amount of D-serine was substituted for D-threonine in step 1 and an appropriate amount 1-bromo-2-methylpropene was substituted in step 2 and steps 4 and 5 were omitted..

### EXAMPLE 24

### (3R)-6,6-DIMETHYL-4-[4-(PYRIDIN-4-YLMETHOXY)-BENZENESULFONYL]-MORPHOLINE-3-CARBOXYLIC ACID HYDROXYAMIDE

The title compound was prepared in similar manner to Example 9 except from an intermediate derived by substituting an appropriate amount of D-serine for D-threonine in step 1 of Example 3.

### EXAMPLE 25

### (3R)-6,6-DIMETHYL-4-[4-(2-METHYL-BENZYLOXY)-BENZENESULFONYL]-MORPHOLINE-3-CARBOXYLIC ACID HYDROXYAMIDE

The title compound was prepared in similar manner to Example 3 except an appropriate amount of D-serine was substituted for D-threonine in step 1 and an appropiate amount of 2-methylbenzyl bromide was used in step 5.

### EXAMPLE 26

### (2S,3R,6S)-4-(4-CYCLOHEXYLMETHOXY-BENZENESULFONYL)-2,6-DIMETHYL-MORPHOLINE-3-CARBOXYLIC ACID HYDROXYAMIDE

The title compound was prepared in similar manner to Example 3 except an appropriate amount of (bromomethyl)cyclohexane was used in step 5 of Example 3.

### EXAMPLE 27

### (3R,6S)-4-[4-(2,5-DIMETHYL-BENZYLOXY)-BENZENESULFONYL]-2,2,6-TRIMETHYL-MORPHOLINE-3-CARBOXYLIC ACID HYDROXYAMIDE

The title compound was prepared in similar manner to Example 3 except an appropriate amount of (2*R*)-2-amino-3-hydroxy-3-methyl-butyric acid was substitued for D-threonine in step 1 and 2,5-dimethylbenzyl bromide was used in step 5 of Example 3.

### EXAMPLE 28

### (2S,3R,6R)-4-[4-(4-FLUORO-BENZYLOXY)-BENZENESULFONYL]-6-METHOXYMETHYL-2-METHYL-MORPHOLINE-3-CARBOXYLIC ACID HYDROXYAMIDE

### (2S,3R,6R)-4-[4-(4-fluoro-benzyloxy)-phenylsulfanyl]-6-iodomethyl-2-methylmorpholine-3-carboxylic acid methyl ester

(2*R*,3*S*)-2-{Allyl-[4-(4-fluoro-benzyloxy)-benzenesulfonyl]-amino}-3-hydroxy-butyric acid methyl ester (1.14 g, 2.6 mmol) was dissolved in tetrahydrofuran (28 mL). Potassium carbonate (0.47 g, 3.4 mmol) and mercury(II) trifluoroacetate (1.46 g, 3.4 mmol) were added sequentially at room temperature. The mixture was stirred for 75 minutes at room temperature, then iodine (1.65 g, 6.5 mmol) was added. After 1 hour, the reaction was partitioned between ethyl acetate and aqueous hydrochloric acid (1N). The organic portion was washed with aqueous saturated sodium bisufite and dried over magnesium sulfate. The solvent was removed *in vacuo* and the residue chromatographed on silica gel with 30% ethyl acetate in hexanes to give the title compound (1.5 g, 60%).

### (2S,3R,6R)-4-[4-(4-fluoro-benzyloxy)-phenylsulfanyl]-6-methoxymethyl-2-methylmorpholine-3-carboxylic acid methyl ester

(2*S*,3*R*,6*R*)4-[4-(4-Fluoro-benzyloxy)-phenylsulfanyl]-6-iodomethyl-2-methylmorpholine-3-carboxylic acid methyl ester (0.365 g, 0.65 mmol) was dissolved in methanol (10 mL) and carbon tetrachloride (10 mL) and a steady stream of chlorine gas was bubbled through the solution for 20 seconds. After stirring for 5 minutes, ethyl acetate was added. The solution was extracted with sodium bisulfite and sodium bicarbonate, then dried over magnesium sulfate. The solvent was removed *in vacuo* and the residue chromatographed on silica gel with 30% ethyl acetate in hexanes to give the title compound (0.211 g, 69%).

(2*S*,3*R*,6*R*)-4-[4-(4-Fluoro-benzyloxy)-benzenesulfonyl]-6-methoxymethyl-2-methylmorpholine-3-carboxylic acid hydroxyamide 4-[4-(4-Fluoro-benzyloxy)-phenylsulfanyl]-6-methoxymethyl-2-methyl-morpholine-3-carboxylic acid methyl ester was transformed to the corresponding hydroxamic acid in a manner similar to steps 6 and 7 for Example 3

### EXAMPLE 29

### (2S,3R,6S)-4-[4-(3-CHLORO-BENZYLOXY)-BENZENESULFONYL]-6-[(ETHYLMETHYL-AMINO)-METHYL]-2-METHYL-MORPHOLINE-3-CARBOXYLIC ACID HYDROXYAMIDE

### (2S,3R,6R)-4-[4-(3-Chloro-benzyloxy)-phenylsulfanyl]-6-iodomethyl-2-methyl-morpholine-3-carboxylic acid methyl ester

The title compound was prepared in a similar manner to (2*S*,3*R*,6*R*)-4-[4-(4-Fluoro-benzyloxy)-phenylsulfanyl]-6-iodomethyl-2-methyl-morpholine-3-carboxylic acid methyl ester in Example 28.

### (2S,3R,6S)-4-[4-(3-Chloro-benzyloxy)-phenylsulfanyl]-6-[(ethyl-methyl-amino)-methyl]-2-methyl-morpholine-3-carboxylic acid methyl ester

4-[4-(3-Chloro-benzyloxy)-phenylsulfanyl]-6-iodomethyl-2-methyl-morpholine-3-carboxylic acid methyl ester (3.8 g, 6.6 mmol) was dissolved in dimethylformamide (10 mL) and ethyl methylamine (10 mL) and stirred for 12 hours at 60°C. The reaction was partitioned between ethyl acetate and water. The organic portion was dried over sodium sulfate and the solvents removed *in vacuo*.

### (2S,3R,6S)-4-[4-(3-Chloro-benzyloxy)-benzenesulfonyl]-6-[(ethyl-methyl-amino)-methyl]-2-methyl-morpholine-3-carboxylic acid hydroxyamide

Hydroxylamine hydrochloride (1.0 g, 14.4 mmol) was suspended in methanol ( 8 mL). Potassium hydroxide (1.0 g, 17.8 mmol) was dissolved in methanol (4 mL) and added to the hydroxylamine solution. After the reaction had stirred for 15 minutes, (2*S*,3*R*,6*S*)-4-[4-(3-Chloro-benzyloxy)-phenylsulfanyl]-6-[(ethyl-methyl-amino)-methyl]-2-methyl-morpholine-3-carboxylic acid methyl ester (1.0 g, 1.96 mmol) was added. The reaction was stirred at room temperature for 1 hour then the solvent was removed *in vacuo*. The residue was partitioned between ethyl acetate and hydrochloric acid (1N). The organic portion was dried over sodium sulfate and the solvent removed *in vacuo*. The title compound (0.175 g, 17%) was isolated by chromatography on silica gel with 80% ethyl acetate in hexanes.

### EXAMPLE 30

### (2S,3R)-4-[4-(3-CHLORO-BENZYLOXY)-BENZENESULFONYL]-6-METHOXY-2-METHYL-MORPHOLINE-3-CARBOXYLIC ACID HYDROXYAMIDE

### 2-[[4-(3-Chloro-benzyloxy)-phenylsulfanyl]-(3-methyl-but-2-enyl)-amino]-3-hydroxybutyric acid methyl ester

The title compound was prepared in a manner similar to 2-{Allyl-[4-(4-fluoro-benzyloxy)-benzenesulfonyl]-amino}-3-hydroxy-butyric acid methyl ester according to step 2 of Example 3.

### 4-[4-(3-Chloro-benzyloxy)-phenylsulfanyl]-6-methoxy-2-methyl-morpholine-3-carboxylic acid methyl ester

2-[[4-(3-Chloro-benzyloxy)-phenylsulfanyl]-(3-methyl-but-2-enyl)-amino]-3-hydroxybutyric acid methyl ester (1.5 g, 3.11), sodium periodate (1.46 g, 6.8 mmol), and osmium tetroxide (4% in water, a few drops) were combined in dioxane (30 mL) and water (15 mL). After stirring overnight, the solution was partitioned between ethyl acetate and water. The organic portion was dried over sodium sufate and the solvent removed *in vacuo*. The residue and toluene sulfonic acid (catalytic) were dissolved in methanol (100 mL) and heated at reflux overnight. The solvent was removed *in vacuo* and the residue chromatographed on silica gel with ethyl acetate in hexanes to give the title compound (0.93 g, 64 %).

### 4-[4-(3-Chloro-benzyloxy)-benzenesulfonyl]-6-methoxy-2-methyl-morpholine-3-carboxylic acid hydroxyamide

The title compound was prepared by conversion of 4-[4-(3-Chloro-benzyloxy)-phenylsulfanyl]-6-methoxy-2-methyl-morpholine-3-carboxylic acid methyl ester directly to the hydroxamic acid in a manner similar to that used for Example 29.

### EXAMPLE 31

### 4-[4-(4-FLUORO-BENZYLOXY)-BENZENESULFONYL]-6-HYDROXYMETHYL-2-METHYL-MORPHOLINE-3-CARBOXYLIC ACID HYDROXYAMIDE

### 2-{[4-(4-Fluoro-benzyloxy)-phenylsulfanyl]-oxiranylmethyl-amino}-3-hydroxy-butyric acid methyl ester

2-{Allyl-[4-(4-fluoro-benzyloxy)-benzenesulfonyl]-amino}-3-hydroxy-butyric acid methyl ester (1.5 g, 3.43 mmol) was dissolved in dichloromethane (10 mL). Sodium bicarbonate (1.41 g, 16.7 mmol) and m-chloroperoxybenzoic acid (0.785 g, 3.4 mmol) were added sequentially. The reaction was stirred for 14.5 hours at room temperature. An additional portion of m-chloroperoxybenzoic (0.4 g, 1.75 mmol) was added. After stirring for 4 hours diethyl ether (100 mL) and ethyl acetate (50 mL) were added. This solution was extracted with sodium bisulfite (2 x 10 mL), sodium bicarbonate (4 x 10 mL) and brine (1 x 10 mL). The organic portion was dried over magnesium sulfate and the solvents removed *in vacuo*. The residue was chromatographed on silica gel (biotage 40M column) with 50 % ethyl acetate in hexanes to give the title compound (1.23 g, 79%).

### 4-[4-(4-Fluoro-benzyloxy)-phenylsulfanyl]-6-hydroxymethyl-2-methyl-morpholine-3-carboxylic acid methyl ester

2-{[4-(4-Fluoro-benzyloxy)-phenylsulfanyl]-oxiranylmethyl-amino}-3-hydroxy-butyric acid methyl ester (1.15g, 2.54 mmol) was dissolved in dichloromethane (20 mL) and cooled to 0°C. *p*-Toluene sulfonic acid monohydrate (0.05 g, 0.26 mmol) was added and the reaction stirred at 0°C for 2 hours. Excess sodium bicarbonate was added and the reaction warmed to room temperature. The solids were removed by filtration and the solvent removed *in vacuo.* The residue was chromatographed on silica gel (biotage 40M column) with 15 % ethyl acetate in dichloromethane to provide the separated diastereomers of the title compound (0.5 g of each, 87%).

### 6-(tert-Butyl-dimethyl-silanyloxymethyl)-4-[4-(4-fluoro-benzyloxy)-phenylsulfanyl]-2-methyl-morpholine-3-carboxylic acid methyl ester

Imidazole (0.4 g, 5.9 mmol) and *t*-butyldimethylsilyl chloride (0.4 g, 2.65 mmol) were added sequentially to dichloromethane (10 mL). The solution was stirred for 5 minutes during which time a precipitate formed. The solids were removed by filtration and the solution added to the 4-[4-(4-Fluoro-benzyloxy)-phenylsulfanyl]-6-hydroxymethyl-2-methyl-morpholine-3-carboxylic acid methyl ester (0.495 g, 1.09 mmol). The reaction was stirred at room temperature for 2 hours then diluted with diethyl ether. This solution was extracted with sodium hydroxide (1N), hydrochloric acid (1N) and brine. The organic portion was dried over magnesium sulfate and the organic portion removed *in vacuo*. The residue was chromatographed on silica gel (biotage 40S column) with a mixture of ethyl acetate and hexane to give the title compound (0.54 g, 87%).

### 6-(tert-Butyl-dimethyl-silanyloxymethyl)-4-[4-(4-fluoro-benzyloxy)-phenylsulfanyl]-2-methyl-morpholine-3-carboxylic acid allyloxy-amide

The title compound (0.230 g, 40 %) was obtained from the 6-(tert-Butyl-dimethyl-silanyloxymethyl)-4-[4-(4-fluoro-benzyloxy)-phenylsulfanyl]-2-methyl-morpholine-3-carboxylic acid methyl ester (0.54 g, 0.95 mmol) in a manner similar to that described in step 6 of Example 3.

### 4-[4-(4-Fluoro-benzyloxy)-benzenesulfonyl]-6-hydroxymethyl-2-methyl-morpholine-3-carboxylic acid hydroxyamide

The hydroxamic acid was obtained from 6-(tert-Butyl-dimethyl-silanyloxymethyl)-4-[4-(4-fluoro-benzyloxy)-phenylsulfanyl]-2-methyl-morpholine-3-carboxylic acid allyloxy-amide (0.23 g, 0.38 mmol) in a manner similar to that described in step 7 of Example 3 and used without further purification. The crude hydroxamic acid was dissolved in tetrahydrofuran (10 mL) and cooled to 0°C. tetra-n-Butyl ammonium fluoride (1.0 mmol, 1M in tetrahydrofuran) was added and the reaction stirred at 0°C for 1 hour then at room temperature for 2 hours. The tetrahydrofuran was removed at the rotory evaporator and the residue partitioned between ethyl acetate and hydrochloric acid (1N). The organic portion was dried over magnesium sulfate and the solvent removed *in vacuo*. The residue was chromatographed on silica gel (biotage 40S column) with 1 % acetic acid in ethyl acetate to give the title compound (0.068 g, 39 %).

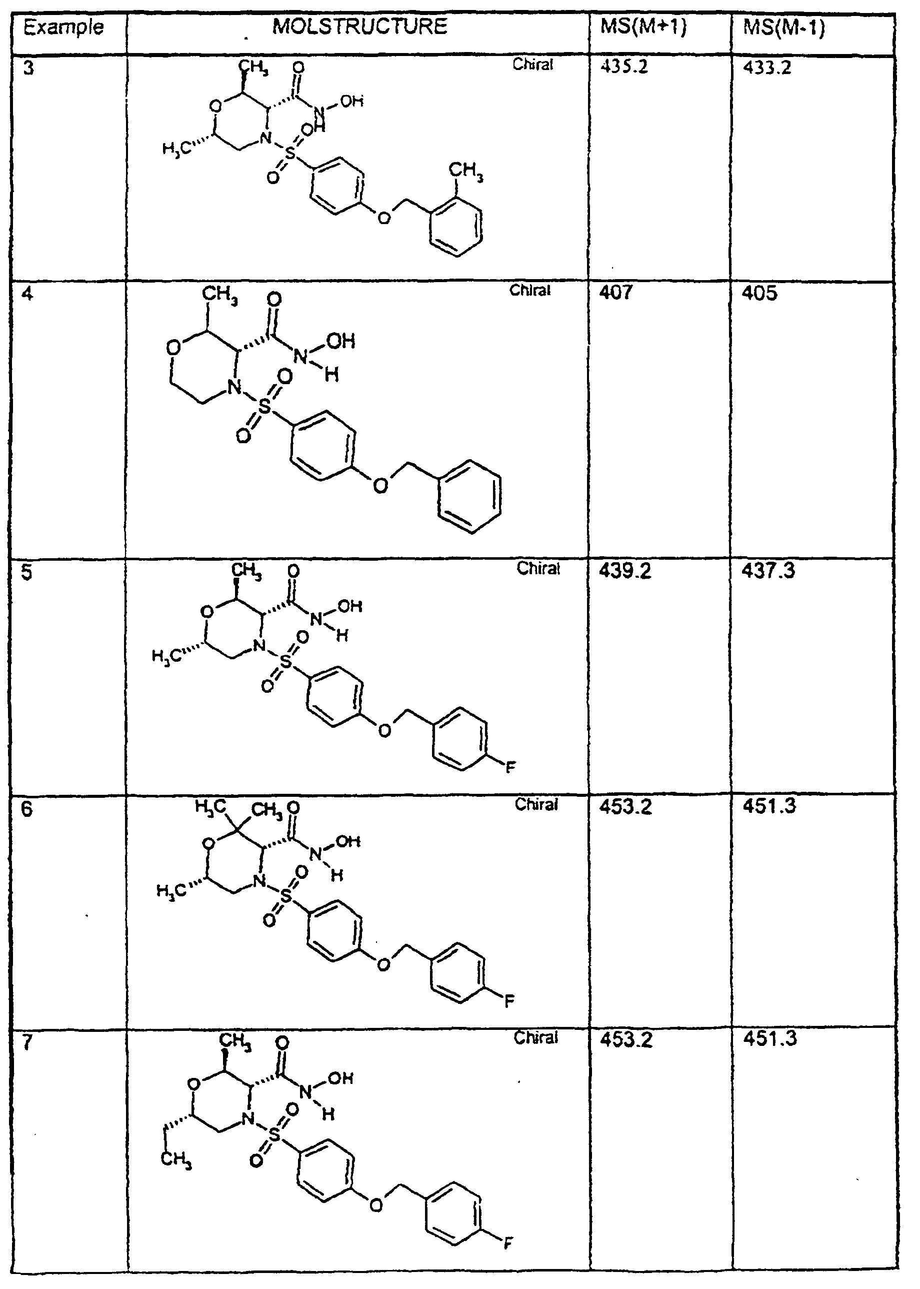

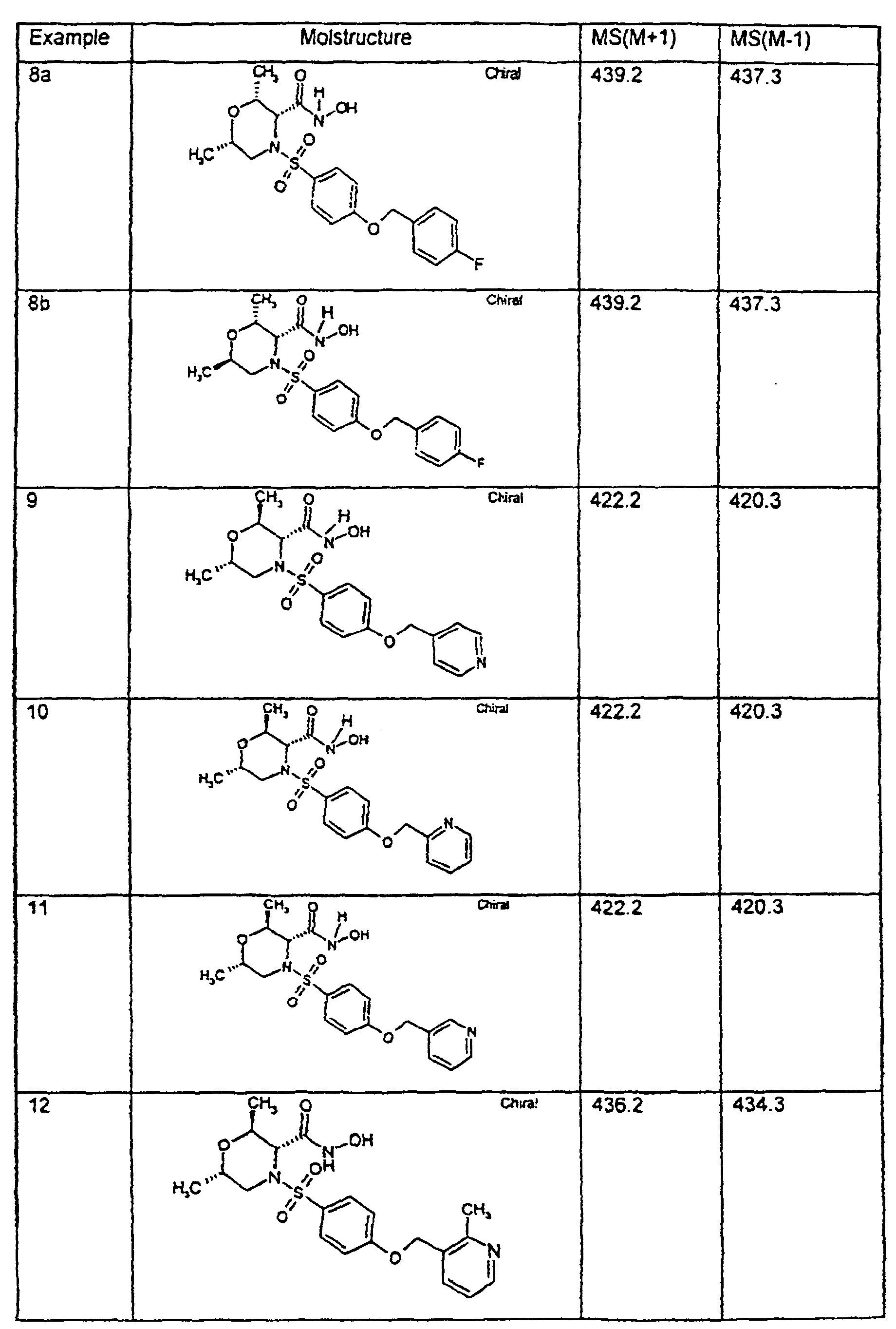

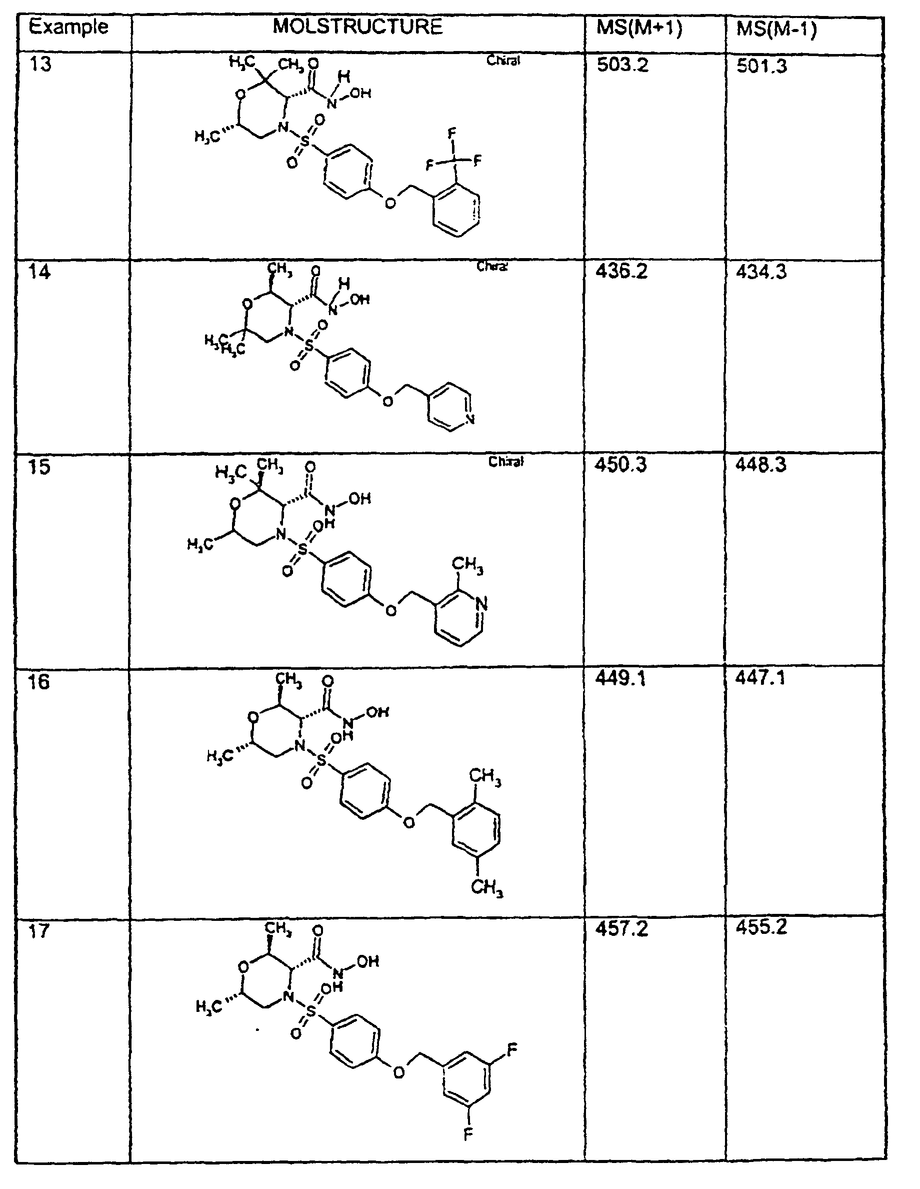

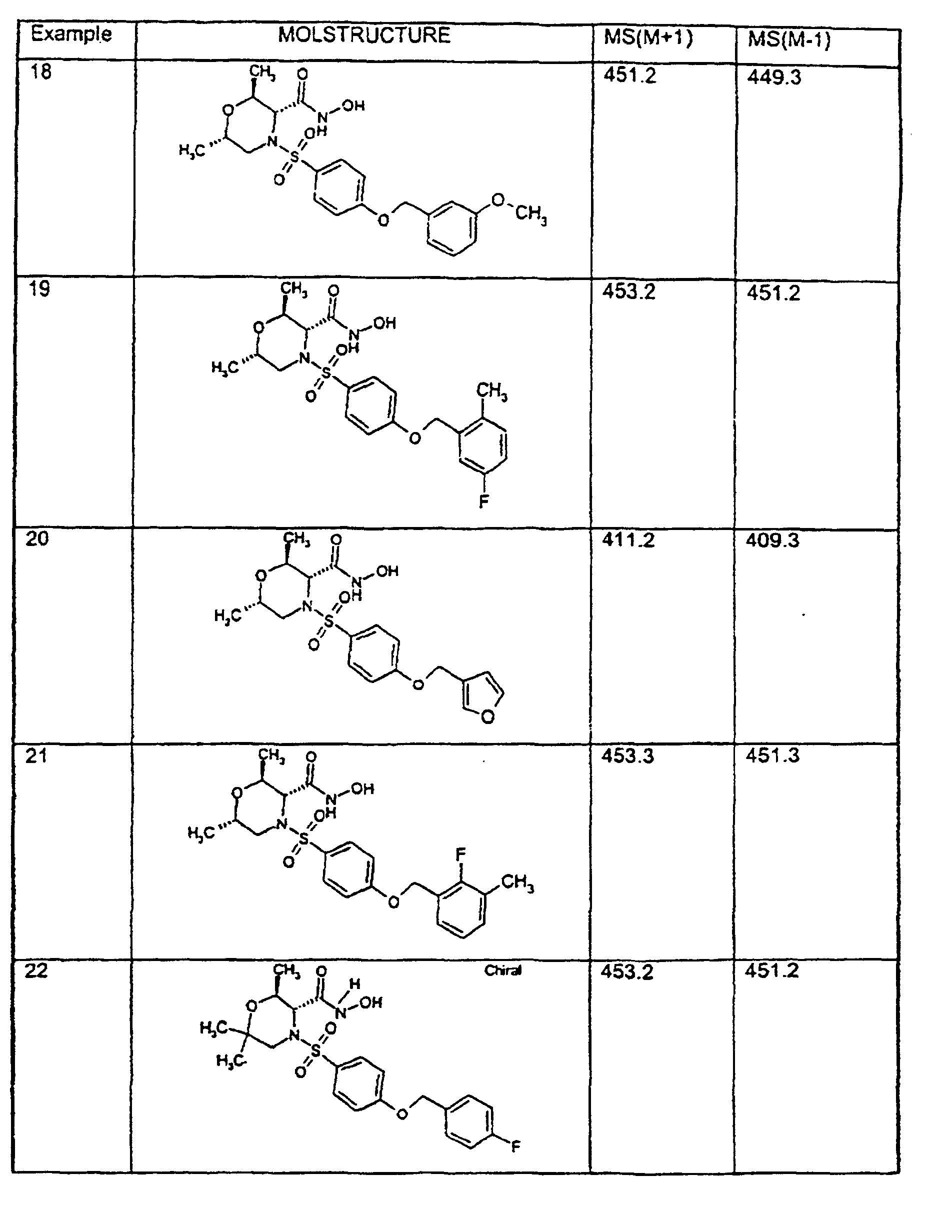

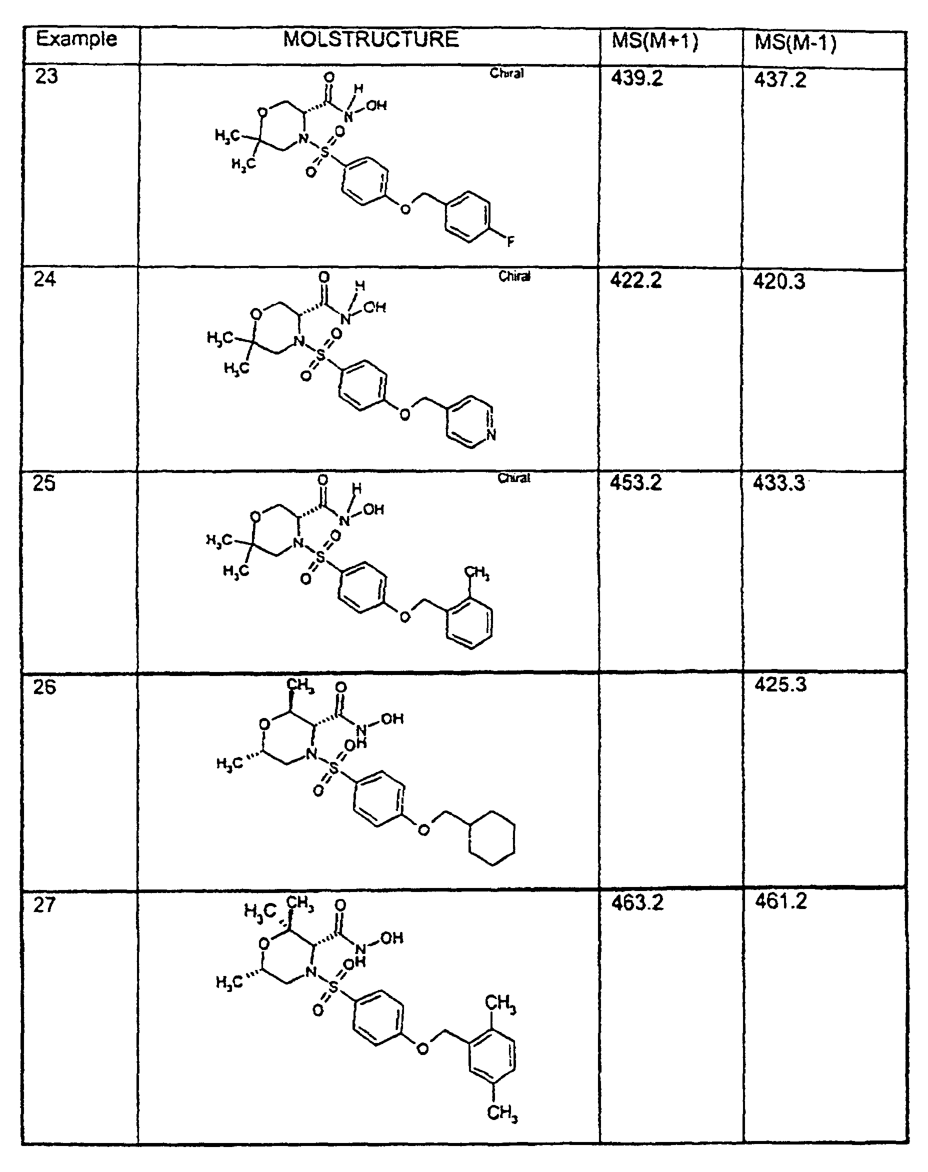

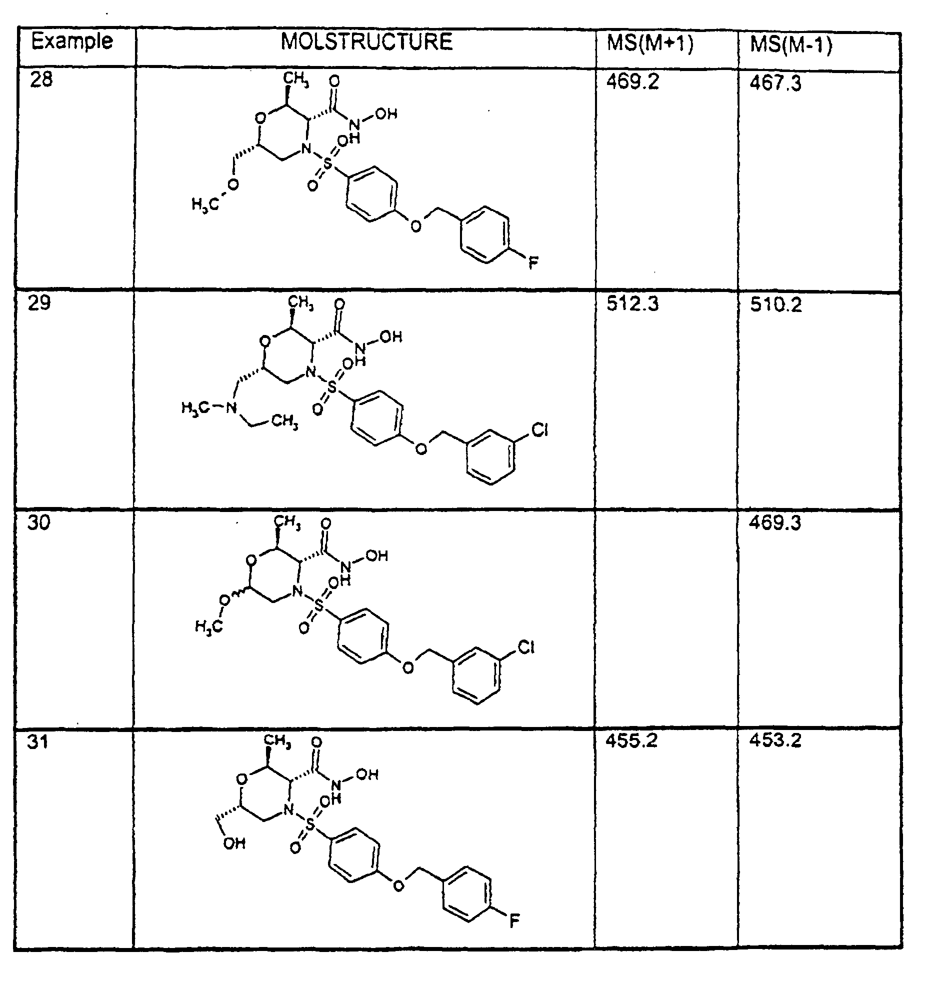

## Claims

1. A compound of the formula 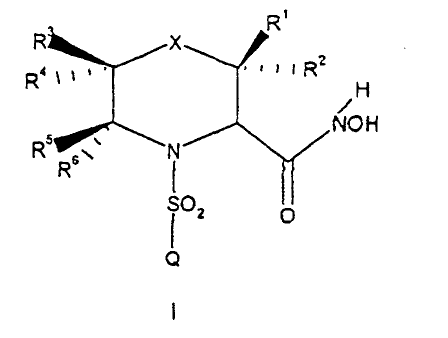 or the pharmaceutically acceptable salt thereof, wherein
X is oxygen, sulfur, SO, SO₂ or NR⁷;
R¹, R², R³, R⁴, R⁵ and R⁶ are selected from the group consisting of hydrogen, hydroxy, -CN, (C₁-C₆)alkyl, (C₂-C₆)alkenyl, (C₆-C₁₀)aryl(C₂-C₆)alkenyl, (C₂-C₉)heteroaryl(C₂-C₆)alkenyl, (C₂-C₆)alkynyl, (C₆-C₁₀)aryl(C₂-C₆)alkynyl, (C₂-C₉)heteroaryl(C₂-C₆)alkynyl, (C₁-C₆)alkylamino, (C₁-C₆)alkylthio, (C₁-C₆)alkoxy, perfluoro(C₁-C₆)alkyl, (C₆-C₁₀)aryl, (C₂-C₉)heteroaryl, (C₆-C₁₀)arylamino, (C₆-C₁₀)arylthio, (C₆-C₁₀)aryloxy, (C₂-C₉)heteroarylamino, (C₂-C₉)heteroarylthio, (C₂-C₉)heteroaryloxy, (C₃-C₆)cycloalkyl, (C₁-C₆)alkyl(hydroxymethylene), piperidyl, (C₁-C₆)alkylpiperidyl, (C₁-C₆)acylamino, (C₁-C₆)acylthio, (C₁-C₆)acyloxy, (C₁-C₆)alkoxy-(C=O)-, -CO₂H, (C₁-C₆)alkyl-NH-(C=O)-, and [(C₁-C₆)alky]₂-N-(C=O)-;
wherein said (C₁-C₆)alkyl is optionally substituted by one or two groups selected from (C₁-C₆)alkylthio, (C₁-C₆)alkoxy, trifluoromethyl, halo, -CN, (C₆-C₁₀)aryl, (C₂-C₉)heteroaryl, (C₆-C₁₀)arylamino, (C₆-C₁₀)arylthio, (C₆-C₁₀)aryloxy, (C₂-C₉)heteroarylamino, (C₂-C₉)heteroarylthio, (C₂-C₉)heteroaryloxy, (C₆-C₁₀)aryl(C₆-C₁₀)aryl, (C₃-C₆)cycloalkyl, hydroxy, piperazinyl, (C₆-C₁₀)aryl(C₁-C₆)alkoxy, (C₂-C₉)heteroaryl(C₁-C₆)alkoxy, (C₁-C₆)acylamino, (C₁-C₆)acylthio, (C₁-C₆)acyloxy, (C₁-C₆)alkylsulfinyl, (C₆-C₁₀)arylsulfinyl, (C₁-C₆)alkylsulfonyl, (C₆-C₁₀)arylsulfonyl, amino, (C₁-C₆)alkylamino or ((C₁-C₆)alkyl)₂amino;
R⁷ is hydrogen or (C₁-C₆)alkyl optionally substituted by one or more of, hydroxy, -CN, (C₁-C₆)alkylamino, (C₁-C₆)alkylthio, (C₁-C₆)alkoxy, perfluoro(C₁-C₆)alkyl, (C₆-C₁₀)aryl, (C₆-C₁₀)arylthio, (C₆-C₁₀)aryloxy, (C₂-C₉)heteroarylamino, (C₃-C₆)cycloalkyl, (C₁-C₆)alkyl(hydroxymethylene), piperidyl, (C₁-C₆)alkylpiperidyl, (C₁-C₆)acylamino, (C₁-C₆)acyloxy, (C₁-C₆)alkoxy-(C=O)-, -CO₂H, (C₁-C₆)alkyl-NH-(C=O)-, and [(C₁-C₆)alky]₂-N-(C=O)-; (C₆-C₁₀)arylsulfonyl, (C₁-C₆)alkylsulfonyl, (C₁-C₆)alkyl-NH-(C=O)-, (C₁-C₆)alkoxy-(C=O)-, (C₁-C₆)alkyl-(C=O)-, [(C₁-C₆)alky]₂-N-(C=O)-, (R⁸R⁹N)-(C=O) where R⁸ and R⁹ are
taken together with the nitrogen that they are attached to form an azetidinyl, pyrrolidinyl, piperidinyl, morpholinyl and thiomorphonyl;
Q is (C₆-C₁₀)aryl(C₁-C₆)alkoxy(C₆-C₁₀)aryl, (C₆-C₁₀)aryl(C₁-C₆)alkoxy(C₂-C₉)heteroaryl, (C₂-C₉)heteroaryl(C₁-C₆)alkoxy(C₆-C₁₀)aryl, or (C₂-C₉)heteroaryl(C₁-C₆)alkoxyC₂-C₉)heteroaryl, wherein each of said (C₆-C₁₀)aryl or (C₂-C₉)heteroaryl groups may optionally be substituted by one or more substituents, preferably one to three substituents per ring, most preferably one to three substituents on the terminal ring (i.e. the ring furthest from the point of attachment) independently selected from the group consisting of halo, -CN, (C₁-C₆)alkyl optionally substituted with one or more fluorine atoms (preferably one to three fluorine atoms), hydroxy, hydroxy-(C₁-C₆)alkyl, (C₁-C₆)alkoxy optionally substituted with one or more fluorine atoms (preferably one to three fluorine atoms), (C₁-C₆)alkoxy(C₁-C₆)alkyl, HO-(C=O)-, (C₁-C₆)alkyl-O-(C=O)-, HO-(C=O)-(C₁-C₆)alkyl, (C₁-C₆)alkyl-O-(C=O)-(C₁-C₆)alkyl, (C₁-C₆)alkyl-(C=O)-O-, (C₁-C₆)alkyl-(C=O)-O-(C₁-C₆)alkyl, H(O=C)-, H(O=C)-(C₁-C₆)alkyl, (C₁-C₆)alkyl(O=C)-, (C₁-C₆)alkyl(O=C)-(C₁-C₆)alkyl, NO₂, amino, (C₁-C₆)alkylamino, [(C₁-C₆)alkyl]₂amino, amino(C₁-C₆)alkyl, (C₁-C₆)alkylamino(C₁-C₆)alkyl, [(C₁-C₆)alkyl]₂amino(C₁-C₆)alkyl, H₂N-(C=O)-, (C₁-C₆)alkyl-NH-(C=O)-, [(C₁-C₆)alkyl]₂N-(C=O)-, H₂N(C=O)-(C₁-C₆)alkyl, (C₁-C₆)alkyl-HN(C=O)-(C₁-C₆)alkyl, [(C₁-C₆)alkyl]₂N-(C=O)-(C₁-C₆)alkyl, H(O=C)-NH-, (C₁-C₆)alkyl(C=O)-NH, (C₁-C₆)alkyl(C=O)-[NH](C₁-C₆)alkyl, (C₁-C₆)alkyl(C=O)-[N(C₁-C₆)alkyl](C₁-C₆)alkyl, (C₁-C₆)alkyl-S-, (C₁-C₆)alkyl-(S=O)-, (C₁-C₆)alkyl-SO₂-, (C₁-C₆)alkyl-SO₂-NH-, (C₁-C₆)alkyl-SO₂-[N-(C₁-C₆)alkyl]-, H₂N-SO₂-, H₂N-SO₂-(C₁-C₆)alkyl, (C₁-C₆)alkylHN-SO₂-(C₁-C₆)alkyl, [(C₁-C₆)alkyl]₂N-SO₂-(C₁-C₆)alkyl, CF₃SO₃-, (C₁-C₆)alkyl-SO₃-, phenyl, phenyl(C₁-C₆)alkyl, (C₃-C₁₀)cycloalkyl, (C₂-C₉)heterocycloalkyl, and (C₂-C₉)heteroaryl;
with the provisio that when X is SO or SO₂, and R³ and R⁴ are a substituent comprising a heteroatom, the heteroatom cannot be bonded to the ring:
and with the proviso that at least one of R¹-R⁶ must be (C₁-C₆)alkyl;
and with the proviso that when X is oxygen or sulfur and R³-R⁶ are each hydrogen then R¹ and R² cannot both be methyl.

2. A compound according to claim 1, wherein X is NR⁷, sulfur or oxygen.

3. A compound according to claim 1, wherein Q is (C₆-C₁₀)aryl(C₁-C₆)alkoxy(C₆-C₁₀)aryl, (C₆-C₁₀)aryl(C₁-C₆)alkoxy(C₂-C₉)heteroaryl, (C₂-C₉)heteroaryl(C₁-C₆)alkoxy(C₆-C₁₀)aryl or (C₂-C₉)heteroaryl(C₁-C₆)alkoxy(C₂-C₉)heteroaryl optionally substituted by one or more, preferably one to three substituents per ring, most preferably one to three substituents on the terminal ring, wherein said substituent is selected from halo, (C₁-C₆)alkyl, (C₁-C₆)alkoxy or perfluoro(C₁-C₃)alkyl.

4. A compound according to claim 1, wherein Q is (C₆-C₁₀)arylmethoxy(C₆-C₁₀)aryl, (C₆-C₁₀)arylmethoxy(C₂-C₉)heteroaryl, (C₂-C₉)heteroarylmethoxy(C₆-C₁₀)aryl or (C₂-C₉)heteroarylmethoxy(C₂-C₉)heteroaryl optionally substituted by one or more, preferably one to three substituents per ring, most preferably one to three substituents on the terminal ring, wherein said substituent is selected from halo, (C₁-C₆)alkyl, (C₁-C₆)alkoxy or perfluoro(C₁-C₃)alkyl.

5. A compound according to claim 1, wherein Q is optionally substituted (C₆-C₁₀)arylmethoxyphenyl, pyridylmethoxyphenyl, furylmethoxyphenyl, pyroylmethoxyphenyl, thienylmethoxyphenyl, isothiazolylmethoxyphenyl, imidazolylmethoxyphenyl, benzimidazolylmethoxyphenyl, tetrazolylmethoxyphenyl, pyrazinylmethoxyphenyl, pyrimidylmethoxyphenyl, quinolylmethoxyphenyl, isoquinolylmethoxyphenyl, benzofurylmethoxyphenyl, isobenzofurylmethoxyphenyl, benzothienylmethoxyphenyl, pyrazolylmethoxyphenyl, indolylmethoxyphenyl, isoindolylmethoxyphenyl, purinylmethoxyphenyl, carbazolylmethoxyphenyl, isoxazolylmethoxyphenyl, thiazolylmethoxyphenyl, oxazolylmethoxyphenyl, benzthiazolylmethoxyphenyl, benzoxazolylmethoxyphenyl.

6. A compound according to claim 1, wherein Q is (C₆-C₁₀)arylmethoxy(C₆)aryl optionally substituted by one or more, preferably one to three substituents per ring, most preferably one to three substituents on the terminal ring, wherein said substituents are independently selected from halo, (C₁-C₆)alkyl, (C₁-C₆)alkoxy or perfluoro(C₁-C₃)alkyl.

7. A compound according to claim 1, wherein Q is (C₆-C₁₀)arylmethoxy(C₂-C₉)heteroaryl optionally substituted by one or more, preferably one to three substituents per ring, most preferably one to three substituents on the terminal ring, wherein said substituents are independently selected from halo, (C₁-C₆)alkyl, (C₁-C₆)alkoxy or perfluoro(C₁-C₃)alkyl.

8. A compound according to claim 1, wherein Q is (C₂-C₉)heteroarylmethoxy(C₆)aryl optionally substituted by one or more, preferably one to three substituents per ring, most preferably one to three substituents on the terminal ring, wherein said substituents are independently selected from halo, (C₁-C₆)alkyl, (C₁-C₆)alkoxy or perfluoro(C₁-C₃)alkyl.

9. A compound according to claim 1, wherein Q is (C₂-C₉)heteroarylmethoxy(C₂-C₉)heteroaryl optionally substituted by one or more, preferably one to three substituents per ring, most preferably one to three substituents on the terminal ring, wherein said substituents are independently selected from halo, (C₁-C₆)alkyl, (C₁-C₆)alkoxy or perfluoro(C₁-C₃)alkyl.

10. A compound according to claim 1, wherein R⁴ is hydrogen.

11. A compound according to claim 1, wherein R² or R³ are hydrogen.

12. A compound according to claim 1, wherein at least one of R² or R³ is other than hydrogen.

13. A compound according to claim 1, wherein at least one of R¹-R³ is (C₁-C₆)alkyl.

14. A compound according to claim 2, wherein at least one of R¹-R³ is (C₁-C₆)alkyl.

15. A compound according to claim 3, wherein at least one of R¹-R³ is (C₁-C₆)alkyl.

16. A compound according to claim 1, wherein at least one of R¹-R³ is methyl.

17. A compound according to claim 2, wherein at least one of R¹-R³ is methyl.

18. A compound according to claim 3, wherein at least one of R¹-R³ is methyl.

19. A compound according to claim 3, wherein R¹ is (C₁-C₆)alkyl.

20. A compound according to claim 3, wherein R² is (C₁-C₆)alkyl.

21. A compound according to claim 3, wherein R³ is (C₁-C₆)alkyl.

22. A compound according to claim 3, wherein R⁴ is (C₁-C₆)alkyl.

23. A compound according to claim 1 wherein R¹ and R⁴ are each methyl.

24. A compound according to claim 2 wherein R¹ and R⁴ are each methyl.

25. A compound according to claim 3 wherein R¹ and R⁴ are each methyl.

26. A compound according to claim 1, wherein R¹ and R² are each (C₁-C₆)alkyl.

27. A compound according to claim 2, wherein R¹ and R² are each (C₁-C₆)alkyl.

28. A compound according to claim 3, wherein R¹ and R² are each (C₁-C₆)alkyl.

29. A compound according to claim 1, wherein R¹ and R² are each methyl.

30. A compound according to claim 2, wherein R¹ and R² are each methyl.

31. A compound according to claim 3, wherein R¹ and R² are each methyl.

32. A compound according to claim 1 wherein R¹ is methyl and R² is hydrogen;

33. A compound according to claim 1 wherein R¹ is hydrogen and R³ is methyl.

34. A compound according to claim 3, wherein R¹ and R³ are each methyl.

35. A compound according to claim 3, wherein R¹ and R⁴ are each methyl.

36. A compound according to claim 3, wherein R² and R³ are each methyl.

37. A compound according to claim 3, wherein R² and R⁴ are each methyl.

38. A compound according to claim 3, wherein R³ and R⁴ are each methyl.

39. A compound according to claim 1 wherein X is NR⁷.

40. A compound according to claim 3 wherein X is NR⁷.

41. A compound according to claim 4 wherein X is NR⁷.

42. A compound according to claim 5 wherein X is NR⁷.

43. A compound according to claim 6 wherein X is NR⁷.

44. A compound according to claim 13 wherein X is NR⁷.

45. A compound according to claim 15 wherein X is NR⁷.

46. A compound according to claim 1 wherein X is nitrogen and R⁷ is (C₁-C₆)alkyl.

47. A compound according to claim 1 wherein X is nitrogen and R⁷ is (C₁-C₆)alkylsulfonyl.

48. A compound according to claim 1 wherein X is nitrogen and R⁷ is (C₆-C₁₀)arylsulfonyl.

49. A compound according to claim 1 wherein X is nitrogen and R⁷ is [(C₁-C₆)alkyl]₂N-(C=O)- or (C₁-C₆)alkylNH-(C=O)-.

50. A compound according to claim 1 wherein X is nitrogen and R⁷ is (C₁-C₆)alkyl(C=O)-.

51. A compound according to claim 1, wherein said compound is selected from the group consisting of:
(2*S*,3*S*)-4-[4-(3,5-difluro-benzyloxy)-benzenesulfonyl]-2-methyl-thiomorpholine-3-carboxylic acid hydroxyamide;
(2*S*,3*S*)-4-[4-(4-fluoro-benzyloxy)-benzensulfonyl]-2-methyl-thiomorpholine-3-carboxylic acid hydroxyamide;
(2*S*,3*R*,6*S*)-2,6-dimethyl-4-[4-(2-methyl-benzyloxy)-benzenesulfonyl]-morpholine-3-carboxylic acid hydroxyamide;
4-(4-benzyloxy-benzenesulfonyl)-2-methyl-morpholine-3-carboxylic acid hydroxyamide;
(2*S*,3*R*,6*S*)-4-[4-(4-fluoro-benzyloxy)-benzenesulfonyl]-2,6-dimethyl-morpholine-3-carboxylic acid hydroxyamide;
(3*R*,6*S*)-4-[4-(4-fluoro-benzyloxy)-benzenesulfonyl]-2,2,6-trimethyl-morpholine-3-carboxylic acid hydroxyamide;
(2*S*,3*R*,6*S*)-6-ethyl-4-[4-(4-fluoro-benzyloxy)-benzenesulfonyl]-2-methyl-morpholine-3-carboxylic acid hydroxyamide;
(2*R*,3*R*,6*S*)-4-[4-(4-fluoro-benzyloxy)-benzenesulfonyl]-2,6-dimethyl-morpholine-3-carboxylic acid hydroxyamide;
(2*R*,3*R*,6*R*)-4-[4-(4-fluoro-benzyloxy)-benzenesulfonyl]-2,6-dimethyl-morpholine-3-carboxylic acid hydroxyamide;
(2*S*,3*R*,6*S*)-2,6-dimethyl-4-[4-(pyridin-4-ylmethoxy)-benzenesulfonyl]-morpholine-3-carboxylic acid hydroxyamide;
(2*S*,3*R*,6*S*)-2,6-dimethyl-4-[4-(pyridin-2-ylmethoxy)-benzenesulfonyl]-morpholine-3-carboxylic acid hydroxyamide;
(2*S*,3*R*,6*S*)-2,6-dimethyl-4-[4-(pyridin-3-ylmethoxy)-benzenesulfonyl]-morpholine-3-carboxylic acid hydroxyamide;
(2*S*,3*R*,6*S*)-2,6-dimethyl-4-[4-(2-methyl-pyridin-3-ylmethoxy)-benzenesulfonyl]-morpholine-3-carboxylic acid hydroxyamide;
(3*R*,6*S*)-2,2,6-trimethyl-4-[4-(2-trifluoromethyl-benzyloxy)-benzenesulfonyl]-morpholine-3-carboxylic acid hydroxyamide;
(2*S*,3*R*)-2,6,6-trimethyl-4-[4-(pyridin-4-ylmethoxy)-benzenesulfonyl]-morpholine-3-carboxylic acid hydroxyamide;
(3*R*,6*S*)-2,2,6-trimethyl-4-[4-(2-methyl-pyridin-3-ylmethoxy)-benzenesulfonyl]-morpholine-3-carboxylic acid hydroxyamide;
(2*S*,3*R*,6*S*)-[4-(2,5-dimethyl-benzyloxy)-benzenesulfonyl]-2,6-dimethyl-morpholine-3-carboxylic acid hydroxyamide;
(2*S*,3*R*,6*S*)-4-[4-(3,5-difluoro-benzyloxy)-benzenesulfonyl]-2,6-dimethyl-morpholine-3-carboxylic acid hydroxyamide;
(2*S*,3*R*,6*S*)-4-[4-(3-methoxy-benzyloxy)-benzenesulfonyl]-2,6-dimethyl-morpholine-3-carboxylic acid hydroxyamide;
(2*S*,3*R*,6*S*)-4-[4-(5-fluoro-2-methyl-benzyloxy)-benzenesulfonyl]-2,6-dimethyl-morpholine-3-carboxylic acid hydroxyamide;
(2*S*,3*R*,6*S*)-4-[4-(furan-3-ylmethoxy)-benzenesulfonyl]-2,6-dimethyl-morpholine-3-carboxylic acid hydroxyamide;
(2*S*,3*R*,6*S*)-4-[4-(2-fluoro-3-methyl-benzyloxy)-benzenesulfonyl]-2,6-dimethyl-morpholine-3-carboxylic acid hydroxyamide;
(2*S*,3*R*)-4-[4-(4-fluoro-benzyloxy)-benzenesulfonyl]-2,6,6-trimethyl-morpholine-3-carboxylic acid hydroxyamide;
(*3R*)-*4*-[4-(4-fluoro-benzyloxy)-benzenesulfonyl]-6,6-dimethyl-morpholine-3-carboxylic acid hydroxyamide;
(*3R*)-*6*,6-dimethyl-4-[4-(pyridin-4-ylmethoxy)-benzenesulfonyl]-morpholine-3-carboxylic acid hydroxyamide;
(*3R*)-*6*,6-dimethyl-4-[4-(2-methyl-benzyloxy)-benzenesulfonyl]-morpholine-3-carboxylic acid hydroxyamide;
(2*S*,3*R*,6*S*)-4-(4-cyclohexylmethoxy-benzenesulfonyl)-2,6-dimethyl-morpholine-3-carboxylic acid hydroxyamide;
(*3R,6S*)-*4*-[4-(2,5-dimethyl-benzyloxy)-benzenesulfonyl]-2,2,6-trimethyl-morpholine-3-carboxylic acid hydroxyamide;
(2*S*,3*R*)-4-[4-(4-fluoro-benzyloxy)-benzenesulfonyl]-6-methoxymethyl-2-methylmorpholine-3-carboxylic acid hydroxyamide;
(2*S*,3*R*,6*S*)-4-[4-(3-chloro-benzyloxy)-benzenesulfonyl]-6-[(ethyl-methyl-amino)-methyl]-2-methyl-morpholine-3-carboxylic acid hydroxyamide;
(2*S*,3*R*)-4-[4-(3-chloro-benzyloxy)-benzenesulfonyl]-6-methoxy-2-methyl-morpholine-3-carboxylic acid hydroxyamide;
(2*S*,3*R*,6*R*)-4-[4-(4-fluoro-benzyloxy)-benzenesulfonyl]-6-hydroxymethyl-2-methylmorpholine-3-carboxylic acid hydroxyamide.

52. A pharmaceutical composition comprising a compound of the formula (I), or a pharmaceutically acceptable salt thereof, as claimed in any one of claims 1 to 51, and a pharmaceutically acceptable carrier.

53. A compound of the formula (I), or a pharmaceutically acceptable salt thereof, as claimed in any one of claims 1 to 51, for use as a medicament.

54. The use of a compound of the formula (I), or a pharmaceutically acceptable salt thereof, as claimed in any one of claims 1 to 51, for the manufacture of a medicament for the treatment of a disease selected from arthritis, inflammatory bowel disease, Crohn's disease, emphysema, acute respiratory distress syndrome, asthma, chronic obstructive pulmonary disease, Alzheimer's disease, organ transplant toxicity, cachexia, allergic reactions, allergic contact hypersensitivity, cancer, tissue ulceration, restenosis, periodontal disease, epidermolysis bullosa, osteoporosis, loosening of artificial joint implants, atherosclerosis, aortic aneurysm, congestive heart failure, myocardial infarction, stroke, cerebral ischemia, head trauma, spinal cord injury, neuro-degenerative disorders, autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, bums, diabetes, tumor invasion, tumor growth, tumor metastasis, corneal scarring, scleritis, AIDS, sepsis and septic shock.

## Patentansprüche

1. Verbindung der Formel 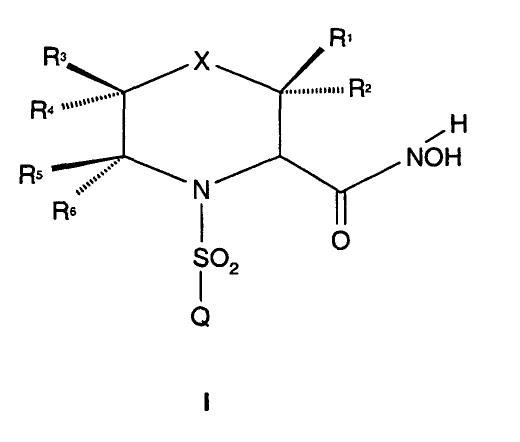 oder deren pharmazeutisch akzeptables Salz, worin
X für Sauerstoff, Schwefel, SO, SO₂ oder NR⁷ steht,
R¹, R², R³, R⁴, R⁵ und R⁶ ausgewählt sind aus Wasserstoff, Hydroxy, -CN, (C₁-C₆)Alkyl, (C₂-C₆)Alkenyl, (C₆-C₁₀)Aryl(C₂-C₆)alkenyl, (C₂-C₉)Heteroaryl(C₂-C₆)alkenyl, (C₂-C₆)Alkynyl, (C₆-C₁₀)Aryl(C₂-C₆)alkynyl, (C₂-C₉)-Heteroaryl(C₂-C₆)alkynyl, (C₁-C₆)Alkylamino, (C₁-C₆)Alkylthio, (C₁-C₆)-Alkoxy, Perfluor(C₁-C₆)alkyl, (C₆-C₁₀)Aryl, (C₂-C₉)Heteroaryl, (C₆-C₁₀)-Arylamino, (C₆-C₁₀)Arylthio, (C₆-C₁₀)Aryloxy, (C₂-C₉)Heteroarylamino, (C₂-C₉)Heteroarylthio, (C₂-C₉)Heteroaryloxy, (C₃-C₆)Cycloalkyl, (C₁-C₆)-Alkyl(hydroxymethylen), Piperidyl, (C₁-C₆)Alkylpiperidyl, (C₁-C₆)Acylamino, (C₁-C₆)Acylthio, (C₁-C₆)Acyloxy, (C₁-C₆)Alkoxy-(C=O)-, -CO₂H, (C₁-C₆)Alkyl-NH-(C=O)- und [(C₁-C₆)Alkyl]₂-N-(C=O)-;
wobei das (C₁-C₆)Alkyl optional substituiert ist durch ein oder zwei Gruppen, die ausgewählt sind aus (C₁-C₆)Alkylthio, (C₁-C₆)Alkoxy, Trifluormethyl, Halogen, -CN, (C₆-C₁₀)Aryl, (C₂-C₉)Heteroaryl, (C₆-C₁₀)-Arylamino, (C₆-C₁₀)Arylthio, (C₆-C₁₀)Aryloxy, (C₂-C₉)Heteroarylamino, (C₂-C₉)Heteroarylthio, (C₂-C₉)Heteroaryloxy, (C₆-C₁₀)Aryl(C₆-C₁₀)aryl, (C₃-C₆)Cycloalkyl, Hydroxy, Piperazinyl, (C₆-C₁₀)Aryl(C₁-C₆)alkoxy, (C₂-C₉)Heteroaryl(C₁-C₆)alkoxy, (C₁-C₆)Acylamino, (C₁-C₆)Acylthio, (C₁-C₆)-Acyloxy, (C₁-C₅)Alkylsulfinyl, (C₆-C₁₀)Arylsulfinyl, (C₁-C₆)Alkylsulfonyl, (C₆-C₁₀)Arylsulfonyl, Amino, (C₁-C₆)Alkylamino und ((C₁-C₆)Alkyl)₂amino;
wobei R⁷ für Wasserstoff, (C₁-C₆)Alkyl, das optional einfach oder mehrfach durch Hydroxy, -CN, (C₁-C₆)Alkylamino, (C₁-C₆)Alkylthio, (C₁-C₆)Alkoxy, Perfluor(C₁-C₆)alkyl, (C₆-C₁₀)Aryl, (C₆-C₁₀)Arylthio, (C₆-C₁₀)Aryloxy, (C₂-C₉)Heteroarylamino, (C₃-C₆)Cycloalkyl, (C₁-C₆)Alkyl(hydroxymethylen), Piperidyl, (C₁-C₆)Alkylpiperidyl, (C₁-C₆)Acylamino, (C₁-C₆)Acyloxy, (C₁-C₆)Alkoxy-(C=O)-, -CO₂H, (C₁-C₆)Alkyl-NH-(C=O)- oder [(C₁-C₆)Alkyl]₂-N-(C=O)- substituiert ist, oder für (C₆-C₁₀)Arylsulfonyl, (C₁-C₆)Alkylsulfonyl, (C₁-C₆)Alkyl-NH-(C=O)-, (C₁-C₆)Alkoxy-(C=O)-, (C₁-C₆)Alkyl-(C=O)-, [(C₁-C₆)Alkyl]₂-N-(C=O)- und (R⁸R⁹N)-(C=O) steht, worin R⁸ und R⁹ gemeinsam mit dem Stickstoffatom, an das sie gebunden sind, Azetidinyl, Pyrrolidinyl, Piperidinyl, Morpholinyl oder Thiomorphonyl bilden;
Q für (C₆-C₁₀)Aryl(C₁-C₆)alkoxy(C₆-C₁₀)aryl, (C₆-C₁₀)Aryl(C₁-C₆)alkoxy-(C₂-C₉)heteroaryl, (C₂-C₉)Heteroaryl(C₁-C₆)alkoxy(C₆-C₁₀)aryl oder (C₂-C₉)Heteroaryl(C₁-C₆)alkoxy(C₂-C₉)heteroaryl steht, wobei jede der genannten (C₆-C₁₀)Aryl- oder (C₂-C₉)Heteroarylgruppen optional durch einen oder mehrere Substituenten, bevorzugt einen bis drei Substituenten pro Ring, besonders bevorzugt durch einen bis drei Substituenten am terminalen Ring (d.h. dem Ring, der am weitesten von der Bindungsstelle entfernt ist), substituiert ist, wobei die Substituenten unabhängig ausgewählt sind aus Halogen, -CN, optional durch ein oder mehrere (bevorzugt 1 - 3) Fluoratome substituiertem (C₁-C₆)Alkyl, Hydroxy, Hydroxy-(C₁-C₆)alkyl, optional durch ein oder mehrere (bevorzugt 1 - 3) Fluoratome substituiertem (C₁-C₆)Alkoxy, (C₁-C₆)Alkoxy(C₁-C₆)alkyl, HO-(C=O)-, (C₁-C₆)Alkyl-O-(C=O)-, HO-(C=O)-(C₁-C₆)Alkyl, (C₁-C₆)Alkyl-O-(C=O)-(C₁-C₆)alkyl, (C₁-C₆)Alkyl-(C=O)-O-, (C₁-C₆)Alkyl-(C=O)-O-(C₁-C₆)alkyl, H(O=C)-, H(O=C)-(C₁-C₆)Alkyl, (C₁-C₆)Alkyl(O=C)-, (C₁-C₆)Alkyl(O=C)-(C₁-C₆)alkyl, NO₂, Amino, (C₁-C₆)Alkylamino, [(C₁-C₆)Alkyl]₂amino, Amino(C₁-C₆)alkyl, (C₁-C₆)Alkylamino(C₁-C₆)alkyl, [(C₁-C₆)Alkyl]₂amino(C₁-C₆)alkyl, H₂N-(C=O)-, (C₁-C₆)Alkyl-NH-(C=O)-, [(C₁-C₆)Alkyl]₂N-(C=O)-, H₂N(C=O)-(C₁-C₆)Alkyl, (C₁-C₆)Alkyl-HN(C=O)-(C₁-C₆)alkyl, [(C₁-C₆)Alkyl]₂N-(C=O)-(C₁-C₆)alkyl, H(O=C)-NH-, (C₁-C₆)-Alkyl(C=O)-NH, (C₁-C₆)Alkyl(C=O)-[NH](C₁-C₆)alkyl, (C₁-C₆)Alkyl(C=O)-[N(C₁-C₆)alkyl](C₁-C₆)alkyl, (C₁-C₆)Alkyl-S-, (C₁-C₆)Alkyl-(S=O)-, (C₁-C₆)Alkyl-SO₂-, (C₁-C₆)Alkyl-SO₂-NH-, (C₁-C₆)Alkyl-SO₂-[N(C₁-C₆)alkyl]-, H₂N-SO₂-, H₂N-SO₂-(C₁-C₆)Alkyl, (C₁-C₆)Alkyl-HN-SO₂-(C₁-C₆)alkyl, [(C₁-C₆)Alkyl]₂N-SO₂-(C₁-C₆)alkyl, CF₃SO₃-, (C₁-C₆)Alkyl-SO₃-, Phenyl, Phenyl(C₁-C₆)alkyl, (C₃-C₁₀)Cycloalkyl, (C₂-C₉)Heterocycloalkyl und (C₂-C₉)Heteroaryl;
unter der Bedingung, dass, wenn X für SO oder SO₂ steht und R³ und R⁴ einen ein Heteroatom enthaltenden Rest darstellen, das Heteroatom nicht an den Ring gebunden sein kann;
und unter der Bedingung, dass wenigstens einer der Reste R¹ bis R⁶ für (C₁-C₆)Alkyl stehen muss;
sowie unter der Bedingung, dass, wenn X für Sauerstoff oder Schwefel steht und R³ bis R⁶ Wasserstoff bedeuten, R¹ und R² nicht beide Methyl sein können.

2. Verbindung nach Anspruch 1, worin X für NR⁷, Schwefel oder Sauerstoff steht.

3. Verbindung nach Anspruch 1, worin Q für (C₆-C₁₀)Aryl(C₁-C₆)alkoxy(C₆-C₁₀)aryl, (C₆-C₁₀)Aryl(C₁-C₆)alkoxy(C₂-C₉)heteroaryl, (C₂-C₉)Heteroaryl(C₁-C₆)-alkoxy(C₆-C₁₀)aryl oder (C₂-C₉)Heteroaryl(C₁-C₆)alkoxy(C₂-C₉)heteroaryl steht, die optional substituiert sind durch einen oder mehrere Substituenten, bevorzugt einen bis drei Substituenten pro Ring, besonders bevorzugt durch einen bis drei Substituenten am terminalen Ring, wobei der Substituent ausgewählt ist aus Halogen, (C₁-C₆)Alkyl, (C₁-C₆)Alkoxy und Perfluor-(C₁-C₃)alkyl.

4. Verbindung nach Anspruch 1, worin Q für (C₆-C₁₀)Arylmethoxy(C₆-C₁₀)aryl, (C₆-C₁₀)Arylmethoxy(C₂-C₉)heteroaryl, (C₂-C₉)Heteroarylmethoxy(C₆-C₁₀)aryl oder (C₂-C₉)Heteroarylmethoxy(C₂-C₉)heteroaryl steht, die optional substituiert sind durch einen oder mehrere Substituenten, bevorzugt einen bis drei Substituenten pro Ring, besonders bevorzugt durch einen bis drei Substituenten am terminalen Ring, wobei die Substituenten ausgewählt sind aus Halogen, (C₁-C₆)Alkyl, (C₁-C₆)Alkoxy und Perfluor-(C₁-C₃)alkyl.

5. Verbindung nach Anspruch 1, worin Q für optional substituiertes (C₁-C₆)Arylmethoxyphenyl, Pyridylmethoxyphenyl, Furylmethoxyphenyl, Pyroylmethoxyphenyl, Thienylmethoxyphenyl, Isothiazolylmethoxyphenyl, Imidazolylmethoxyphenyl, Benzimidazolylmethoxyphenyl, Tetrazolylmethoxyphenyl, Pyrazinylmethoxyphenyl, Pyrimidinylmethoxyphenyl, Chinolylmethoxyphenyl, Isochinolylmethoxyphenyl, Benzofurylmethoxyphenyl, Isobenzofurylmethoxyphenyl, Benzothienylmethoxyphenyl, Pyrazolylmethoxyphenyl, Indolylmethoxyphenyl, Isoindolylmethoxyphenyl, Purinylmethoxyphenyl, Carbazolylmethoxyphenyl, Isoxalolylmethoxyphenyl, Thiazolylmethoxyphenyl, Oxazolylmethoxyphenyl, Benzthiazolylmethoxyphenyl oder Benzoxazolylmethoxyphenyl steht.

6. Verbindung nach Anspruch 1, worin Q für (C₆-C₁₀)Arylmethoxy(C₆)aryl steht, das optional durch einen oder mehrere, bevorzugt einen bis drei Substituenten pro Ring, besonders bevorzugt einen bis drei Substituenten am terminalen Ring substituiert ist, wobei die Substituenten unabhängig ausgewählt sind aus Halogen, (C₁-C₆)Alkyl, (C₁-C₆)Alkoxy und Perfluor(C₁-C₃)alkyl.

7. Verbindung nach Anspruch 1, worin Q für (C₆-C₁₀)Arylmethoxy(C₂-C₉)heteroaryl steht, das optional durch einen oder mehrere, bevorzugt einen bis drei Substituenten pro Ring, besonders bevorzugt einen bis drei Substituenten am terminalen Ring substituiert ist, wobei die Substituenten unabhängig ausgewählt sind aus Halogen, (C₁-C₆)Alkyl, (C₁-C₆)Alkoxy und Perfluor(C₁-C₃)alkyl.

8. Verbindung nach Anspruch 1, worin Q für (C₂-C₉)Heteroarylmethoxy(C₆)aryl steht, das optional durch einen oder mehrere, bevorzugt einen bis drei Substituenten pro Ring, besonders bevorzugt einen bis drei Substituenten am terminalen Ring substituiert ist, wobei die Substituenten unabhängig ausgewählt sind aus Halogen, (C₁-C₆)Alkyl, (C₁-C₆)Alkoxy und Perfluor(C₁-C₃)alkyl.

9. Verbindung nach Anspruch 1, worin Q für (C₂-C₉)Heteroarylmethoxy(C₂-C₉)-heteroaryl steht, das optional durch einen oder mehrere, bevorzugt einen bis drei Substituenten pro Ring, besonders bevorzugt einen bis drei Substituenten am terminalen Ring substituiert ist, wobei die Substituenten unabhängig ausgewählt sind aus Halogen, (C₁-C₆)Alkyl, (C₁-C₆)Alkoxy und Perfluor(C₁-C₃)Alkyl.

10. Verbindung nach Anspruch 1, worin R⁴ für Wasserstoff steht.

11. Verbindung nach Anspruch 1, worin R² oder R³ für Wasserstoff steht.

12. Verbindung nach Anspruch 1, worin mindestens einer der Reste R² und R³ von Wasserstoff verschieden ist.

13. Verbindung nach Anspruch 1, worin mindestens einer der Reste R¹ bis R³ für (C₁-C₆)Alkyl steht.

14. Verbindung nach Anspruch 2, worin mindestens einer der Reste R¹ bis R³ für (C₁-C₆)Alkyl steht.

15. Verbindung nach Anspruch 3, worin mindestens einer der Reste R¹ bis R³ für (C₁-C₆)Alkyl steht.

16. Verbindung nach Anspruch 1, worin mindestens einer der Reste R¹ bis R³ für Methyl steht.

17. Verbindung nach Anspruch 2, worin mindestens einer der Reste R¹ bis R³ für Methyl steht.

18. Verbindung nach Anspruch 3, worin mindestens einer der Reste R¹ bis R³ für Methyl steht.

19. Verbindung nach Anspruch 3, worin R¹ für (C₁-C₆)Alkyl steht.

20. Verbindung nach Anspruch 3, worin R² für (C₁-C₆)Alkyl steht.

21. Verbindung nach Anspruch 3, worin R³ für (C₁-C₆)Alkyl steht.

22. Verbindung nach Anspruch 3, worin R⁴ für (C₁-C₆)Alkyl steht.

23. Verbindung nach Anspruch 1, worin R¹ und R⁴ jeweils für Methyl stehen.

24. Verbindung nach Anspruch 2, worin R¹ und R⁴ jeweils für Methyl stehen.

25. Verbindung nach Anspruch 3, worin R¹ und R⁴ jeweils für Methyl stehen.

26. Verbindung nach Anspruch 1, worin R¹ und R² jeweils für (C₁-C₆)Alkyl stehen.

27. Verbindung nach Anspruch 2, worin R¹ und R² jeweils für (C₁-C₆)Alkyl stehen.

28. Verbindung nach Anspruch 3, worin R¹ und R² jeweils für (C₁-C₆)Alkyl stehen.

29. Verbindung nach Anspruch 1, worin R¹ und R² jeweils für Methyl stehen.

30. Verbindung nach Anspruch 2, worin R¹ und R² jeweils für Methyl stehen.

31. Verbindung nach Anspruch 3, worin R¹ und R² jeweils für Methyl stehen.

32. Verbindung nach Anspruch 1, worin R¹ für Methyl und R² für Wasserstoff steht.

33. Verbindung nach Anspruch 1, worin R¹ für Wasserstoff und R³ für Methyl steht.

34. Verbindung nach Anspruch 3, worin R¹ und R³ jeweils für Methyl stehen.

35. Verbindung nach Anspruch 3, worin R¹ und R⁴ jeweils für Methyl stehen.

36. Verbindung nach Anspruch 3, worin R² und R³ jeweils für Methyl stehen.

37. Verbindung nach Anspruch 3, worin R² und R⁴ jeweils für Methyl stehen.

38. Verbindung nach Anspruch 3, worin R³ und R⁴ jeweils für Methyl stehen.

39. Verbindung nach Anspruch 1, worin X für NR⁷ steht.

40. Verbindung nach Anspruch 3, worin X für NR⁷ steht.

41. Verbindung nach Anspruch 4, worin X für NR⁷ steht.

42. Verbindung nach Anspruch 5, worin X für NR⁷ steht.

43. Verbindung nach Anspruch 6, worin X für NR⁷ steht.

44. Verbindung nach Anspruch 13, worin X für NR⁷ steht.

45. Verbindung nach Anspruch 15, worin X für NR⁷ steht.

46. Verbindung nach Anspruch 1, worin X für Stickstoff und R⁷ für (C₁-C₆)Alkyl steht.

47. Verbindung nach Anspruch 1, worin X für Stickstoff und R⁷ für (C₁-C₆)Alkylsulfonyl steht.

48. Verbindung nach Anspruch 1, worin X für Stickstoff und R⁷ für (C₁-C₆)Arylsulfonyl steht.

49. Verbindung nach Anspruch 1, worin X für Stickstoff und R⁷ für [(C₁-C₆)Alkyl]₂-N-(C=O)- oder (C₁-C₆)AlkylNH-(C=O)- steht.

50. Verbindung nach Anspruch 1, worin X für Stickstoff und R⁷ für (C₁-C₆)Alkyl-(C=O)- steht.

51. Verbindung nach Anspruch 1, ausgewählt aus:
(2S,3S)-4-[4-(3,5-Difluor-benzyloxy)-benzolsulfonyl]-2-methyl-thiomorpholin-3-carbonsäure-Hydroxyamid;
(2S,3S)-4-[4-(4-Fluor-benzyloxy)-benzolsulfonyl]-2-methyl-thiomorpholin-3-carbonsäure-Hydroxyamid;
(2S,3*R*,6S)-2,6-Dimethyl-4-[4-(2-methyl-benzyloxy)-benzolsulfonyl]-morpholin-3-carbonsäure-Hydroxyamid;
4-(4-Benzyloxy-benzolsulfonyl)-2-methyl-morpholin-3-carbonsäure-Hydroxyamid;
(2S,3*R*,6S)-4-[4-(4-Fluor-benzyloxy)-benzolsulfonyl]-2,6-dimethyl-morpholin-3-carbonsäure-Hydroxyamid;
(3*R*,6S)-4-[4-(4-Fluor-benzyloxy)-benzolsulfonyl]-2,2,6-trimethyl-morpholin-3-carbonsäure-Hydroxyamid;
(2S,3*R*,6S)-6-Ethyl-4-[4-(4-fluor-benzyloxy)-benzolsulfonyl]-2-methyl-morpholin-3-carbonsäure-Hydroxyamid;
(2*R*,3*R*,6S)-4-[4-(4-Fluor-benzyloxy)-benzolsulfonyl]-2,6-dimethyl-morpholin-3-carbonsäure-Hydroxyamid;
(*2R*,3*R*,6*R*)-4-[4-(4-Fluor-benzyloxy)-benzolsulfonyl]-2,6-dimethyl-morpholin-3-carbonsäure-Hydroxyamid;
(2S,3*R*,6S)-2,6-Dimethyl-4-[4-(pyridin-4-ylmethoxy)-benzolsulfonyl]-morpholin-3-carbonsäure-Hydroxyamid;
(2S,3*R*,6S)-2,6-Dimethyl-4-[4-(pyridin-2-ylmethoxy)-benzolsulfonyl]-morpholin-3-carbonsäure-Hydroxyamid;
(2S,3*R*,6S)-2,6-Dimethyl-4-[4-(pyridin-3-ylmethoxy)-benzolsulfonyl]-morpholin-3-carbonsäure-Hydroxyamid;
(2S,3*R*,6S)-2,6-Dimethyl-4-[4-(2-methyl-pyridin-3-ylmethoxy)-benzolsulfonyl]-morpholin-3-carbonsäure-Hydroxyamid;
(3*R*,6S)-2,2,6-Trimethyl-4-[4-(2-trifluormethyl-benzyloxy)-benzolsulfonyl]-morpholin-3-carbonsäure-Hydroxyamid;
(2S,3*R*)-2,6,6-Trimethyl-4-[4-(pyridin-4-ylmethoxy)-benzolsulfonyl]-morpholin-3-carbonsäure-Hydroxyamid;
(3*R*,6S)-2,2,6-Trimethyl-4-[4-(2-methyl-pyridin-3-ylmethoxy)-benzolsulfonyl]-morpholin-3-carbonsäure-Hydroxyamid;
(2S,3*R*,6S)-[4-(2,5-Dimethyl-benzyloxy)-benzolsulfonyl]-2,6-dimethyl-morpholin-3-carbonsäure-Hydroxyamid;
(2S,3*R*,6S)-4-[4-(3,5-Difluor-benzyloxy)-benzolsulfonyl]-2,6-dimethyl-morpholin-3-carbonsäure-Hydroxyamid;
(2S,3*R*,6S)-4-[4-(3-Methoxy-benzyloxy)-benzolsulfonyl]-2,6-dimethyl-morpholin-3-carbonsäure-Hydroxyamid;
(2S,3*R*,6S)-4-[4-(5-Fluor-2-methyl-benzyloxy)-benzolsulfonyl]-2,6-dimethyl-morpholin-3-carbonsäure-Hydroxyamid;
(2S,3*R*,6S)-4-[4-(Furan-3-ylmethoxy)-benzolsulfonyl]-2,6-dimethyl-morpholin-3-carbonsäure-Hydroxyamid;
(2S,3*R*,6S)-4-[4-(2-Fluor-3-methyl-benzyloxy)-benzolsulfonyl]-2,6-dimethyl-morpholin-3-carbonsäure-Hydroxyamid;
(2S,3*R*)-4-[4-(4-Fluor-benzyloxy)-benzolsulfonyl]-2,6,6-trimethyl-morpholin-3-carbonsäure-Hydroxyamid;
(3*R*)-4-[4-(4-Fluor-benzyloxy)-benzolsulfonyl]-6,6-dimethyl-morpholin-3-carbonsäure-Hydroxyamid;
(3*R*)-6,6-Dimethyl-4-[4-(pyridin-4-ylmethoxy)-benzolsulfonyl]-morpholin-3-carbonsäure-Hydroxyamid;
(3*R*)-6,6-Dimethyl-4-[4-(2-methyl-benzyloxy)-benzolsulfonyl]-morpholin-3-carbonsäure-Hydroxyamid;
(2S,3*R*,6S)-4-(4-Cyclohexylmethoxy-benzolsulfonyl)-2,6-dimethyl-morpholin-3-carbonsäure-Hydroxyamid;
(3*R*,6S)-4-[4-(2,5-Dimethyl-benzyloxy)-benzolsulfonyl]-2,2,6-trimethyl-morpholin-3-carbonsäure-Hydroxyamid;
(2S,3*R*)-4-[4-(4-Fluor-benzyloxy)-benzolsulfonyl]-6-methoxymethyl-2-methylmorpholin-3-carbonsäure-Hydroxyamid;
(2S,3*R*,6S)-4-[4-(3-Chlor-benzyloxy)-benzolsulfonyl]-6-[(ethyl-methyl-amino)-methyl]-2-methyl-morpholin-3-carbonsäure-Hydroxyamid;
(2S,3*R*)-4-[4-(3-Chlor-benzyloxy)-benzolsulfonyl]-6-methoxy-2-methyl-morpholin-3-carbonsäure-Hydroxyamid;
(2S,3*R*,6*R*)-4-[4-(4-Fluor-benzyloxy)-benzolsulfonyl]-6-hydroxymethyl-2-methylmorpholin-3-carbonsäure-Hydroxyamid.

52. Pharmazeutische Komposition, enthaltend eine Verbindung der Formel I oder deren pharmazeutisch akzeptables Salz gemäß einem der Ansprüche 1 bis 51, und einen pharmazeutisch akzeptablenen Träger.

53. Verbindung der Formel I oder deren pharmazeutisch akzeptables Salz gemäß einem der Ansprüche 1 bis 51 zur Verwendung als Arzneimittel.

54. Verwendung einer Verbindung der Formel I oder deren pharmazeutisch akzeptablenen Salzes gemäß einem der Ansprüche 1 bis 51 zur Herstellung eines Arzneimittels zur Behandlung einer Krankheit, die ausgewählt ist aus Arthritis, entzündlichen Darmerkrankungen, Morbus Crohn, Emphysem, akutem Lungenversagen (ARDS), Asthma, chronischer obstruktiver Lungenerkrankung (COPD), Alzheimer, Toxizität nach Organtransplantation, Cachexie, allergischen Reaktionen, allergischer Kontakthypersensibilität, Krebs, Geschwürbildung in Geweben, Restenose, Parodontitis, Epidermolysis bullosa, Osteoporose, Lockerung künstlicher Gelenkimplantate, Atherosklerose, Aorten-Aneurysma, dekompensierter Herzinsuffizienz, Myocardinfarkt, Schlaganfall, zerebraler Ischämie, Schädelverletzungen, Verletzungen des Rückenmarks, neurodegenerativen Störungen, Autoimmunkrankheiten, Chorea Huntington, Parkinson, Migräne, Depression, peripherer Neuropathie, Schmerzen, zerebraler Amyloidangiopathie (CAA), nootropem Enhancement und Cognition Enhancement, amyotropher Lateralsklerose, multipler Sklerose, okulärer Angiogenesis, Verletzungen der Hornhaut, Degeneration der Macula, abnormaler Wundheilung, Verbrennungen, Diabetes, Tumorinvasion, Tumorwachstum, Tumormetastasen, Vernarbung der Hornhaut, Scleritis, AIDS, Sepsis und septischem Schock.

## Revendications

1. Composé de formule 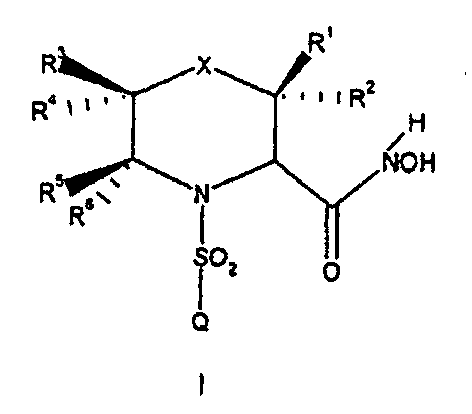 ou son sel pharmaceutiquement acceptable, formule dans laquelle
X représente un atome d'oxygène ou de soufre ou un groupe SO, SO₂ ou NR⁷ ;
R¹, R², R³, R⁴, R⁵ et R⁸ sont choisis dans le groupe consistant en un atome d'hydrogène, des groupes hydroxy, -CN, alkyle en C₁ à C₆, alcényle en C₂ à C₆, (aryle en C₆ à C₁₀)(alcényle en C₂ à C₆), (hétéroaryle en C₂ à C₉) (alcényle en C₂ à C₆), alcynyle en C₂ à C₆, (aryle en C₆ à C₁₀)(alcynyle en C₂ à C₆), (hétéroaryle en C₂ à C₉)(alcynyle en C₂ à C₆), alkylamino en C₁ à C₆, alkylthio en C₁ à C₆, alkoxy en C₁ à C₆, perfluoralkyle en C₁ à C₆, aryle en C₆ à C₁₀, hétéroaryle en C₂ à C₉, arylamino en C₆ à C₁₀, arylthio en C₆ à C₁₀, aryloxy en C₆ à C₁₀, hétéroarylamino en C₂ à C₉, hétéroarylthio en C₂ à C₉, hétéroaryloxy en C₂ à C₉, cycloalkyle en C₃ à C₆, (alkyle en C₁ à C₆)(hydroxyméthylène), pipéridyle, (alkyle en C₁ à C₆)pipéridyle, acylamino en C₁ à C₆, acylthio en C₁ à C₆, acyloxy en C₁ à C₆, (alkoxy en C₁ à C₆)(C=O)-, -CO₂H, (alkyle en C₁ à C₆)-NH-(C=O)- et [(alkyle en C₁ à C₆)]₂-N-(C=O)-,
ledit groupe alkyle en C₁ à C₆ étant facultativement substitué avec un ou deux groupes choisis entre des groupes alkylthio en C₁ à C₆, alkoxy en C₁ à C₆, trifluorométhyle, halogéno, -CN, aryle en C₆ à C₁₀, hétéroaryle en C₂ à C₉, arylamino en C₆ à C₁₀, arylthio en C₆ à C₁₀, aryloxy en C₆ à C₁₀, hétéroarylamino en C₂ à C₉, hétéroarylthio en C₂ à C₉, hétéroaryloxy en C₂ à C₉, (aryle en C₆ à C₁₀)(aryle en C₆ à C₁₀), cycloalkyle en C₃ à C₆, hydroxy, pipérazinyle, (aryle en C₆ à C₁₀)(alkoxy en C₁ à C₆), (hétéroaryle en C₂ à C₉)(alkoxy en C₁ à C₆), acylamino en C₁ à C₆, acylthio en C₁ à C₆, acyloxy en C₁ à C₆, alkylsulfinyle en C₁ à C₆, arylsulfinyle en C₆ à C₁₀, alkylsulfonyle en C₁ à C₆, arylsulfonyle en C₆ à C₁₀, amino, alkylamino en C₁ à C₆ et (alkyle en C₁ à C₆)₂amino ;
R⁷ représente un atome d'hydrogène ou un groupe alkyle en C₁ à C₆ facultativement substitué avec un ou plusieurs substituants hydroxy -CN, alkylamino en C₁ à C₆, alkylthio en C₁ à C₆, alkoxy en C₁ à C₆, perfluoralkyle en C₁ à C₆, aryle en C₆ à C₁₀, arylthio en C₆ à C₁₀, aryloxy en C₆ à C₁₀, hétéroarylamino en C₂ à C₉, cycloalkyle en C₃ à C₆, (alkyle en C₁ à C₆)(hydroxyméthylène), pipéridyle, (alkyle en C₁ à C₆)pipéridyle, acylamino en C₁ à C₆, acyloxy en C₁ à C₆, (alkoxy en C₁ à C₆)-(C=O)-, -CO₂H, (alkyle en C₁ à C₆)-NH-(C=O)- et [(alkyle en C₁ à C₆)]₂-N-(C=O)-, arylsulfonyle en C₆ à C₁₀, alkylsulfonyle en C₁ à C₆, (alkyle en C₁ à C₆)-NH-(C=O)-, (alkoxy en C₁ à C₆)-(C=O)-, (alkyle en C₁ à C₆)-(C=O)-, [(alkyle en C₁ à C₆)]₂-N-(C=O)-, (R⁸R⁹N)-(C=O) dans lequel R⁸ et R⁹ sont pris conjointement avec l'atome d'azote auquel ils sont fixés pour former un groupe azétidinyle, pyrrolidinyle, pipéridinyle, morpholinyle ou thiomorpholinyle ;
Q représente un groupe (aryle en C₆ à C₁₀)(alkoxy en C₁ à C₆) (aryle en C₆ à C₁₀), (aryle en C₆ à C₁₀)(alkoxy en C₁ à C₆)(hétéroaryle en C₂ à C₉), (hétéroaryle en C₂ à C₉)(alkoxy en C₁ à C₆)(aryle en C₆ à C₁₀) ou (hétéroaryle en C₂ à C₉)(alkoxy en C₁ à C₆)(hétéroaryle en C₂ à C₉), dans lequel chacun desdits groupes aryle en C₆ à C₁₀ ou hétéroaryle en C₂ à C₉ peut être facultativement substitué avec un ou plusieurs substituants, avantageusement avec un à trois substituants par noyau, de préférence un à trois substituants sur le noyau terminal (c'est-à-dire le noyau le plus éloigné du point de fixation) choisis indépendamment dans le groupe consistant en des substituants halogéno, -CN, alkyle en C₁ à C₆ facultativement substitué avec un ou plusieurs atomes de fluor (de préférence un à trois atomes de fluor), hydroxy, hydroxyalkyle en C₁ à C₆, alkoxy en C₁ à C₆ facultativement substitué avec ou plusieurs atomes de fluor (de préférence un à trois atomes de fluor), (alkoxy en C₁ à C₆) (alkyle en C₁ à C₆), HO-(C=O)-, (alkyle en C₁ à C₆)-O-(C=O), HO-(C=O)-(alkyle en C₁ à C₆), (alkyle en C₁ à C₆)-O-(C=O) (alkyle en C₁ à C₆), (alkyle en C₁ à C₆)-(C=O)-, (alkyle en C₁ à C₆)-(C=O)-O-(alkyle en C₁ à C₆), H(O=C)-, H(O=C)(alkyle en C₁ à C₆), (alkyle en C₁ à C₆)-(O=C)-, (alkyle en C₁ à C₆)-(O=C)-(alkyle en C₁ à C₆), NO₂, amino, alkylamino en C₁ à C₆, [(alkyle en C₁ à C₆)]₂amino, aminoalkyle en C₁ à C₆, (alkyle en C₁ à C₆)amino(alkyle en C₁ à C₆), [(alkyle en C₁ à C₆)]₂amino (alkyle en C₁ à C₆), H₂N-(C=O)-, (alkyle en C₁ à C₆)-NH-(C=O)-, [(alkyle en C₁ à C₆)]₂-N-(C=O)-, H₂N(C=O) (alkyle en C₁ à C₆), (alkyle en C₁ à C₆)-HN(C=O)-(alkyle en C₁ à C₆), [(alkyle en C₁ à C₆)]₂N(C=O) (alkyle en C₁ à C₆), H(O=C)-NH-, (alkyle en C₁ à C₆)-(C=O)-NH, (alkyle en C₁ à C₆)(C=O)-NH, (alkyle en C₁ à C₆) (C=O)-[NH] (alkyle en C₁ à C₆), (alkyle en C₁ à C₆) (C=O)-[N(alkyle en C₁ à C₆)](alkyle en C₁ à C₆), (alkyle en C₁ à C₆)-S-, (alkyle en C₁ à C₆)-(S=O)-, (alkyle en C₁ à C₆)-SO₂-, (alkyle en C₁ à C₆)-SO₂-NH-, (alkyle en C₁ à C₆)-SO₂-[N(alkyle en C₁ à C₆)]-, H₂N-SO₂-, H₂N-SO₂(alkyle en C₁ à C₆), (alkyle en C₁ à C₆)HN-SO₂(alkyle en C₁ à C₆), [(alkyle en C₁ à C₆)]₂N-SO₂(alkyle en C₁ à C₆), CF₃SO₃-, (alkyle en C₁ à C₆)-SO₃-, phényle, phényl(alkyle en C₁ à C₆), cycloalkyle en C₃ à C₁₀, hétérocycloalkyle en C₂ à C₉ et hétéroaryle en C₂ à C₉ ;
sous réserve que, lorsque X représente un groupe SO ou SO₂ et R³ et R⁴ représentent un substituant comprenant un hétéroatome, l'hétéroatome ne puisse être lié au noyau ;
et sous réserve qu'au moins un des groupes R¹ à R⁶ représente un groupe alkyle en C₁ à C₆ ;
et sous réserve que, lorsque X représente un atome d'oxygène ou de soufre et R³ et R⁶ représentent chacun un atome d'hydrogène, alors R¹ et R² ne puisse représenter l'un et l'autre un groupe méthyle.

2. Composé suivant la revendication 1, dans lequel X représente un groupe NR⁷ ou un atome de soufre ou d'oxygène.

3. Composé suivant la revendication 1, dans lequel Q représente un groupe (aryle en C₆ à C₁₀)(alkoxy en C₁ à C₆)(aryle en C₆ à C₁₀), (aryle en C₆ à C₁₀)(alkoxy en C₁ à C₆)(hétéroaryle en C₂ à C₉), (hétéroaryle en C₂ à C₉)(alkoxy en C₁ à C₆)(aryle en C₆ à C₁₀) ou (hétéroaryle en C₂ à C₉)(alkoxy en C₁ à C₆) (hétéroaryle en C₂ à C₉), facultativement substitué avec un ou plusieurs, avantageusement un à trois substituants par noyau, de préférence un à trois substituants sur le noyau terminal, ledit substituant étant choisi indépendamment entre des substituants halogéno, alkyle en C₁ à C₆, alkoxy en C₁ à C₆ et perfluoralkyle en C₁ à C₃.

4. Composé suivant la revendication 1, dans lequel Q représente un groupe (aryle en C₁ à C₁₀)méthoxy(aryle en C₆ à C₁₀), (aryle en C₆ à C₁₀)méthoxy(hétéroaryle en C₂ à C₉), (hétéroaryle en C₂ à C₉)méthoxy(aryle en C₆ à C₁₀) ou (hétéroaryle en C₂ à C₉)méthoxy(hétéroaryle en C₂ à C₉) facultativement substitué avec un ou plusieurs, avantageusement un à trois substituants par noyau, de préférence un à trois substituants sur le noyau terminal, ledit substituant étant choisi entre des substituants halogéno, alkyle en C₁ à C₆, alkoxy en C₁ à C₆ et perfluoralkyle en C₁ à C₃.

5. Composé suivant la revendication 1, dans lequel Q représente un groupe, facultativement substitué, (aryle en C₆ à C₁₀)méthoxyphényle, pyridylméthoxyphényle, furyl-méthoxyphényle, pyroylméthoxyphényle, thiénylméthoxyphényle, isothiazolylméthoxyphényle, imidazolylméthoxyphényle, benzimidazolylméthoxyphényle, tétrazolylméthoxyphényle, pirazinylméthoxyphényle, pyrimidylméthoxyphényle, quinolylméthoxyphényle, isoquinolylméthoxyphényle, benzofurylméthoxyphényle, isobenzofurylméthoxyphényle, benzothiénylméthoxyphényle, pyrazolylméthoxyphényle, indolylméthoxyphényle, isoindolylméthoxyphényle, purinylméthoxyphényle, carbazolylméthoxyphényle, isoxazolylméthoxyphényle, thiazolylméthoxyphényle, oxazolylméthoxyphényle, benzthiazolylméthoxyphényle, benzoxazolylméthoxyphényle,

6. Composé suivant la revendication 1, dans lequel Q représente un groupe (aryle en C₁ à C₆)méthoxy(aryle en C₆) facultativement substitué avec un ou plusieurs, avantageusement avec un à trois substituants par noyau, de préférence un à trois substituants sur le noyau terminal, ledit substituant étant choisi indépendamment entre des substituants halogéno, alkyle en C₁ à C₆, alkoxy en C₁ à C₆ et perfluoralkyle en C₁ à C₃.

7. Composé suivant la revendication 1, dans lequel Q représente un groupe (aryle en C₁ à C₆)méthoxy(hétéroaryle en C₂ à C₉) facultativement substitué avec un ou plusieurs, avantageusement avec un à trois substituants par noyau, de préférence un à trois substituants sur le noyau terminal, ledit substituant étant choisi indépendamment entre des substituants halogéno, alkyle en C₁ à C₆, alkoxy en C₁ à C₆ et perfluoralkyle en C₁ à C₃.

8. Composé suivant la revendication 1, dans lequel Q représente un groupe (hétéroaryle en C₂ à C₉)méthoxy(aryle en C₆) facultativement substitué avec un ou plusieurs, avantageusement avec un à trois substituants par noyau, de préférence un à trois substituants sur le noyau terminal, lesdits substituants étant choisis indépendamment entre des substituants halogéno, alkyle en C₁ à C₆, alkoxy en C₁ à C₆ et perfluoralkyle en C₁ à C₃.

9. Composé suivant la revendication 1, dans lequel Q représente un groupe (hétéroaryle en C₂ à C₉)méthoxy(hétéroaryle en C₂ à C₉) facultativement substitué avec un ou plusieurs, avantageusement avec un à trois substituants par noyau, de préférence un à trois substituants sur le noyau terminal, lesdits substituants étant choisis indépendamment entre des substituants halogéno, alkyle en C₁ à C₆, alkoxy en C₁ à C₆ et perfluoralkyle en C₁ à C₃.

10. Composé suivant la revendication 1, dans lequel R⁴ représente un atome d'hydrogène.

11. Composé suivant la revendication 1, dans lequel R² ou R³ représente un atome d'hydrogène.

12. Composé suivant la revendication 1, dans lequel au moins un des groupes R² et R³ est autre qu'un atome d'hydrogène.

13. Composé suivant la revendication 1, dans lequel au moins un des groupes R¹ à R³ représente un groupe alkyle en C₁ à C₆.

14. Composé suivant la revendication 2, dans lequel au moins un des groupes R¹ à R³ représente un groupe alkyle en C₁ à C₆.

15. Composé suivant la revendication 3, dans lequel au moins un des groupes R¹ à R³ représente un groupe alkyle en C₁ à C₆.

16. Composé suivant la revendication 1, dans lequel au moins un des groupes R¹ à R³ représente un groupe méthyle.

17. Composé suivant la revendication 2, dans lequel au moins un des groupes R¹ à R³ représente un groupe méthyle.

18. Composé suivant la revendication 3, dans lequel au moins un des groupes R¹ à R³ représente un groupe méthyle.

19. Composé suivant la revendication 3, dans lequel R¹ représente un groupe alkyle en C₁ à C₆.

20. Composé suivant la revendication 3, dans lequel R² représente un groupe alkyle en C₁ à C₆.

21. Composé suivant la revendication 3, dans lequel R³ représente un groupe alkyle en C₁ à C₆.

22. Composé suivant la revendication 3, dans lequel R⁴ représente un groupe alkyle en C₁ à C₆.

23. Composé suivant la revendication 1, dans lequel R¹ et R⁴ représentent chacun un groupe méthyle.

24. Composé suivant la revendication 2, dans lequel R¹ et R⁴ représentent chacun un groupe méthyle.

25. Composé suivant la revendication 3, dans lequel R¹ et R⁴ représentent chacun un groupe méthyle.

26. Composé suivant la revendication 1, dans lequel R¹ et R² représentent chacun un groupe alkyle en C₁ à C₆.

27. Composé suivant la revendication 2, dans lequel R¹ et R² représentent chacun un groupe alkyle en C₁ à C₆.

28. Composé suivant la revendication 3, dans lequel R¹ et R² représentent chacun un groupe alkyle en C₁ à C₆.

29. Composé suivant la revendication 1, dans lequel R¹ et R² représentent chacun un groupe méthyle.

30. Composé suivant la revendication 2, dans lequel R¹ et R² représentent chacun un groupe méthyle.

31. Composé suivant la revendication 3, dans lequel R¹ et R² représentent chacun un groupe méthyle.

32. Composé suivant la revendication 1, dans lequel R¹ représente un groupe méthyle et R² représente un atome d'hydrogène.

33. Composé suivant la revendication 1, dans lequel R¹ représente un atome d'hydrogène et R³ représente un groupe méthyle.

34. Composé suivant la revendication 3, dans lequel R¹ et R³ représentent chacun un groupe méthyle.

35. Composé suivant la revendication 3, dans lequel R¹ et R⁴ représentent chacun un groupe méthyle.

36. Composé suivant la revendication 3, dans lequel R² et R³ représentent chacun un groupe méthyle.

37. Composé suivant la revendication 3, dans lequel R² et R⁴ représentent chacun un groupe méthyle.

38. Composé suivant la revendication 3, dans lequel R³ et R⁴ représentent chacun un groupe méthyle.

39. Composé suivant la revendication 1, dans lequel X représente un groupe NR⁷.

40. Composé suivant la revendication 3, dans lequel X représente un groupe NR⁷.

41. Composé suivant la revendication 4, dans lequel X représente un groupe NR⁷.

42. Composé suivant la revendication 5, dans lequel X représente un groupe NR⁷.

43. Composé suivant la revendication 6, dans lequel X représente un groupe NR⁷.

44. Composé suivant la revendication 13, dans lequel X représente un groupe NR⁷.

45. Composé suivant la revendication 15, dans lequel X représente un groupe NR⁷.

46. Composé suivant la revendication 1, dans lequel X représente un atome d'azote et R⁷ représente un groupe alkyle en C₁ à C₆.

47. Composé suivant la revendication 1, dans lequel X représente un atome d'azote et R⁷ représente un groupe alkylsulfonyle en C₁ à C₆.

48. Composé suivant la revendication 1, dans lequel X représente un atome d'azote et R⁷ représente un groupe arylsulfonyle en C₆ à C₁₀.

49. Composé suivant la revendication 1, dans lequel X représente un atome d'azote et R⁷ représente un groupe [(alkyle en C₁ à C₆)]₂N-(C=O)- ou (alkyle en C₁ à C₆)NH-(C=O)-.

50. Composé suivant la revendication 1, dans lequel X représente un atome d'azote et R⁷ représente un groupe (alkyle en C₁ à C₆) (C=O)-.

51. Composé suivant la revendication 1, dans lequel ledit composé est choisi dans le groupe consistant en :
hydroxamide d'acide (2*S*,3*S*)-4-[4-(3,5-difluorobenzyloxy)-benzènesulfonyl]-2-méthylthiomorpholine-3-carboxylique ;
hydroxamide d'acide (2*S*,3*S*)-4-[4-(4-fluorobenzyloxy)-benzènesulfonyl]-2-méthylthiomorpholine-3-carboxylique ;
hydroxamide d'acide (2*S*,3*R*,6*S*)-2,6-diméthyl-4-[4-(2-méthylbenzyloxy)-benzènesulfonyl]-morpholine-3-carboxylique ;
hydroxamide d'acide 4-(4-benzyloxybenzènesulfonyl)-2-méthylmorpholine-3-carboxylique ;
hydroxamide d'acide , (2*S*,3*R*,6*S*)-4-[4-(4-fluorobenzyloxy)-benzènesulfonyl]-2,6-diméthylmorpholine-3-carboxylique ;
hydroxamide d'acide (3*R*,6*S*)-4-[4-(4-fluorobenzyloxy)-benzènesulfonyl]-2,2,6-triméthylmorpholine-3-carboxylique ;
hydroxamide d'acide (2*S*,3*R*,6*S*)-6-éthyl-4-[4-(4-fluorobenzyloxy)-benzènesulfonyl]-2-méthylmorpholine-3-carboxylique ;
hydroxamide d'acide (2*R*,3*R*,6*S*)-4-[4-(4-fluorobenzyloxy)-benzènesulfonyl]-2,6-diméthylmorpholine-3-carboxylique ;
hydroxamide d'acide (2*S*,3*R*,6*R*)-4-[4-(4-fluorobenzyloxy)-benzènesulfonyl]-2,6-diméthylmorpholine-3-carboxylique ;
hydroxamide d'acide (2*S*,3*R*,6*S*)-2,6-diméthyl-4-[4-(pyridine-4-ylméthoxy)-benzènesulfonyl]-morpholine-3-carboxylique ;
hydroxamide d'acide (2*S*,3*R*,6*S*)-2,6-diméthyl-4-[4-(pyridine-2-ylméthoxy)-benzènesulfonyl]-morpholine-3-carboxylique ;
hydroxamide d'acide (2*S*,3*R*,6*S*)-2,6-diméthyl-4-[4-(pyridine-3-ylméthoxy)-benzènesulfonyl]-morpholine-3-carboxylique ;
hydroxamide d'acide (2*S*,3*R*,6*S*)-2,6-diméthyl-4-[4-(2-méthylpyridine-3ylméthoxy)-benzènesulfonyl]-morpholine-3-carboxylique ;
hydroxamide d'acide (3*R*,6*S*)-2,2,6-triméthyl-4-[4-(2-trifluorométhylbenzyloxy)-benzènesulfonyl]-morpholine-3-carboxylique ;
hydroxamide d'acide (2*S*,3*R*)-2,6,6-triméthyl-4-[4-(pyridine-4-ylméthoxy)-benzènesulfonyl]-morpholine-3-carboxylique ;
hydroxamide d'acide (3*R*,6*S*)-2,2,6-triméthyl-4-[4-(2-méthylpyridine-3-ylméthoxy)-benzènesulfonyl]-morpholine-3-carboxylique ;
hydroxamide d'acide (2*S*,3*R*,6*S*)-[4-(2,5-diméthylbenzyloxy)-benzènesulfonyl]-2,6-diméthylmorpholine-3-carboxylique ;
hydroxamide d'acide (2*S*,3*R*,6*S*)-4-[4-(3,5-difluorobenzyloxy)-benzènesulfonyl]-2,6-diméthylmorpholine-3-carboxylique ;
hydroxamide d'acide (2*S*,3*R*,6*S*)-4-[4-(3-méthoxybenzyloxy)-benzènesulfonyl]-2,6-diméthylmorpholine-3-carboxylique ;
hydroxamide d'acide (2*S*,3*R*,6*S*)-4-[4-(5-fluoro-2-méthylbenzyloxy)-benzènesulfonyl]-2,6-diméthylmorpholine-3-carboxylique ;
hydroxamide d'acide (2*S*,3*R*,6*S*)-4-[4-(furanne-3-ylméthoxy)-benzènesulfonyl]-2,6-diméthylmorpholine-3-carboxylique ;
hydroxamide d'acide (2*S*,3*R*,6*S*)-4-[4-(2-fluoro-3-méthylbenzyloxy)-benzènesulfonyl]-2,6-diméthylmorpholine-3-carboxylique ;
hydroxamide d'acide (2*S*,3*R*)-4-[4-(4-fluorobenzyloxy)-benzènesulfonyl]-2,6,6-triméthylmorpholine-3-carboxylique ;
hydroxamide d' acide (3*R*)-4-[4-(4-fluorobenzyloxy)-benzènesulfonyl]-6,6-diméthylmorpholine-3-carboxylique ;
hydroxamide d'acide (3*R*)-6,6-diméthyl-4-[4-(pyridine-4-yl-méthoxy)-benzènesulfonyl]-morpholine-3-carboxylique ;
hydroxamide d'acide (3*R*)-6,6-diméthyl-4-[4-(2-méthylbenzyloxy)-benzènesulfonyl]-morpholine-3-carboxylique ;
hydroxamide d'acide (2*S*,3*R*,6*S*)-4-(4-cyclohexylméthoxybenzènesulfonyl)-2,6-diméthylmorpholine-3-carboxylique ;
hydroxamide d'acide (3*R*,6*S*)-4-[4-(2,5-diméthylbenzyloxy)-benzènesulfonyl]-2,2,6-triméthylmorpholine-3-carboxylique ;
hydroxamide d'acide (2*S*,3*R*)-4-[4-(4-fluorobenzyloxy)-benzènesulfonyl]-6-méthoxyméthyl-2-méthylmorpholine-3-carboxylique ;
hydroxamide d'acide (2*S*,3*R*,6*S*)-4-[4-(3-chlorobenzyloxy)-benzènesulfonyl]-6-[(éthylméthylamino)-méthyl]-2-méthylmorpholine-3-carboxylique ;
hydroxamide d'acide (2*S*,3*R*)-4-[4-(3-chlorobenzyloxy)-benzènesulfonyl]-6-méthoxy-2-méthylmorpholine-3-carboxylique ;
hydroxamide d'acide (2*S*,3*R*,6*R*)-4-[4-(4-fluororobenzyloxy)-benzènesulfonyl]-6-hydroxyméthyl-2-méthylmorpholine-3-carboxylique.

52. Composition pharmaceutique comprenant un composé de formule (I), ou un de ses sels pharmaceutiquement acceptables, suivant l'une quelconque des revendications 1 à 51, et un support pharmaceutiquement acceptable.

53. Composé de formule (I), ou un de ses sels pharmaceutiquement acceptables, suivant l'une quelconque des revendications 1 à 51, destiné à être utilisé comme médicament.

54. Utilisation d'un composé de formule (I), ou d'un de ses sels pharmaceutiquement acceptables, suivant l'une quelconque des revendications 1 à 51, pour la production d'un médicament destiné au traitement d'une maladie choisie entre l'arthrite, une maladie intestinale inflammatoire, la maladie de Crohn, l'emphysème, le syndrome de détresse respiratoire aiguë, l'asthme, une maladie pulmonaire obstructive chronique, la maladie d'Alzheimer, la toxicité provoquée par une transplantation d'organe, la cachexie, des réactions allergiques, l'hypersensibilité de contact allergique, un cancer, une ulcération tissulaire, une resténose, la périodontite, l'épidermolyse bulleuse, l'ostéoporose, l'affaiblissement d'implants articulaires artificiels, l'athérosclérose, un anévrisme aortique, l'insuffisance cardiaque congestive, l'infarctus du myocarde, un ictus, l'ischémie cérébrale, un traumatisme crânien, une lésion de la moelle épinière, des troubles neurodégénératifs, des troubles auto-immuns, la maladie de Huntington, la maladie de Parkinson, la migraine, la dépression, la neuropathie périphérique, la douleur, l'angiopathie amyloïde cérébrale, une amplification nootrope ou cognitive, la sclérose latérale amyotrophique, la sclérose en plaques, l'angiogénèse oculaire, une lésion de la cornée, la dégénérescence maculaire, la cicatrisation anormale de plaies, des brûlures, le diabète, une invasion tumorale, une croissance tumorale, une métastase tumorale, une cicatrisation de la cornée, la sclérite, le SIDA, la septicémie et un choc septique.
